(19) Europäisches Patentamt
European Patent Office
Office européen des brevets

(11) EP 1 914 240 B1

(12) EUROPEAN PATENT SPECIFICATION

(45) Date of publication and mention of the grant of the patent:
**02.12.2009 Bulletin 2009/49**

(51) Int Cl.:
***C07K 14/435*** *(2006.01)*

(21) Application number: **07254044.6**

(22) Date of filing: **11.10.2007**

# (54) EML4-ALK fusion gene

EML4-ALK-Fusionsgen

Gène de fusion EML4-ALK

(84) Designated Contracting States:
**AT BE BG CH CY CZ DE DK EE ES FI FR GB GR HU IE IS IT LI LT LU LV MC MT NL PL PT RO SE SI SK TR**

(30) Priority: **11.10.2006 JP 2006277718**
**01.05.2007 JP 2007120670**
**24.08.2007 CA 2598893**

(43) Date of publication of application:
**23.04.2008 Bulletin 2008/17**

(60) Divisional application:
**09006058.3 / 2 116 553**

(73) Proprietors:
- **Astellas Pharma Inc.**
  **Tokyo 103-8411 (JP)**
- **CureGene K.K.**
  **Tokyo 113-0033 (JP)**

(72) Inventors:
- **Mano, Hiroyuki**
  **Tokyo 113-0033 (JP)**
- **Kuromitsu, Sadao**
  **Tokyo 103-8411 (JP)**
- **Shindo, Nobuaki**
  **Tokyo 103-8411 (JP)**
- **Soga, Takatoshi**
  **Tokyo 103-8411 (JP)**
- **Furutani, Takashi**
  **Tokyo 103-8411 (JP)**

(74) Representative: **Denison, Christopher Marcus et al**
**Mewburn Ellis LLP**
**33 Gutter Lane**
**London**
**EC2V 8AS (GB)**

(56) References cited:
- **SODA MANABU ET AL: "Identification of the transforming EML4-ALK fusion gene in non-small-cell lung cancer." NATURE 2 AUG 2007, vol. 448, no. 7153, 2 August 2007 (2007-08-02), pages 561-566, XP002464689 ISSN: 1476-4687**
- **MARZEC MICHAL ET AL: "Inhibition of ALK enzymatic activity in T-cell lymphoma cells induces apoptosis and suppresses proliferation and STAT3 phosphorylation independently of Jak3." LABORATORY INVESTIGATION; A JOURNAL OF TECHNICAL METHODS AND PATHOLOGY DEC 2005, vol. 85, no. 12, December 2005 (2005-12), pages 1544-1554, XP002464690 ISSN: 0023-6837**
- **PULFORD K ET AL: "Anaplastic lymphoma kinase proteins in growth control and cancer." JOURNAL OF CELLULAR PHYSIOLOGY JUN 2004, vol. 199, no. 3, June 2004 (2004-06), pages 330-358, XP002464691 ISSN: 0021-9541**
- **WAN WEIHUA ET AL: "Anaplastic lymphoma kinase activity is essential for the proliferation and survival of anaplastic large-cell lymphoma cells." BLOOD 15 FEB 2006, vol. 107, no. 4, 15 February 2006 (2006-02-15), pages 1617-1623, XP002464692 ISSN: 0006-4971**
- **POLLMANN MARC ET AL: "Human EML4, a novel member of the EMAP family, is essential for microtubule formation." EXPERIMENTAL CELL RESEARCH 15 OCT 2006, vol. 312, no. 17, 8 July 2006 (2006-07-08), pages 3241-3251, XP002464702 ISSN: 0014-4827**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 914 240 B1

SEQUENCE LISTING

<110> Astellas Pharma Inc., CureGene K.K.,

<120> EML4-ALK fusion gene

<130> PH-3247-EP

<150> JP 2006-277718
<151> 2006-10-11

<150> JP 2007-120670

<151> 2007-05-01

< 150> CA 2,598,893
<151> 2007-08-24

<160> 141

<170> PatentIn version 3.4

<210> 1
<211> 3900
<212> DNA
<213> Homo sapiens

<220>
<223> Inventor: MANO, HIROYUKI
Inventor: KUROMITSU, SADAO
Inventor: SHINDO, NOBUAKI
Inventor: SOGA, TAKATOSHI
Inventor: FURUTANI, TAKASHI

<220>
<221> CDS
<222> (271).. (3447)

<400> 1

ggcggcgcgg cgcggcgctc gcggctgctg cctgggaggg aggccgggca ggcggctgag 60

```
cggcgcggct ctcaacgtga cggggaagtg gttcgggcgg ccgcggctta ctaccccagg   120

gcgaacggac ggacgacgga ggcgggagcc ggtagccgag ccgggcgacc tagagaacga   180

gcgggtcagg ctcagcgtcg gccactctgt cggtccgctg aatgaagtgc ccgcccctct   240

gagcccggag cccggcgctt tccccgcaag atg gac ggt ttc gcc ggc agt ctc    294
                                 Met Asp Gly Phe Ala Gly Ser Leu
                                 1               5

gat gat agt att tct gct gca agt act tct gat gtt caa gat cgc ctg     342
Asp Asp Ser Ile Ser Ala Ala Ser Thr Ser Asp Val Gln Asp Arg Leu
    10                  15                  20

tca gct ctt gag tca cga gtt cag caa caa gaa gat gaa atc act gtg     390
Ser Ala Leu Glu Ser Arg Val Gln Gln Gln Glu Asp Glu Ile Thr Val
25                  30                  35                  40

cta aag gcg gct ttg gct gat gtt ttg agg cgt ctt gca atc tct gaa     438
Leu Lys Ala Ala Leu Ala Asp Val Leu Arg Arg Leu Ala Ile Ser Glu
                45                  50                  55

gat cat gtg gcc tca gtg aaa aaa tca gtc tca agt aaa ggc caa cca     486
Asp His Val Ala Ser Val Lys Lys Ser Val Ser Ser Lys Gly Gln Pro
            60                  65                  70

agc cct cga gca gtt att ccc atg tcc tgt ata acc aat gga agt ggt     534
Ser Pro Arg Ala Val Ile Pro Met Ser Cys Ile Thr Asn Gly Ser Gly
        75                  80                  85

gca aac aga aaa cca agt cat acc agt gct gtc tca att gca gga aaa     582
Ala Asn Arg Lys Pro Ser His Thr Ser Ala Val Ser Ile Ala Gly Lys
    90                  95                  100

gaa act ctt tca tct gct gct aaa agt ggt aca gaa aaa aag aaa gaa     630
Glu Thr Leu Ser Ser Ala Ala Lys Ser Gly Thr Glu Lys Lys Lys Glu
105                 110                 115                 120

aaa cca caa gga cag aga gaa aaa aaa gag gaa tct cat tct aat gat     678
Lys Pro Gln Gly Gln Arg Glu Lys Lys Glu Glu Ser His Ser Asn Asp
                125                 130                 135
```

caa agt cca caa att cga gca tca cct tct ccc cag ccc tct tca caa 726
Gln Ser Pro Gln Ile Arg Ala Ser Pro Ser Pro Gln Pro Ser Ser Gln
140 145 150

cct ctc caa ata cac aga caa act cca gaa agc aag aat gct act ccc 774
Pro Leu Gln Ile His Arg Gln Thr Pro Glu Ser Lys Asn Ala Thr Pro
155 160 165

acc aaa agc ata aaa cga cca tca cca gct gaa aag tca cat aat tct 822
Thr Lys Ser Ile Lys Arg Pro Ser Pro Ala Glu Lys Ser His Asn Ser
170 175 180

tgg gaa aat tca gat gat agc cgt aat aaa ttg tcg aaa ata cct tca 870
Trp Glu Asn Ser Asp Asp Ser Arg Asn Lys Leu Ser Lys Ile Pro Ser
185 190 195 200

aca ccc aaa tta ata cca aaa gtt acc aaa act gca gac aag cat aaa 918
Thr Pro Lys Leu Ile Pro Lys Val Thr Lys Thr Ala Asp Lys His Lys
205 210 215

gat gtc atc atc aac caa gaa gga gaa tat att aaa atg ttt atg cgc 966
Asp Val Ile Ile Asn Gln Glu Gly Glu Tyr Ile Lys Met Phe Met Arg
220 225 230

ggt cgg cca att acc atg ttc att cct tcc gat gtt gac aac tat gat 1014
Gly Arg Pro Ile Thr Met Phe Ile Pro Ser Asp Val Asp Asn Tyr Asp
235 240 245

gac atc aga acg gaa ctg cct cct gag aag ctc aaa ctg gag tgg gca 1062
Asp Ile Arg Thr Glu Leu Pro Pro Glu Lys Leu Lys Leu Glu Trp Ala
250 255 260

tat ggt tat cga gga aag gac tgt aga gct aat gtt tac ctt ctt ccg 1110
Tyr Gly Tyr Arg Gly Lys Asp Cys Arg Ala Asn Val Tyr Leu Leu Pro
265 270 275 280

acc ggg gaa ata gtt tat ttc att gca tca gta gta gta cta ttt aat 1158
Thr Gly Glu Ile Val Tyr Phe Ile Ala Ser Val Val Val Leu Phe Asn
285 290 295

tat gag gag aga act cag cga cac tac ctg ggc cat aca gac tgt gtg 1206
Tyr Glu Glu Arg Thr Gln Arg His Tyr Leu Gly His Thr Asp Cys Val

300 305 310

aaa tgc ctt gct ata cat cct gac aaa att agg att gca act gga cag 1254
Lys Cys Leu Ala Ile His Pro Asp Lys Ile Arg Ile Ala Thr Gly Gln
315 320 325

ata gct ggc gtg gat aaa gat gga agg cct cta caa ccc cac gtc aga 1302
Ile Ala Gly Val Asp Lys Asp Gly Arg Pro Leu Gln Pro His Val Arg
330 335 340

gtg tgg gat tct gtt act cta tcc aca ctg cag att att gga ctt ggc 1350
Val Trp Asp Ser Val Thr Leu Ser Thr Leu Gln Ile Ile Gly Leu Gly
345 350 355 360

act ttt gag cgt gga gta gga tgc ctg gat ttt tca aaa gca gat tca 1398
Thr Phe Glu Arg Gly Val Gly Cys Leu Asp Phe Ser Lys Ala Asp Ser
365 370 375

ggt gtt cat tta tgt gtt att gat gac tcc aat gag cat atg ctt act 1446
Gly Val His Leu Cys Val Ile Asp Asp Ser Asn Glu His Met Leu Thr
380 385 390

gta tgg gac tgg cag aag aaa gca aaa gga gca gaa ata aag aca aca 1494
Val Trp Asp Trp Gln Lys Lys Ala Lys Gly Ala Glu Ile Lys Thr Thr
395 400 405

aat gaa gtt gtt ttg gct gtg gag ttt cac cca aca gat gca aat acc 1542
Asn Glu Val Val Leu Ala Val Glu Phe His Pro Thr Asp Ala Asn Thr
410 415 420

ata att aca tgc ggt aaa tct cat att ttc ttc tgg acc tgg agc ggc 1590
Ile Ile Thr Cys Gly Lys Ser His Ile Phe Phe Trp Thr Trp Ser Gly
425 430 435 440

aat tca cta aca aga aaa cag gga att ttt ggg aaa tat gaa aag cca 1638
Asn Ser Leu Thr Arg Lys Gln Gly Ile Phe Gly Lys Tyr Glu Lys Pro
445 450 455

aaa ttt gtg cag tgt tta gca ttc ttg ggg aat gga gat gtt ctt act 1686
Lys Phe Val Gln Cys Leu Ala Phe Leu Gly Asn Gly Asp Val Leu Thr
460 465 470

gga gac tca ggt gga gtc atg ctt ata tgg agc aaa act act gta gag 1734
Gly Asp Ser Gly Gly Val Met Leu Ile Trp Ser Lys Thr Thr Val Glu
475 480 485

ccc aca cct ggg aaa gga cct aaa gtg tac cgc cgg aag cac cag gag 1782
Pro Thr Pro Gly Lys Gly Pro Lys Val Tyr Arg Arg Lys His Gln Glu
490 495 500

ctg caa gcc atg cag atg gag ctg cag agc cct gag tac aag ctg agc 1830
Leu Gln Ala Met Gln Met Glu Leu Gln Ser Pro Glu Tyr Lys Leu Ser
505 510 515 520

aag ctc cgc acc tcg acc atc atg acc gac tac aac ccc aac tac tgc 1878
Lys Leu Arg Thr Ser Thr Ile Met Thr Asp Tyr Asn Pro Asn Tyr Cys
525 530 535

ttt gct ggc aag acc tcc tcc atc agt gac ctg aag gag gtg ccg cgg 1926
Phe Ala Gly Lys Thr Ser Ser Ile Ser Asp Leu Lys Glu Val Pro Arg
540 545 550

aaa aac atc acc ctc att cgg ggt ctg ggc cat gga gcc ttt ggg gag 1974
Lys Asn Ile Thr Leu Ile Arg Gly Leu Gly His Gly Ala Phe Gly Glu
555 560 565

gtg tat gaa ggc cag gtg tcc gga atg ccc aac gac cca agc ccc ctg 2022
Val Tyr Glu Gly Gln Val Ser Gly Met Pro Asn Asp Pro Ser Pro Leu
570 575 580

caa gtg gct gtg aag acg ctg cct gaa gtg tgc tct gaa cag gac gaa 2070
Gln Val Ala Val Lys Thr Leu Pro Glu Val Cys Ser Glu Gln Asp Glu
585 590 595 600

ctg gat ttc ctc atg gaa gcc ctg atc atc agc aaa ttc aac cac cag 2118
Leu Asp Phe Leu Met Glu Ala Leu Ile Ile Ser Lys Phe Asn His Gln
605 610 615

aac att gtt cgc tgc att ggg gtg agc ctg caa tcc ctg ccc cgg ttc 2166
Asn Ile Val Arg Cys Ile Gly Val Ser Leu Gln Ser Leu Pro Arg Phe
620 625 630

atc ctg ctg gag ctc atg gcg ggg gga gac ctc aag tcc ttc ctc cga 2214
Ile Leu Leu Glu Leu Met Ala Gly Gly Asp Leu Lys Ser Phe Leu Arg

635 640 645

gag acc cgc cct cgc ccg agc cag ccc tcc tcc ctg gcc atg ctg gac 2262
Glu Thr Arg Pro Arg Pro Ser Gln Pro Ser Ser Leu Ala Met Leu Asp
650 655 660

ctt ctg cac gtg gct cgg gac att gcc tgt ggc tgt cag tat ttg gag 2310
Leu Leu His Val Ala Arg Asp Ile Ala Cys Gly Cys Gln Tyr Leu Glu
665 670 675 680

gaa aac cac ttc atc cac cga gac att gct gcc aga aac tgc ctc ttg 2358
Glu Asn His Phe Ile His Arg Asp Ile Ala Ala Arg Asn Cys Leu Leu
685 690 695

acc tgt cca ggc cct gga aga gtg gcc aag att gga gac ttc ggg atg 2406
Thr Cys Pro Gly Pro Gly Arg Val Ala Lys Ile Gly Asp Phe Gly Met
700 705 710

gcc cga gac atc tac agg gcg agc tac tat aga aag gga ggc tgt gcc 2454
Ala Arg Asp Ile Tyr Arg Ala Ser Tyr Tyr Arg Lys Gly Gly Cys Ala
715 720 725

atg ctg cca gtt aag tgg atg ccc cca gag gcc ttc atg gaa gga ata 2502
Met Leu Pro Val Lys Trp Met Pro Pro Glu Ala Phe Met Glu Gly Ile
730 735 740

ttc act tct aaa aca gac aca tgg tcc ttt gga gtg ctg cta tgg gaa 2550
Phe Thr Ser Lys Thr Asp Thr Trp Ser Phe Gly Val Leu Leu Trp Glu
745 750 755 760

atc ttt tct ctt gga tat atg cca tac ccc agc aaa agc aac cag gaa 2598
Ile Phe Ser Leu Gly Tyr Met Pro Tyr Pro Ser Lys Ser Asn Gln Glu
765 770 775

gtt ctg gag ttt gtc acc agt gga ggc cgg atg gac cca ccc aag aac 2646
Val Leu Glu Phe Val Thr Ser Gly Gly Arg Met Asp Pro Pro Lys Asn
780 785 790

tgc cct ggg cct gta tac cgg ata atg act cag tgc tgg caa cat cag 2694
Cys Pro Gly Pro Val Tyr Arg Ile Met Thr Gln Cys Trp Gln His Gln
795 800 805

cct gaa gac agg ccc aac ttt gcc atc att ttg gag agg att gaa tac 2742
Pro Glu Asp Arg Pro Asn Phe Ala Ile Ile Leu Glu Arg Ile Glu Tyr
810 815 820

tgc acc cag gac ccg gat gta atc aac acc gct ttg ccg ata gaa tat 2790
Cys Thr Gln Asp Pro Asp Val Ile Asn Thr Ala Leu Pro Ile Glu Tyr
825 830 835 840

ggt cca ctt gtg gaa gag gaa gag aaa gtg cct gtg agg ccc aag gac 2838
Gly Pro Leu Val Glu Glu Glu Glu Lys Val Pro Val Arg Pro Lys Asp
845 850 855

cct gag ggg gtt cct cct ctc ctg gtc tct caa cag gca aaa cgg gag 2886
Pro Glu Gly Val Pro Pro Leu Leu Val Ser Gln Gln Ala Lys Arg Glu
860 865 870

gag gag cgc agc cca gct gcc cca cca cct ctg cct acc acc tcc tct 2934
Glu Glu Arg Ser Pro Ala Ala Pro Pro Pro Leu Pro Thr Thr Ser Ser
875 880 885

ggc aag gct gca aag aaa ccc aca gct gca gag gtc tct gtt cga gtc 2982
Gly Lys Ala Ala Lys Lys Pro Thr Ala Ala Glu Val Ser Val Arg Val
890 895 900

cct aga ggg ccg gcc gtg gaa ggg gga cac gtg aat atg gca ttc tct 3030
Pro Arg Gly Pro Ala Val Glu Gly Gly His Val Asn Met Ala Phe Ser
905 910 915 920

cag tcc aac cct cct tcg gag ttg cac agg gtc cac gga tcc aga aac 3078
Gln Ser Asn Pro Pro Ser Glu Leu His Arg Val His Gly Ser Arg Asn
925 930 935

aag ccc acc agc ttg tgg aac cca acg tac ggc tcc tgg ttt aca gag 3126
Lys Pro Thr Ser Leu Trp Asn Pro Thr Tyr Gly Ser Trp Phe Thr Glu
940 945 950

aaa ccc acc aaa aag aat aat cct ata gca aag aag gag cca cac gag 3174
Lys Pro Thr Lys Lys Asn Asn Pro Ile Ala Lys Lys Glu Pro His Glu
955 960 965

agg ggt aac ctg ggg ctg gag gga agc tgt act gtc cca cct aac gtt 3222
Arg Gly Asn Leu Gly Leu Glu Gly Ser Cys Thr Val Pro Pro Asn Val

970 975 980

gca act ggg aga ctt ccg ggg gcc tca ctg ctc cta gag ccc tct tcg 3270
Ala Thr Gly Arg Leu Pro Gly Ala Ser Leu Leu Leu Glu Pro Ser Ser
985 990 995 1000

ctg act gcc aat atg aag gag gta cct ctg ttc agg cta cgt cac 3315
Leu Thr Ala Asn Met Lys Glu Val Pro Leu Phe Arg Leu Arg His
1005 1010 1015

ttc cct tgt ggg aat gtc aat tac ggc tac cag caa cag ggc ttg 3360
Phe Pro Cys Gly Asn Val Asn Tyr Gly Tyr Gln Gln Gln Gly Leu
1020 1025 1030

ccc tta gaa gcc gct act gcc cct gga gct ggt cat tac gag gat 3405
Pro Leu Glu Ala Ala Thr Ala Pro Gly Ala Gly His Tyr Glu Asp
1035 1040 1045

acc att ctg aaa agc aag aat agc atg aac cag cct ggg ccc 3447
Thr Ile Leu Lys Ser Lys Asn Ser Met Asn Gln Pro Gly Pro
1050 1055

tgagctcggt cacacactca cttctcttcc ttgggatccc taagaccgtg gaggagagag 3507

aggcaatcaa tggctccttc acaaaccaga gaccaaatgt cacgttttgt tttgtgccaa 3567

cctattttga agtaccacca aaaaagctgt attttgaaaa tgctttagaa aggttttgag 3627

catgggttca tcctattctt tcgaaagaag aaaatatcat aaaaatgagt gataaataca 3687

aggcccagat gtggttgcat aaggttttta tgcatgtttg ttgtatactt ccttatgctt 3747

cttttaaatt gtgtgtgctc tgcttcaatg tagtcagaat tagctgcttc tatgtttcat 3807

agttggggtc atagatgttt ccttgccttg ttgatgtgga catgagccat ttgaggggag 3867

agggaacgga aataaaggag ttatttgtaa tga 3900

<210> 2
<211> 1059
<212> PRT
<213> Homo sapiens

<400> 2

Met Asp Gly Phe Ala Gly Ser Leu Asp Asp Ser Ile Ser Ala Ala Ser
1 5 10 15

Thr Ser Asp Val Gln Asp Arg Leu Ser Ala Leu Glu Ser Arg Val Gln
20 25 30

Gln Gln Glu Asp Glu Ile Thr Val Leu Lys Ala Ala Leu Ala Asp Val
35 40 45

Leu Arg Arg Leu Ala Ile Ser Glu Asp His Val Ala Ser Val Lys Lys
50 55 60

Ser Val Ser Ser Lys Gly Gln Pro Ser Pro Arg Ala Val Ile Pro Met
65 70 75 80

Ser Cys Ile Thr Asn Gly Ser Gly Ala Asn Arg Lys Pro Ser His Thr
85 90 95

Ser Ala Val Ser Ile Ala Gly Lys Glu Thr Leu Ser Ser Ala Ala Lys
100 105 110

Ser Gly Thr Glu Lys Lys Lys Glu Lys Pro Gln Gly Gln Arg Glu Lys
115 120 125

Lys Glu Glu Ser His Ser Asn Asp Gln Ser Pro Gln Ile Arg Ala Ser
130 135 140

Pro Ser Pro Gln Pro Ser Ser Gln Pro Leu Gln Ile His Arg Gln Thr
145 150 155 160

Pro Glu Ser Lys Asn Ala Thr Pro Thr Lys Ser Ile Lys Arg Pro Ser
165 170 175

Pro Ala Glu Lys Ser His Asn Ser Trp Glu Asn Ser Asp Asp Ser Arg
180 185 190

Asn Lys Leu Ser Lys Ile Pro Ser Thr Pro Lys Leu Ile Pro Lys Val
195 200 205

Thr Lys Thr Ala Asp Lys His Lys Asp Val Ile Ile Asn Gln Glu Gly
210 215 220

Glu Tyr Ile Lys Met Phe Met Arg Gly Arg Pro Ile Thr Met Phe Ile
225 230 235 240

Pro Ser Asp Val Asp Asn Tyr Asp Asp Ile Arg Thr Glu Leu Pro Pro
245 250 255

Glu Lys Leu Lys Leu Glu Trp Ala Tyr Gly Tyr Arg Gly Lys Asp Cys
260 265 270

Arg Ala Asn Val Tyr Leu Leu Pro Thr Gly Glu Ile Val Tyr Phe Ile
275 280 285

Ala Ser Val Val Val Leu Phe Asn Tyr Glu Glu Arg Thr Gln Arg His
290 295 300

Tyr Leu Gly His Thr Asp Cys Val Lys Cys Leu Ala Ile His Pro Asp
305 310 315 320

Lys Ile Arg Ile Ala Thr Gly Gln Ile Ala Gly Val Asp Lys Asp Gly
325 330 335

Arg Pro Leu Gln Pro His Val Arg Val Trp Asp Ser Val Thr Leu Ser
340 345 350

Thr Leu Gln Ile Ile Gly Leu Gly Thr Phe Glu Arg Gly Val Gly Cys
355 360 365

Leu Asp Phe Ser Lys Ala Asp Ser Gly Val His Leu Cys Val Ile Asp
370 375 380

Asp Ser Asn Glu His Met Leu Thr Val Trp Asp Trp Gln Lys Lys Ala
385 390 395 400

Lys Gly Ala Glu Ile Lys Thr Thr Asn Glu Val Val Leu Ala Val Glu
405 410 415

Phe His Pro Thr Asp Ala Asn Thr Ile Ile Thr Cys Gly Lys Ser His
420 425 430

Ile Phe Phe Trp Thr Trp Ser Gly Asn Ser Leu Thr Arg Lys Gln Gly
435 440 445

Ile Phe Gly Lys Tyr Glu Lys Pro Lys Phe Val Gln Cys Leu Ala Phe
450 455 460

Leu Gly Asn Gly Asp Val Leu Thr Gly Asp Ser Gly Gly Val Met Leu
465 470 475 480

Ile Trp Ser Lys Thr Thr Val Glu Pro Thr Pro Gly Lys Gly Pro Lys
485 490 495

Val Tyr Arg Arg Lys His Gln Glu Leu Gln Ala Met Gln Met Glu Leu
500 505 510

Gln Ser Pro Glu Tyr Lys Leu Ser Lys Leu Arg Thr Ser Thr Ile Met
515 520 525

Thr Asp Tyr Asn Pro Asn Tyr Cys Phe Ala Gly Lys Thr Ser Ser Ile
530 535 540

Ser Asp Leu Lys Glu Val Pro Arg Lys Asn Ile Thr Leu Ile Arg Gly
545 550 555 560

Leu Gly His Gly Ala Phe Gly Glu Val Tyr Glu Gly Gln Val Ser Gly
565 570 575

Met Pro Asn Asp Pro Ser Pro Leu Gln Val Ala Val Lys Thr Leu Pro
580 585 590

Glu Val Cys Ser Glu Gln Asp Glu Leu Asp Phe Leu Met Glu Ala Leu
595 600 605

Ile Ile Ser Lys Phe Asn His Gln Asn Ile Val Arg Cys Ile Gly Val
610 615 620

Ser Leu Gln Ser Leu Pro Arg Phe Ile Leu Leu Glu Leu Met Ala Gly
625 630 635 640

Gly Asp Leu Lys Ser Phe Leu Arg Glu Thr Arg Pro Arg Pro Ser Gln
645 650 655

Pro Ser Ser Leu Ala Met Leu Asp Leu Leu His Val Ala Arg Asp Ile
660 665 670

Ala Cys Gly Cys Gln Tyr Leu Glu Glu Asn His Phe Ile His Arg Asp
675 680 685

Ile Ala Ala Arg Asn Cys Leu Leu Thr Cys Pro Gly Pro Gly Arg Val
690 695 700

Ala Lys Ile Gly Asp Phe Gly Met Ala Arg Asp Ile Tyr Arg Ala Ser
705 710 715 720

Tyr Tyr Arg Lys Gly Gly Cys Ala Met Leu Pro Val Lys Trp Met Pro
725 730 735

Pro Glu Ala Phe Met Glu Gly Ile Phe Thr Ser Lys Thr Asp Thr Trp
740 745 750

Ser Phe Gly Val Leu Leu Trp Glu Ile Phe Ser Leu Gly Tyr Met Pro
755 760 765

Tyr Pro Ser Lys Ser Asn Gln Glu Val Leu Glu Phe Val Thr Ser Gly
770 775 780

Gly Arg Met Asp Pro Pro Lys Asn Cys Pro Gly Pro Val Tyr Arg Ile
785 790 795 800

Met Thr Gln Cys Trp Gln His Gln Pro Glu Asp Arg Pro Asn Phe Ala
805 810 815

Ile Ile Leu Glu Arg Ile Glu Tyr Cys Thr Gln Asp Pro Asp Val Ile
820 825 830

Asn Thr Ala Leu Pro Ile Glu Tyr Gly Pro Leu Val Glu Glu Glu Glu
835 840 845

Lys Val Pro Val Arg Pro Lys Asp Pro Glu Gly Val Pro Pro Leu Leu
850 855 860

Val Ser Gln Gln Ala Lys Arg Glu Glu Glu Arg Ser Pro Ala Ala Pro
865 870 875 880

Pro Pro Leu Pro Thr Thr Ser Ser Gly Lys Ala Ala Lys Lys Pro Thr
885 890 895

Ala Ala Glu Val Ser Val Arg Val Pro Arg Gly Pro Ala Val Glu Gly
900 905 910

Gly His Val Asn Met Ala Phe Ser Gln Ser Asn Pro Pro Ser Glu Leu
915 920 925

His Arg Val His Gly Ser Arg Asn Lys Pro Thr Ser Leu Trp Asn Pro
930 935 940

Thr Tyr Gly Ser Trp Phe Thr Glu Lys Pro Thr Lys Lys Asn Asn Pro
945 950 955 960

Ile Ala Lys Lys Glu Pro His Glu Arg Gly Asn Leu Gly Leu Glu Gly
965 970 975

Ser Cys Thr Val Pro Pro Asn Val Ala Thr Gly Arg Leu Pro Gly Ala
980 985 990

Ser Leu Leu Leu Glu Pro Ser Ser Leu Thr Ala Asn Met Lys Glu Val
995 1000 1005

Pro Leu Phe Arg Leu Arg His Phe Pro Cys Gly Asn Val Asn Tyr
1010 1015 1020

Gly Tyr Gln Gln Gln Gly Leu Pro Leu Glu Ala Ala Thr Ala Pro
1025 1030 1035

Gly Ala Gly His Tyr Glu Asp Thr Ile Leu Lys Ser Lys Asn Ser
1040 1045 1050

Met Asn Gln Pro Gly Pro
1055

<210>
<211> 942
<212> DNA
<213> Homo sapiens

<400> 3

ggagatgttc ttactggaga ctcaggtgga gtcatgctta tatggagcaa aactactgta 60

gagcccacac ctgggaaagg acctaaaggt gtatatcaaa tcagcaaaca aatcaaagct 120

catgatggca gtgtgttcac actttgtcag atgagaaatg ggatgttatt aactggagga 180

gggaaagaca gaaaaataat tctgtgggat catgatctga atcctgaaag agaaatagag 240

gttcctgatc agtatggcac aatcagagct gtagcagaag gaaaggcaga tcaattttta 300

gtaggcacat cacgaaactt tattttacga ggaacattta atgatggctt ccaaatagaa 360

gtacagggtc atacagatga gctttggggt cttgccacac atcccttcaa agatttgctc 420

ttgacatgtg ctcaggacag gcaggtgtgc ctgtggaact caatggaaca caggctggaa 480

tggaccaggc tggtagatga accaggacac tgtgcagatt ttcatccaag tggcacagtg 540

gtggccatag gaacgcactc aggcaggtgg tttgttctgg atgcagaaac cagagatcta 600

gtttctatcc acacagacgg gaatgaacag ctctctgtga tgcgctactc aatagatggt 660

accttcctgg ctgtaggatc tcatgacaac tttatttacc tctatgtagt ctctgaaaat 720

ggaagaaaat atagcagata tggaaggtgc actggacatt ccagctacat cacacacctt 780

gactggtccc cagacaacaa gtatataatg tctaactcgg gagactatga aatattgtac 840

ttgtaccgcc ggaagcacca ggagctgcaa gccatgcaga tggagctgca gagccctgag 900

tacaagctga gcaagctccg cacctcgacc atcatgaccg ac 942

<210> 4
<211> 3979
<212> DNA
<213> Homo sapiens

<400> 4

gtacagtatt cttatattaa actcatttct ggtaattctc acatagtact ctttcagtcc 60

catctcttag accaggagag aaagagctgc agtgtaacaa tatgagcaag tcaccaacat 120

accttttgtt ttcagcattc ttcataatct ttttttaatg aaattattta tccagttatt 180

tattaagctt atataaccca catttgacta tactgaacca ttccctttag gaagttaata 240

attagaaaga aatgatatgg atatatgtta gtttaaaaag tataaaggct cactttcccc 300

tgagctggtt ctgggatatc tgttagagca gaatgcatgg ccatgtgata aaatggaaga 360

gtggagagaa aaggaataaa ctcatagttc aatccacttc tcctttttct tttcctcact 420

gcagcccttc ttcccaacct tagtgtaggg gtccttggca tccattcagc tttacctcaa 480

atcagttttc ctaaaaaaca cagatttatt cttaagtttt atttgcaccc aaatatacag 540

gtcctctttg cccttttcta cggtaaatga aaaaattaga ggaaggacat ggaaaataac 600

cttttttaaa ccccattttt cgttactatt taagaataat caaactttga aaaaaatgtc 660

cagagtacca tgtacttcct tcagagtagg aggttctaag agaaaaattg gagacctggt 720

tctaaaacca gtgggccagg agaagagaga gattcttagc agcaacaggt ggtaacagta 780

gaaatagaac tagctaggtc agccagtcct caataattca ccaaaaaaaa agtaagaaag 840

gaggtaatgg ggaaatcacc ctgagttttc tgagacattt cccctgggga ttggcagggc 900

aacatgtcct ttctcttgtt cattcacctc ccctaaacac actggaacaa caccttactt 960

ctctctctct ctctctctct ggagtccttg catcagacca tgaaaatcac cctaagtgat 1020

ttatgaataa gggaatccct gttcgtgtgc tcagaaagac ccgatttaca tatctgcctt 1080

ccccaataag ctttccctgg gctagcacag tgtctggcat ttgctattag ctgtttgaaa 1140

actatttgtc aaacattaag gaaataactt tcatcttaac tttgggtgtc aatgtaggag 1200

taaaagttga gtagtattga tgaaagcact gttttcaccg aaatgtggaa tttaaatttc 1260

gcattactgt tttcttatga ctgcacagag tttcatactg tatttttttag gacagatatt 1320

cagatgctcc ttgacttctg gatggcatta tatcccgata aatctatcat aatgtcaaaa 1380

aataataaat tgaaccaaca taagttaggc actatctgta cgaaattaat gttttaatta 1440

aatgtttttc aaatccattc acctgaatgt ctaagcttgg cagttgaatt agacctatta 1500

agaagtttaa aacaagaagc cttaaattgt attaccattg actcctatct caatcattgg 1560

ccatatattc ttagcctctt tcgttcagtt taggttcaaa atttacacac gagaagaatg 1620

gcagtgggtt gagggttctt aaaaaataga gttacaacaa caatatatta tcaactactt 1680

ttcccattta tttctctttt cttttttttct tatttttctt tcctgggcac atttacttag 1740

tatcagaaat gccaggaaca gcatagtcta ttaaaacagt gacttttaaa ttgttcacga 1800

atctccaaca ctgagcagaa tttgcgtata aatataccct tttgttagcc ttaatatttg 1860

tgttcaggtt ggggacagga agaagaggtt cagaggtttt tattttggtc tttgaaaaat 1920

ttgtggctca acctctctac atatgcacaa acaggaaaca ggaaatccaa tcttgtaaat 1980

tgctgggcac atgaaatggc ctgtgttgta ctttgccaca tgactaaccc tattatgggc 2040

aaagttgcta agaatttgag gttttcaatt ggctgggaag aatttggtga gcgcagtgaa 2100

ataaatggaa gtccagacca gaataagtgt ccacagtttc cattttttaa ttctggcccc 2160

cagatttcag tagatccaat ttacatattc ccaactatga catttctgca cctgtggcag 2220

tcttaccaac caaaatttga gtgtactgtg acatcctgtc tgataaaagg agattttttg 2280

gatggcagaa gtataatttc acaactaatc cttttacttt tgtaatagga gacaaaccaa 2340

aactgtattt tatgtcttaa aagtgctttt atatattttt ttcttgtttc caaaccattt 2400

cttccttaaa catgaattaa tgtgaatctt caaagtaatt agtgttttct gtttcttaag 2460

taggtatgag gtatttgtta tttatttcct acacatatga aatagattga aattgttctt 2520

atgcaaaata atttgtaatc ttttaagtgg gtaagtggaa gttgagagta tctacagaac 2580

cattaaaatg ttcaccattc ttaagtcttg caaaggagct aagaaacact gccattttat 2640

gtgttctcca aataatcaaa tgggcccagt tgctaagaat acaacataaa tttcaaagtc 2700

tccctaatat tttattcttt aaacaggcac agaaagcctt ccaaacatct ttatttggca 2760

ggcagtgtaa acttgctttt tatggaagct ttgtgtctgt tagagatgag aagaaagggt 2820

gaggtttgtg attaagtttg gatggacttg accatatgag taagcctgaa ggcagagtag 2880

gtttttttaa ttcaaggata attattttcc ttggcaggga tttgaaacat tatttggcaa 2940

ctgaaatatc agaaatacag gcccataaga aagacatctt tggctataaa tttgttttac 3000

aggtaaatac tatataaagg aaactttgat aatttgttta aattagaaac actaaatttt 3060

ttaagctaag tcgatactag gtccacctca ggaaataact ttcctggagt gagaaggact 3120

cactgacttg atcactatat agagtatttt tatttctgat gaagcatttt tttcttcatt 3180
tttttttcca gttttccatc attctgggag gattttaagt gtttaacaag gtttttgtca 3240
tgtttagaaa tacggaaagc agacttaagc atagaaaagc tttattttct tacattctga 3300
tagagaacta tgaaactccc acacctttgc tttttgtgtt ttcttacatg ataccttcag 3360
gctactcttg ttagtttgac catgcacagg gaaataagcc tagaatttgc ttttctctat 3420
ttttattatc caaataaagc cagtagtact ctcagaaaat tctcatctct caggtgtcct 3480
ccctctcgtg gtaacatcag aacagagata gatacttatc tacctatgcc agtgaacaca 3540
gttgtgttgt tcaattttta aggtattttt agatgataaa tattgatgta agtggagaca 3600
gttgacctga acagcaagtt tgttggagtc taatcccatc tccagtctgc ttcttggagg 3660
aaccagacta acatgactct gccctatata atacaaataa ttattttcca tatatctgat 3720
ttttagcttt gcatttactt taaatcatgc ttcaattaaa gacacacctt ctttaatcat 3780
tttattagta tttctaagta tgatggaaag gttcagagct caggggagga tatggagatc 3840
cagggaggct tcctgtagga agtggcctgt gtagtgcttc aagggccagg ctgccaggcc 3900
atgttgcagc tgaccaccca cctgcagtgt accgccggaa gcaccaggag ctgcaagcca 3960
tgcagatgga gctgcagag 3979

<210> 5
<211> 853
<212> DNA
<213> Homo sapiens

<400> 5

aatgtctaac tcgggagact atgaaatatt gtactgtaag tatgaatgat tttatatata 60
tatatatatg ctatgattat atttatatat atatatatat atgctaagat gtgtctgtca 120

ggggcgctaa tgaacaggct gcatggaatc tgaattgtgc agagaatgct tgccaacctc 180

tttaacctga caaagcatat gttatgctga gctaaggtaa tgagaatctc aaatgtgatt 240

cacttctcca agagtaatga attaatgtta atagtgtaga acagaaggca catatagtaa 300

taaaaaatta ctctgtcaaa ttgatgctgc tctgaatggt ttttcattta attacttctc 360

ctggaggcag ggaggaatat gatagatggg catttatgct ttttagagga aaaaaaaaac 420

ttccatggga atcagtttgt agttttataa accctgttaa agtgaacact ttcttttcct 480

ttttaaatgt gtcttaatgt ttttcagtgt atggattata aatacaagta aacgtggcta 540

gtttgaatca agatgcactt tcaaatacat ttgtacacaa aataattatt ttccatatat 600

ctgattttta gctttgcatt tactttaaat catgcttcaa ttaaagacac accttcttta 660

atcattttat tagtatttct aagtatgatg gaaaggttca gagctcaggg gaggatatgg 720

agatccaggg aggcttcctg taggaagtgg cctgtgtagt gcttcaaggg ccaggctgcc 780

aggccatgtt gcagctgacc acccacctgc agtgtaccgc cggaagcacc aggagctgca 840

agccatgcag atg 853

<210> 6
<211> 3933
<212> DNA
<213> Homo sapiens

<220>
<221> CDS
<222> (1).. (3930)

<400> 6

atg gac ggt ttc gcc ggc agt ctc gat gat agt att tct gct gca agt 48
Met Asp Gly Phe Ala Gly Ser Leu Asp Asp Ser Ile Ser Ala Ala Ser
1 5 10 15

act tct gat gtt caa gat cgc ctg tca gct ctt gag tca cga gtt cag 96
Thr Ser Asp Val Gln Asp Arg Leu Ser Ala Leu Glu Ser Arg Val Gln
20 25 30

caa caa gaa gat gaa atc act gtg cta aag gcg gct ttg gct gat gtt 144
Gln Gln Glu Asp Glu Ile Thr Val Leu Lys Ala Ala Leu Ala Asp Val
35 40 45

ttg agg cgt ctt gca atc tct gaa gat cat gtg gcc tca gtg aaa aaa 192
Leu Arg Arg Leu Ala Ile Ser Glu Asp His Val Ala Ser Val Lys Lys
50 55 60

tca gtc tca agt aaa ggc caa cca agc cct cga gca gtt att ccc atg 240
Ser Val Ser Ser Lys Gly Gln Pro Ser Pro Arg Ala Val Ile Pro Met
65 70 75 80

tcc tgt ata acc aat gga agt ggt gca aac aga aaa cca agt cat acc 288
Ser Cys Ile Thr Asn Gly Ser Gly Ala Asn Arg Lys Pro Ser His Thr
85 90 95

agt gct gtc tca att gca gga aaa gaa act ctt tca tct gct gct aaa 336
Ser Ala Val Ser Ile Ala Gly Lys Glu Thr Leu Ser Ser Ala Ala Lys
100 105 110

agt ggt aca gaa aaa aag aaa gaa aaa cca caa gga cag aga gaa aaa 384
Ser Gly Thr Glu Lys Lys Lys Glu Lys Pro Gln Gly Gln Arg Glu Lys
115 120 125

aaa gag gaa tct cat tct aat gat caa agt cca caa att cga gca tca 432
Lys Glu Glu Ser His Ser Asn Asp Gln Ser Pro Gln Ile Arg Ala Ser
130 135 140

cct tct ccc cag ccc tct tca caa cct ctc caa ata cac aga caa act 480
Pro Ser Pro Gln Pro Ser Ser Gln Pro Leu Gln Ile His Arg Gln Thr
145 150 155 160

cca gaa agc aag aat gct act ccc acc aaa agc ata aaa cga cca tca 528
Pro Glu Ser Lys Asn Ala Thr Pro Thr Lys Ser Ile Lys Arg Pro Ser
165 170 175

cca gct gaa aag tca cat aat tct tgg gaa aat tca gat gat agc cgt 576
Pro Ala Glu Lys Ser His Asn Ser Trp Glu Asn Ser Asp Asp Ser Arg

180 185 190

aat aaa ttg tcg aaa ata cct tca aca ccc aaa tta ata cca aaa gtt 624
Asn Lys Leu Ser Lys Ile Pro Ser Thr Pro Lys Leu Ile Pro Lys Val
195 200 205

acc aaa act gca gac aag cat aaa gat gtc atc atc aac caa gaa gga 672
Thr Lys Thr Ala Asp Lys His Lys Asp Val Ile Ile Asn Gln Glu Gly
210 215 220

gaa tat att aaa atg ttt atg cgc ggt cgg cca att acc atg ttc att 720
Glu Tyr Ile Lys Met Phe Met Arg Gly Arg Pro Ile Thr Met Phe Ile
225 230 235 240

cct tcc gat gtt gac aac tat gat gac atc aga acg gaa ctg cct cct 768
Pro Ser Asp Val Asp Asn Tyr Asp Asp Ile Arg Thr Glu Leu Pro Pro
245 250 255

gag aag ctc aaa ctg gag tgg gca tat ggt tat cga gga aag gac tgt 816
Glu Lys Leu Lys Leu Glu Trp Ala Tyr Gly Tyr Arg Gly Lys Asp Cys
260 265 270

aga gct aat gtt tac ctt ctt ccg acc ggg gaa ata gtt tat ttc att 864
Arg Ala Asn Val Tyr Leu Leu Pro Thr Gly Glu Ile Val Tyr Phe Ile
275 280 285

gca tca gta gta gta cta ttt aat tat gag gag aga act cag cga cac 912
Ala Ser Val Val Val Leu Phe Asn Tyr Glu Glu Arg Thr Gln Arg His
290 295 300

tac ctg ggc cat aca gac tgt gtg aaa tgc ctt gct ata cat cct gac 960
Tyr Leu Gly His Thr Asp Cys Val Lys Cys Leu Ala Ile His Pro Asp
305 310 315 320

aaa att agg att gca act gga cag ata gct ggc gtg gat aaa gat gga 1008
Lys Ile Arg Ile Ala Thr Gly Gln Ile Ala Gly Val Asp Lys Asp Gly
325 330 335

agg cct cta caa ccc cac gtc aga gtg tgg gat tct gtt act cta tcc 1056
Arg Pro Leu Gln Pro His Val Arg Val Trp Asp Ser Val Thr Leu Ser
340 345 350

```
aca ctg cag att att gga ctt ggc act ttt gag cgt gga gta gga tgc      1104
Thr Leu Gln Ile Ile Gly Leu Gly Thr Phe Glu Arg Gly Val Gly Cys
        355                 360                 365

ctg gat ttt tca aaa gca gat tca ggt gtt cat tta tgt gtt att gat      1152
Leu Asp Phe Ser Lys Ala Asp Ser Gly Val His Leu Cys Val Ile Asp
    370                 375                 380

gac tcc aat gag cat atg ctt act gta tgg gac tgg cag aag aaa gca      1200
Asp Ser Asn Glu His Met Leu Thr Val Trp Asp Trp Gln Lys Lys Ala
385                 390                 395                 400

aaa gga gca gaa ata aag aca aca aat gaa gtt gtt ttg gct gtg gag      1248
Lys Gly Ala Glu Ile Lys Thr Thr Asn Glu Val Val Leu Ala Val Glu
                405                 410                 415

ttt cac cca aca gat gca aat acc ata att aca tgc ggt aaa tct cat      1296
Phe His Pro Thr Asp Ala Asn Thr Ile Ile Thr Cys Gly Lys Ser His
            420                 425                 430

att ttc ttc tgg acc tgg agc ggc aat tca cta aca aga aaa cag gga      1344
Ile Phe Phe Trp Thr Trp Ser Gly Asn Ser Leu Thr Arg Lys Gln Gly
        435                 440                 445

att ttt ggg aaa tat gaa aag cca aaa ttt gtg cag tgt tta gca ttc      1392
Ile Phe Gly Lys Tyr Glu Lys Pro Lys Phe Val Gln Cys Leu Ala Phe
    450                 455                 460

ttg ggg aat gga gat gtt ctt act gga gac tca ggt gga gtc atg ctt      1440
Leu Gly Asn Gly Asp Val Leu Thr Gly Asp Ser Gly Gly Val Met Leu
465                 470                 475                 480

ata tgg agc aaa act act gta gag ccc aca cct ggg aaa gga cct aaa      1488
Ile Trp Ser Lys Thr Thr Val Glu Pro Thr Pro Gly Lys Gly Pro Lys
                485                 490                 495

ggt gta tat caa atc agc aaa caa atc aaa gct cat gat ggc agt gtg      1536
Gly Val Tyr Gln Ile Ser Lys Gln Ile Lys Ala His Asp Gly Ser Val
            500                 505                 510

ttc aca ctt tgt cag atg aga aat ggg atg tta tta act gga gga ggg      1584
Phe Thr Leu Cys Gln Met Arg Asn Gly Met Leu Leu Thr Gly Gly Gly
```

515 520 525

aaa gac aga aaa ata att ctg tgg gat cat gat ctg aat cct gaa aga 1632
Lys Asp Arg Lys Ile Ile Leu Trp Asp His Asp Leu Asn Pro Glu Arg
530 535 540

gaa ata gag gtt cct gat cag tat ggc aca atc aga gct gta gca gaa 1680
Glu Ile Glu Val Pro Asp Gln Tyr Gly Thr Ile Arg Ala Val Ala Glu
545 550 555 560

gga aag gca gat caa ttt tta gta ggc aca tca cga aac ttt att tta 1728
Gly Lys Ala Asp Gln Phe Leu Val Gly Thr Ser Arg Asn Phe Ile Leu
565 570 575

cga gga aca ttt aat gat ggc ttc caa ata gaa gta cag ggt cat aca 1776
Arg Gly Thr Phe Asn Asp Gly Phe Gln Ile Glu Val Gln Gly His Thr
580 585 590

gat gag ctt tgg ggt ctt gcc aca cat ccc ttc aaa gat ttg ctc ttg 1824
Asp Glu Leu Trp Gly Leu Ala Thr His Pro Phe Lys Asp Leu Leu Leu
595 600 605

aca tgt gct cag gac agg cag gtg tgc ctg tgg aac tca atg gaa cac 1872
Thr Cys Ala Gln Asp Arg Gln Val Cys Leu Trp Asn Ser Met Glu His
610 615 620

agg ctg gaa tgg acc agg ctg gta gat gaa cca gga cac tgt gca gat 1920
Arg Leu Glu Trp Thr Arg Leu Val Asp Glu Pro Gly His Cys Ala Asp
625 630 635 640

ttt cat cca agt ggc aca gtg gtg gcc ata gga acg cac tca ggc agg 1968
Phe His Pro Ser Gly Thr Val Val Ala Ile Gly Thr His Ser Gly Arg
645 650 655

tgg ttt gtt ctg gat gca gaa acc aga gat cta gtt tct atc cac aca 2016
Trp Phe Val Leu Asp Ala Glu Thr Arg Asp Leu Val Ser Ile His Thr
660 665 670

gac ggg aat gaa cag ctc tct gtg atg cgc tac tca ata gat ggt acc 2064
Asp Gly Asn Glu Gln Leu Ser Val Met Arg Tyr Ser Ile Asp Gly Thr
675 680 685

ttc ctg gct gta gga tct cat gac aac ttt att tac ctc tat gta gtc 2112
Phe Leu Ala Val Gly Ser His Asp Asn Phe Ile Tyr Leu Tyr Val Val
690 695 700

tct gaa aat gga aga aaa tat agc aga tat gga agg tgc act gga cat 2160
Ser Glu Asn Gly Arg Lys Tyr Ser Arg Tyr Gly Arg Cys Thr Gly His
705 710 715 720

tcc agc tac atc aca cac ctt gac tgg tcc cca gac aac aag tat ata 2208
Ser Ser Tyr Ile Thr His Leu Asp Trp Ser Pro Asp Asn Lys Tyr Ile
725 730 735

atg tct aac tcg gga gac tat gaa ata ttg tac ttg tac cgc cgg aag 2256
Met Ser Asn Ser Gly Asp Tyr Glu Ile Leu Tyr Leu Tyr Arg Arg Lys
740 745 750

cac cag gag ctg caa gcc atg cag atg gag ctg cag agc cct gag tac 2304
His Gln Glu Leu Gln Ala Met Gln Met Glu Leu Gln Ser Pro Glu Tyr
755 760 765

aag ctg agc aag ctc cgc acc tcg acc atc atg acc gac tac aac ccc 2352
Lys Leu Ser Lys Leu Arg Thr Ser Thr Ile Met Thr Asp Tyr Asn Pro
770 775 780

aac tac tgc ttt gct ggc aag acc tcc tcc atc agt gac ctg aag gag 2400
Asn Tyr Cys Phe Ala Gly Lys Thr Ser Ser Ile Ser Asp Leu Lys Glu
785 790 795 800

gtg ccg cgg aaa aac atc acc ctc att cgg ggt ctg ggc cat gga gcc 2448
Val Pro Arg Lys Asn Ile Thr Leu Ile Arg Gly Leu Gly His Gly Ala
805 810 815

ttt ggg gag gtg tat gaa ggc cag gtg tcc gga atg ccc aac gac cca 2496
Phe Gly Glu Val Tyr Glu Gly Gln Val Ser Gly Met Pro Asn Asp Pro
820 825 830

agc ccc ctg caa gtg gct gtg aag acg ctg cct gaa gtg tgc tct gaa 2544
Ser Pro Leu Gln Val Ala Val Lys Thr Leu Pro Glu Val Cys Ser Glu
835 840 845

cag gac gaa ctg gat ttc ctc atg gaa gcc ctg atc atc agc aaa ttc 2592
Gln Asp Glu Leu Asp Phe Leu Met Glu Ala Leu Ile Ile Ser Lys Phe

850 855 860

aac cac cag aac att gtt cgc tgc att ggg gtg agc ctg caa tcc ctg 2640
Asn His Gln Asn Ile Val Arg Cys Ile Gly Val Ser Leu Gln Ser Leu
865 870 875 880

ccc cgg ttc atc ctg ctg gag ctc atg gcg ggg gga gac ctc aag tcc 2688
Pro Arg Phe Ile Leu Leu Glu Leu Met Ala Gly Gly Asp Leu Lys Ser
885 890 895

ttc ctc cga gag acc cgc cct cgc ccg agc cag ccc tcc tcc ctg gcc 2736
Phe Leu Arg Glu Thr Arg Pro Arg Pro Ser Gln Pro Ser Ser Leu Ala
900 905 910

atg ctg gac ctt ctg cac gtg gct cgg gac att gcc tgt ggc tgt cag 2784
Met Leu Asp Leu Leu His Val Ala Arg Asp Ile Ala Cys Gly Cys Gln
915 920 925

tat ttg gag gaa aac cac ttc atc cac cga gac att gct gcc aga aac 2832
Tyr Leu Glu Glu Asn His Phe Ile His Arg Asp Ile Ala Ala Arg Asn
930 935 940

tgc ctc ttg acc tgt cca ggc cct gga aga gtg gcc aag att gga gac 2880
Cys Leu Leu Thr Cys Pro Gly Pro Gly Arg Val Ala Lys Ile Gly Asp
945 950 955 960

ttc ggg atg gcc cga gac atc tac agg gcg agc tac tat aga aag gga 2928
Phe Gly Met Ala Arg Asp Ile Tyr Arg Ala Ser Tyr Tyr Arg Lys Gly
965 970 975

ggc tgt gcc atg ctg cca gtt aag tgg atg ccc cca gag gcc ttc atg 2976
Gly Cys Ala Met Leu Pro Val Lys Trp Met Pro Pro Glu Ala Phe Met
980 985 990

gaa gga ata ttc act tct aaa aca gac aca tgg tcc ttt gga gtg ctg 3024
Glu Gly Ile Phe Thr Ser Lys Thr Asp Thr Trp Ser Phe Gly Val Leu
995 1000 1005

cta tgg gaa atc ttt tct ctt gga tat atg cca tac ccc agc aaa 3069
Leu Trp Glu Ile Phe Ser Leu Gly Tyr Met Pro Tyr Pro Ser Lys
1010 1015 1020

agc aac cag gaa gtt ctg gag ttt gtc acc agt gga ggc cgg atg 3114
Ser Asn Gln Glu Val Leu Glu Phe Val Thr Ser Gly Gly Arg Met
1025 1030 1035

gac cca ccc aag aac tgc cct ggg cct gta tac cgg ata atg act 3159
Asp Pro Pro Lys Asn Cys Pro Gly Pro Val Tyr Arg Ile Met Thr
1040 1045 1050

cag tgc tgg caa cat cag cct gaa gac agg ccc aac ttt gcc atc 3204
Gln Cys Trp Gln His Gln Pro Glu Asp Arg Pro Asn Phe Ala Ile
1055 1060 1065

att ttg gag agg att gaa tac tgc acc cag gac ccg gat gta atc 3249
Ile Leu Glu Arg Ile Glu Tyr Cys Thr Gln Asp Pro Asp Val Ile
1070 1075 1080

aac acc gct ttg ccg ata gaa tat ggt cca ctt gtg gaa gag gaa 3294
Asn Thr Ala Leu Pro Ile Glu Tyr Gly Pro Leu Val Glu Glu Glu
1085 1090 1095

gag aaa gtg cct gtg agg ccc aag gac cct gag ggg gtt cct cct 3339
Glu Lys Val Pro Val Arg Pro Lys Asp Pro Glu Gly Val Pro Pro
1100 1105 1110

ctc ctg gtc tct caa cag gca aaa cgg gag gag gag cgc agc cca 3384
Leu Leu Val Ser Gln Gln Ala Lys Arg Glu Glu Glu Arg Ser Pro
1115 1120 1125

gct gcc cca cca cct ctg cct acc acc tcc tct ggc aag gct gca 3429
Ala Ala Pro Pro Pro Leu Pro Thr Thr Ser Ser Gly Lys Ala Ala
1130 1135 1140

aag aaa ccc aca gct gca gag gtc tct gtt cga gtc cct aga ggg 3474
Lys Lys Pro Thr Ala Ala Glu Val Ser Val Arg Val Pro Arg Gly
1145 1150 1155

ccg gcc gtg gaa ggg gga cac gtg aat atg gca ttc tct cag tcc 3519
Pro Ala Val Glu Gly Gly His Val Asn Met Ala Phe Ser Gln Ser
1160 1165 1170

aac cct cct tcg gag ttg cac agg gtc cac gga tcc aga aac aag 3564
Asn Pro Pro Ser Glu Leu His Arg Val His Gly Ser Arg Asn Lys

1175 1180 1185

ccc acc agc ttg tgg aac cca acg tac ggc tcc tgg ttt aca gag 3609
Pro Thr Ser Leu Trp Asn Pro Thr Tyr Gly Ser Trp Phe Thr Glu
1190 1195 1200

aaa ccc acc aaa aag aat aat cct ata gca aag aag gag cca cac 3654
Lys Pro Thr Lys Lys Asn Asn Pro Ile Ala Lys Lys Glu Pro His
1205 1210 1215

gag agg ggt aac ctg ggg ctg gag gga agc tgt act gtc cca cct 3699
Glu Arg Gly Asn Leu Gly Leu Glu Gly Ser Cys Thr Val Pro Pro
1220 1225 1230

aac gtt gca act ggg aga ctt ccg ggg gcc tca ctg ctc cta gag 3744
Asn Val Ala Thr Gly Arg Leu Pro Gly Ala Ser Leu Leu Leu Glu
1235 1240 1245

ccc tct tcg ctg act gcc aat atg aag gag gta cct ctg ttc agg 3789
Pro Ser Ser Leu Thr Ala Asn Met Lys Glu Val Pro Leu Phe Arg
1250 1255 1260

cta cgt cac ttc cct tgt ggg aat gtc aat tac ggc tac cag caa 3834
Leu Arg His Phe Pro Cys Gly Asn Val Asn Tyr Gly Tyr Gln Gln
1265 1270 1275

cag ggc ttg ccc tta gaa gcc gct act gcc cct gga gct ggt cat 3879
Gln Gly Leu Pro Leu Glu Ala Ala Thr Ala Pro Gly Ala Gly His
1280 1285 1290

tac gag gat acc att ctg aaa agc aag aat agc atg aac cag cct 3924
Tyr Glu Asp Thr Ile Leu Lys Ser Lys Asn Ser Met Asn Gln Pro
1295 1300 1305

ggg ccc tga 3933
Gly Pro
1310

<210> 7
<211> 1310
<212> PRT
<213> Homo sapiens

<400> 7

Met Asp Gly Phe Ala Gly Ser Leu Asp Asp Ser Ile Ser Ala Ala Ser
1 5 10 15

Thr Ser Asp Val Gln Asp Arg Leu Ser Ala Leu Glu Ser Arg Val Gln
20 25 30

Gln Gln Glu Asp Glu Ile Thr Val Leu Lys Ala Ala Leu Ala Asp Val
35 40 45

Leu Arg Arg Leu Ala Ile Ser Glu Asp His Val Ala Ser Val Lys Lys
50 55 60

Ser Val Ser Ser Lys Gly Gln Pro Ser Pro Arg Ala Val Ile Pro Met
65 70 75 80

Ser Cys Ile Thr Asn Gly Ser Gly Ala Asn Arg Lys Pro Ser His Thr
85 90 95

Ser Ala Val Ser Ile Ala Gly Lys Glu Thr Leu Ser Ser Ala Ala Lys
100 105 110

Ser Gly Thr Glu Lys Lys Lys Glu Lys Pro Gln Gly Gln Arg Glu Lys
115 120 125

Lys Glu Glu Ser His Ser Asn Asp Gln Ser Pro Gln Ile Arg Ala Ser
130 135 140

Pro Ser Pro Gln Pro Ser Ser Gln Pro Leu Gln Ile His Arg Gln Thr
145 150 155 160

Pro Glu Ser Lys Asn Ala Thr Pro Thr Lys Ser Ile Lys Arg Pro Ser
165 170 175

Pro Ala Glu Lys Ser His Asn Ser Trp Glu Asn Ser Asp Asp Ser Arg
180 185 190

Asn Lys Leu Ser Lys Ile Pro Ser Thr Pro Lys Leu Ile Pro Lys Val
195 200 205

Thr Lys Thr Ala Asp Lys His Lys Asp Val Ile Ile Asn Gln Glu Gly
210 215 220

Glu Tyr Ile Lys Met Phe Met Arg Gly Arg Pro Ile Thr Met Phe Ile
225 230 235 240

Pro Ser Asp Val Asp Asn Tyr Asp Asp Ile Arg Thr Glu Leu Pro Pro
245 250 255

Glu Lys Leu Lys Leu Glu Trp Ala Tyr Gly Tyr Arg Gly Lys Asp Cys
260 265 270

Arg Ala Asn Val Tyr Leu Leu Pro Thr Gly Glu Ile Val Tyr Phe Ile
275 280 285

Ala Ser Val Val Val Leu Phe Asn Tyr Glu Glu Arg Thr Gln Arg His
290 295 300

Tyr Leu Gly His Thr Asp Cys Val Lys Cys Leu Ala Ile His Pro Asp
305 310 315 320

Lys Ile Arg Ile Ala Thr Gly Gln Ile Ala Gly Val Asp Lys Asp Gly
325 330 335

Arg Pro Leu Gln Pro His Val Arg Val Trp Asp Ser Val Thr Leu Ser
340 345 350

Thr Leu Gln Ile Ile Gly Leu Gly Thr Phe Glu Arg Gly Val Gly Cys
355 360 365

Leu Asp Phe Ser Lys Ala Asp Ser Gly Val His Leu Cys Val Ile Asp
370 375 380

Asp Ser Asn Glu His Met Leu Thr Val Trp Asp Trp Gln Lys Lys Ala
385 390 395 400

Lys Gly Ala Glu Ile Lys Thr Thr Asn Glu Val Val Leu Ala Val Glu
405 410 415

Phe His Pro Thr Asp Ala Asn Thr Ile Ile Thr Cys Gly Lys Ser His
420 425 430

Ile Phe Phe Trp Thr Trp Ser Gly Asn Ser Leu Thr Arg Lys Gln Gly
435 440 445

Ile Phe Gly Lys Tyr Glu Lys Pro Lys Phe Val Gln Cys Leu Ala Phe
450 455 460

Leu Gly Asn Gly Asp Val Leu Thr Gly Asp Ser Gly Gly Val Met Leu
465 470 475 480

Ile Trp Ser Lys Thr Thr Val Glu Pro Thr Pro Gly Lys Gly Pro Lys
485 490 495

Gly Val Tyr Gln Ile Ser Lys Gln Ile Lys Ala His Asp Gly Ser Val
500 505 510

Phe Thr Leu Cys Gln Met Arg Asn Gly Met Leu Leu Thr Gly Gly Gly
515 520 525

Lys Asp Arg Lys Ile Ile Leu Trp Asp His Asp Leu Asn Pro Glu Arg
530 535 540

Glu Ile Glu Val Pro Asp Gln Tyr Gly Thr Ile Arg Ala Val Ala Glu
545 550 555 560

Gly Lys Ala Asp Gln Phe Leu Val Gly Thr Ser Arg Asn Phe Ile Leu
565 570 575

Arg Gly Thr Phe Asn Asp Gly Phe Gln Ile Glu Val Gln Gly His Thr
580 585 590

Asp Glu Leu Trp Gly Leu Ala Thr His Pro Phe Lys Asp Leu Leu Leu
595 600 605

Thr Cys Ala Gln Asp Arg Gln Val Cys Leu Trp Asn Ser Met Glu His
610 615 620

Arg Leu Glu Trp Thr Arg Leu Val Asp Glu Pro Gly His Cys Ala Asp
625 630 635 640

Phe His Pro Ser Gly Thr Val Val Ala Ile Gly Thr His Ser Gly Arg
645 650 655

Trp Phe Val Leu Asp Ala Glu Thr Arg Asp Leu Val Ser Ile His Thr
660 665 670

Asp Gly Asn Glu Gln Leu Ser Val Met Arg Tyr Ser Ile Asp Gly Thr
675 680 685

Phe Leu Ala Val Gly Ser His Asp Asn Phe Ile Tyr Leu Tyr Val Val
690 695 700

Ser Glu Asn Gly Arg Lys Tyr Ser Arg Tyr Gly Arg Cys Thr Gly His
705 710 715 720

Ser Ser Tyr Ile Thr His Leu Asp Trp Ser Pro Asp Asn Lys Tyr Ile
725 730 735

Met Ser Asn Ser Gly Asp Tyr Glu Ile Leu Tyr Leu Tyr Arg Arg Lys
740 745 750

His Gln Glu Leu Gln Ala Met Gln Met Glu Leu Gln Ser Pro Glu Tyr
755 760 765

Lys Leu Ser Lys Leu Arg Thr Ser Thr Ile Met Thr Asp Tyr Asn Pro
770 775 780

Asn Tyr Cys Phe Ala Gly Lys Thr Ser Ser Ile Ser Asp Leu Lys Glu
785 790 795 800

Val Pro Arg Lys Asn Ile Thr Leu Ile Arg Gly Leu Gly His Gly Ala
805 810 815

Phe Gly Glu Val Tyr Glu Gly Gln Val Ser Gly Met Pro Asn Asp Pro
820 825 830

Ser Pro Leu Gln Val Ala Val Lys Thr Leu Pro Glu Val Cys Ser Glu
835 840 845

Gln Asp Glu Leu Asp Phe Leu Met Glu Ala Leu Ile Ile Ser Lys Phe
850 855 860

Asn His Gln Asn Ile Val Arg Cys Ile Gly Val Ser Leu Gln Ser Leu
865 870 875 880

Pro Arg Phe Ile Leu Leu Glu Leu Met Ala Gly Gly Asp Leu Lys Ser
885 890 895

Phe Leu Arg Glu Thr Arg Pro Arg Pro Ser Gln Pro Ser Ser Leu Ala
900 905 910

Met Leu Asp Leu Leu His Val Ala Arg Asp Ile Ala Cys Gly Cys Gln
915 920 925

Tyr Leu Glu Glu Asn His Phe Ile His Arg Asp Ile Ala Ala Arg Asn
930 935 940

Cys Leu Leu Thr Cys Pro Gly Pro Gly Arg Val Ala Lys Ile Gly Asp
945 950 955 960

Phe Gly Met Ala Arg Asp Ile Tyr Arg Ala Ser Tyr Tyr Arg Lys Gly
965 970 975

Gly Cys Ala Met Leu Pro Val Lys Trp Met Pro Pro Glu Ala Phe Met
980 985 990

Glu Gly Ile Phe Thr Ser Lys Thr Asp Thr Trp Ser Phe Gly Val Leu
995 1000 1005

Leu Trp Glu Ile Phe Ser Leu Gly Tyr Met Pro Tyr Pro Ser Lys
1010 1015 1020

Ser Asn Gln Glu Val Leu Glu Phe Val Thr Ser Gly Gly Arg Met
1025 1030 1035

Asp Pro Pro Lys Asn Cys Pro Gly Pro Val Tyr Arg Ile Met Thr
1040 1045 1050

Gln Cys Trp Gln His Gln Pro Glu Asp Arg Pro Asn Phe Ala Ile
1055 1060 1065

Ile Leu Glu Arg Ile Glu Tyr Cys Thr Gln Asp Pro Asp Val Ile
1070 1075 1080

Asn Thr Ala Leu Pro Ile Glu Tyr Gly Pro Leu Val Glu Glu Glu
1085 1090 1095

Glu Lys Val Pro Val Arg Pro Lys Asp Pro Glu Gly Val Pro Pro
1100 1105 1110

Leu Leu Val Ser Gln Gln Ala Lys Arg Glu Glu Glu Arg Ser Pro
1115 1120 1125

Ala Ala Pro Pro Pro Leu Pro Thr Thr Ser Ser Gly Lys Ala Ala
1130 1135 1140

Lys Lys Pro Thr Ala Ala Glu Val Ser Val Arg Val Pro Arg Gly
1145 1150 1155

Pro Ala Val Glu Gly Gly His Val Asn Met Ala Phe Ser Gln Ser
1160 1165 1170

Asn Pro Pro Ser Glu Leu His Arg Val His Gly Ser Arg Asn Lys
1175 1180 1185

Pro Thr Ser Leu Trp Asn Pro Thr Tyr Gly Ser Trp Phe Thr Glu
1190 1195 1200

Lys Pro Thr Lys Lys Asn Asn Pro Ile Ala Lys Lys Glu Pro His
1205 1210 1215

Glu Arg Gly Asn Leu Gly Leu Glu Gly Ser Cys Thr Val Pro Pro
1220 1225 1230

Asn Val Ala Thr Gly Arg Leu Pro Gly Ala Ser Leu Leu Leu Glu
1235 1240 1245

Pro Ser Ser Leu Thr Ala Asn Met Lys Glu Val Pro Leu Phe Arg
1250 1255 1260

Leu Arg His Phe Pro Cys Gly Asn Val Asn Tyr Gly Tyr Gln Gln
1265 1270 1275

Gln Gly Leu Pro Leu Glu Ala Ala Thr Ala Pro Gly Ala Gly His
1280 1285 1290

Tyr Glu Asp Thr Ile Leu Lys Ser Lys Asn Ser Met Asn Gln Pro
1295 1300 1305

Gly Pro
1310

<210> 8
<211> 24
<212> DNA
<213> Homo sapiens

<400> 8
gtgcagtgtt tagcattctt gggg 24

<210> 9
<211> 24
<212> DNA
<213> Homo sapiens

<400> 9
tcttgccagc aaagcagtag ttgg 24

<210> 10
<211> 20
<212> DNA
<213> Homo sapiens

<400> 10
gtcagtggtg gacctgacct 20

<210> 11
<211> 20
<212> DNA
<213> Homo sapiens

<400> 11
tgagcttgac aaagtggtcg 20

<210> 12
<211> 18
<212> DNA
<213> Homo sapiens

<400> 12
gggaaggtga aggtcgga 18

<210> 13
<211> 17
<212> DNA
<213> Homo sapiens

<400> 13
gcagccctgg tgaccag 17

<210> 14
<211> 247
<212> DNA
<213> Homo sapiens

<400> 14

gtgcagtgtt tagcattctt ggggaatgga gatgttctta ctggagactc aggtggagtc 60

atgcttatat ggagcaaaac tactgtagag cccacacctg ggaaaggacc taaagtgtac 120

cgccggaagc accaggagct gcaagccatg cagatggagc tgcagagccc tgagtacaag 180

ctgagcaagc tccgcacctc gaccatcatg accgactaca accccaacta ctgctttgct 240

ggcaaga 247

<210> 15
<211> 16
<212> DNA
<213> Homo sapiens

<400> 15
gcacagggaa ataagc 16

<210> 16
<211> 16
<212> DNA
<213> Homo sapiens

<400> 16
gggcagagtc atgtta 16

<210> 17
<211> 16
<212> DNA
<213> Homo sapiens

<400> 17
ccagtagtac tctcag 16

<210> 18
<211> 16
<212> DNA
<213> Homo sapiens

<400> 18
cccctgagct ctgaac 16

<210> 19
<211> 17
<212> DNA
<213> Homo sapiens

<400> 19
ccagtgaaca cagttgt 17

<210> 20

<211> 17
<212> DNA
<213> Homo sapiens

<400> 20
cctggatctc catatcc 17

<210> 21
<211> 17
<212> DNA
<213> Homo sapiens

<400> 21
gatgtaagtg gagacag 17

<210> 22
<211> 17
<212> DNA
<213> Homo sapiens

<400> 22
ttcctacagg aagcctc 17

<210> 23
<211> 18
<212> DNA
<213> Homo sapiens

<400> 23
gatacttatc tacctatg 18

<210> 24
<211> 18
<212> DNA
<213> Homo sapiens

<400> 24
cctttccatc atacttag 18

<210> 25
<211> 18
<212> DNA
<213> Homo sapiens

<400> 25
gatgataaat attgatgt 18

<210> 26
<211> 18
<212> DNA
<213> Homo sapiens

<400> 26
aagcactaca caggccac 18

<210> 27
<211> 19
<212> RNA

<213> Homo sapiens

<400> 27
tgtcctccct ctcgtggta 19

<210> 28
<211> 19
<212> DNA
<213> Homo sapiens

<400> 28
gattaaagaa ggtgtgtct 19

<210> 29
<211> 19
<212> DNA
<213> Homo sapiens

<400> 29
ggctactctt gttagtttg 19

<210> 30
<211) 19
<212> DNA
<213> Homo sapiens

<400> 30
gtctggttcc tccaagaag 19

<210> 31
<211> 20
<212> DNA
<213> Homo sapiens

<400> 31
cttacatgat accttcaggc 20

<210> 32
<211> 20
<212> DNA
<213> Homo sapiens

<400> 32
caagaagcag actggagatg 20

<210> 33
<211> 20
<212> DNA
<213> Homo sapiens

<400> 33
ttgacctgaa cagcaagttt 20

<210> 34
<211> 20
<212> DNA
<213> Homo sapiens

<400> 34
ggtgggtggt cagctgcaac 20

<210> 35
<211> 24
<212> DNA
<213> Homo sapiens

<400> 35
agctacatca cacaccttga ctgg 24

<210> 36
<211> 24
<212> DNA
<213> Homo sapiens

<400> 36
agcttgctca gcttgtactc aggg 24

<210> 37
<211> 16
<212> DNA
<213> Homo sapiens

<400> 37
cttccatggg aatcag 16

<210> 38
<211> 16
<212> DNA
<213> Homo sapiens

<400> 38
ttgcagctcc tggtgc 16

<210> 39
<211> 16
<212> DNA
<213> Homo sapiens

<400> 39
gtagaacaga aggcac 16

<210> 40
<211> 224
<212> DNA
<213> Homo sapiens

<400> 40
ccacacctgg gaaaggacct aaag 24

<210> 41
<211> 17
<212> DNA
<213> Homo sapiens

<400> 41
atgctgctct gaatggt 17

<210> 42
<211> 23
<212> DNA
<213> Homo sapiens

<400> 42
aagcagtggt atcaacgcag agt 23

<210> 43
<211> 17
<212> DNA
<213> Homo sapiens

<400> 43
ggcagggagg aatatga 17

<210> 44
<211> 22
<212> DNA
<213> Homo sapiens

<400> 44
caattacggc taccagcaac ag 22

<210> 45
<211> 18
<212> DNA
<213> Homo sapiens

<400> 45
gatagatggg catttatg 18

<210> 46
<211> 23
<212> DNA
<213> Homo sapiens

<400> 46
ccacggtctt agggatccca agg 23

<210> 47
<211> 18
<212> DNA
<213> Homo sapiens

<400> 47
gggaatcagt ttgtagtt 18

<210> 48
<211> 23
<212> DNA
<213> Homo sapiens

<400> 48
tatcctcgta atgaccagct cca 23

<210> 49
<211> 19

<212> DNA
<213> Homo sapiens

<400> 49
tacttctcct ggaggcagg 19

<210> 50
<211> 24
<212> DNA
<213> Mus musculus

<400> 50
caggagagaa aggatttggc taca 24

<210> 51
<211> 19
<212> DNA
<213> Homo sapiens

<400> 51
tacaagtaaa cgtggctag 19

<210> 52
<211> 19
<212> DNA
<213> Homo sapiens

<400> 52
cttccggcgg tacactgca 19

<210> 53
<211> 20
<212> DNA
<213> Homo sapiens

<400> 53
ctgttaaagt gaacactttc 20

<210> 54
<211> 20
<212> DNA
<213> Homo sapiens

<400> 54
acatggcctg gcagcctggc 20

<210> 55
<211> 20
<212> DNA
<213> Homo sapiens

<400> 55
gtgtcttaat gtttttcagt 20

<210> 56
<211> 21
<212> DNA
<213> Mus musculus

<400> 56
tccaccctgg atcatgaagt c 21

<210> 57
<211> 27
<212> DNA
<213> Artificial

<220>
<223> Description of Artificial Sequence: an artificially synthesized primer sequence

<400> 57
ggggaattca tggacggttt cgccggc 27

<210> 58
<211> 30
<212> DNA
<213> Homo sapiens

<400> 58
cggcggtaca agtacaatat ttcatagtct 30

<210> 59
<211> 27
<212> DNA
<213> Artificial

<220>
<223> Description of Artificial Sequence: an artificially synthesized primer sequence

<400> 59
aaggaattcg gtttcgccgg cagtctc 27

<210> 60
<211> 27
<212> DNA
<213> Artificial

<220>
<223> Description of Artificial Sequence: an artificially synthesized primer sequence

<400> 60
agagaattca gtgtgcgacc gagctca 27

<210> 61
<211> 16
<212> DNA
<213> Homo sapiens

<400> 61
tggagcggca attcac 16

<210> 62
<211> 16
<212> DNA
<213> Homo sapiens

<400> 62

aaagcagtag ttgggg 16

<210> 63
<211> 16
<212> DNA
<213> Homo sapiens

<400> 63
ggggaatgga gatgtt 16

<210> 64
<211> 16
<212> DNA
<213> Homo sapiens

<400> 64
tttccgcggc acctcc 16

<210> 65
<211> 17
<212> DNA
<213> Homo sapiens

<400> 65
ctaacaagaa aacaggg 17

<210> 66
<211> 17
<212> DNA
<213> Homo sapiens

<400> 66
ggaggaggtc ttgccag 17

<210> 67
<211> 17
<212> DNA
<213> Homo sapiens

<400> 67
cttctggacc tggagcg 17

<210> 68
<211> 17
<212> DNA
<213> Homo sapiens

<400> 68
gggttgtagt cggtcat 17

<210> 69
<211> 18
<212> DNA
<213> Homo sapiens

<400> 69
ggctgtggag tttcaccc 18

<210> 70
<211> 18
<212> DNA
<213> Homo sapiens

<400> 70
agctcctggt gcttccgg 18

<210> 71
<211> 18
<212> DNA
<213> Homo sapiens

<400> 71
cccaacagat gcaaatac 18

<210> 72
<211> 18
<212> DNA
<213> Homo sapiens

<400> 72
tcagggctct gcagctcc 18

<210> 73
<211> 19
<212> DNA
<213> Homo sapiens

<400> 73
gacaacaaat gaagttgtt 19

<210> 74
<211> 19
<212> DNA
<213> Homo sapiens

<400> 74
atctgcatgg cttgcagct 19

<210> 75
<211> 19
<212> DNA
<213> Homo sapiens

<400> 75
ccataattac atgcggtaa 19

<210> 76
<211> 19
<212> DNA
<213> Homo sapiens

<400> 76
gagcttgctc agcttgtac 19

<210> 77
<211> 20

<212> DNA
<213> Homo sapiens

<400> 77
gtgtttagca ttcttgggga 20

<210> 78
<211> 20
<212>. DNA
<213> Homo sapiens

<400> 78
gaccccgaat gagggtgatg 20

<210> 79
<211> 20
<212> DNA
<213> Homo sapiens

<400> 79
gggaattttt gggaaatatg 20

<210> 80
<211> 20
<212> DNA
<213> Homo sapiens

<400> 80
cctccttcag gtcactgatg 20

<210> 81
<211> 16
<212> DNA
<213> Homo sapiens

<400> 81
cctggctgta ggatct 16

<210> 82
<211> 24
<212> DNA
<213> Homo sapiens

<400> 82
tcttgccagc aaagcagtag ttgg 24

<210> 83
<211> 16
<212> DNA
<213> Homo sapiens

<400> 83
gtggccatag gaacgc 16

<210> 84
<211> 16
<212> DNA
<213> Homo Sapiens

<400> 84
gtgcttccgg cggtac 16

<210> 85
<211> 17
<212> DNA
<213> Homo sapiens

<400> 85
ctcaatagat ggtacct 17

<210> 86
<211> 515
<212> DNA
<213> Homo sapiens

<400> 86

taccagtgct gtctcaattg caggaaaaga aactctttca tctgctgcta aaagtggtac 60

agaaaaaaag aaagaaaaac cacaaggaca gagagaaaaa aaagaggaat ctcattctaa 120

tgatcaaagt ccacaaattc gagcatcacc ttctccccag ccctcttcac aacctctcca 180

aatacacaga caaactccag aaagcaagaa tgctactccc accaaaagca taaaacgacc 240

atcaccagct gaaaagtcac ataattcttg ggaaaattca gatgatagcc gtaataaatt 300

gtcgaaaata ccttcaacac ccaaattaat accaaaagtt accaaaactg cagacaagca 360

taaagatgtc atcatcaacc aagtgtaccg ccggaagcac caggagctgc aagccatgca 420

gatggagctg cagagccctg agtacaagct gagcaagctc cgcacctcga ccatcatgac 480

cgactacaac cccaactact gctttgctgg caaga 515

<210> 87
<211> 17
<212> DNA
<213> Homo sapiens

<400> 87
gctctctgtg atgcgct 17

<210> 88
<211> 17
<212> DNA
<213> Homo sapiens

<400> 88
ggtgcggagc ttgctca 17

<210> 89
<211> 18
<212> DNA
<213> Homo sapiens

<400> 89
caggcaggtg gtttgttc 18

<210> 90
<211> 548
<212> DNA
<213> Homo sapiens

<400> 90

taccagtgct gtctcaattg caggaaaaga aactctttca tctgctgcta aaagtggtac 60

agaaaaaaag aaagaaaaac cacaaggaca gagagaaaaa aaagaggaat ctcattctaa 120

tgatcaaagt ccacaaattc gagcatcacc ttctccccag ccctcttcac aacctctcca 180

aatacacaga caaactccag aaagcaagaa tgctactccc accaaaagca taaaacgacc 240

atcaccagct gaaaagtcac ataattcttg ggaaaattca gatgatagcc gtaataaatt 300

gtcgaaaata ccttcaacac ccaaattaat accaaaagtt accaaaactg cagacaagca 360

taaagatgtc atcatcaacc aagcaaaaat gtcaactcgc aaaaaaaaca gccaagtgta 420

ccgccggaag caccaggagc tgcaagccat gcagatggag ctgcagagcc ctgagtacaa 480

gctgagcaag ctccgcacct cgaccatcat gaccgactac aacccccaact actgctttgc 540

tggcaaga 548

<210> 91
<211> 18
<212> DNA
<213> Homo sapiens

<400> 91
ggaatgaaca gctctctg 18

<210> 92
<211> 18
<212> DNA

<213> Homo sapiens

<400> 92
tcggtcatga tggtcgag 18

<210> 93
<211> 19
<212> DNA
<213> Homo sapiens

<400> 93
ggatgcagaa accagagat 19

<210> 94
<211> 24
<212> DNA
<213> Homo sapiens

<400> 94
actctgtcgg tccgctgaat gaag 24

<210> 95
<211> 19
<212> RNA
<213> Homo sapiens

<400> 95
ggaacgcact caggcaggt 19

<210> 96
<211> 19
<212> DNA
<213> Homo sapiens

<400> 96
ctcagggctc tgcagctcc 19

<210> 97
<211> 20
<212> DNA
<213> Homo sapiens

<400> 97
ctatccacac agacgggaat 20

<210> 98
<211> 20
<212> DNA
<213> Homo sapiens

<400> 98
gcttgtactc agggctctgc 20

<210> 99
<211> 20
<212> DNA
<213> Homo sapiens

<400> 99
gtagtctctg aaaatggaag 20

<210> 100
<211> 20
<212> DNA
<213> Homo sapiens <400> 100

tcttgccagc aaagcagtag 20

<210> 101
<211> 30
<212> DNA
<213> Homo sapiens

<400> 101
atattgtact tgtaccgccg gaagcaccag 30

<210> 102
<211> 27
<212> DNA
<213> Artificial

<220>
<223> Description of Artificial Sequence: an artificially synthesized primer sequence

<400> 102
gggtctagat cagggcccag gctggtt 27

<210> 103
<211> 35
<212> DNA
<213> Artificial

<220>
<223> Description of Artificial Sequence: an artificially synthesized primer sequence

<400> 103
ccctgcaagt ggctgtgatg acgctgcctg aagtg 35

<210> 104
<211> 35
<212> DNA
<213> Artificial

<220>
<223> Description of Artificial Sequence: an artificially synthesized primer sequence

<400> 104
cacttcaggc agcgtcatca cagccacttg caggg 35

<210> 105
<211> 30
<212> DNA
<213> Artificial

<220>
<223> Description of Artificial Sequence: an artificially synthesized primer sequence

<400> 105
aagcttatgg acggtttcgc cggcagtctc 30

<210> 106
<211> 24
<212> DNA
<213> Artificial

<220>
<223> Description of Artificial Sequence: an artificially synthesized primer sequence

<400> 106
tctagatcag ggcccaggct ggtt 24

<210> 107
<211> 2391
<212> DNA
<213> Homo sapiens

<220>
<221> CDS
<222> (1).. (2388)

<400> 107

```
atg gac ggt ttc gcc ggc agt ctc gat gat agt att tct gct gca agt      48
Met Asp Gly Phe Ala Gly Ser Leu Asp Asp Ser Ile Ser Ala Ala Ser
1               5                   10                  15

act tct gat gtt caa gat cgc ctg tca gct ctt gag tca cga gtt cag      96
Thr Ser Asp Val Gln Asp Arg Leu Ser Ala Leu Glu Ser Arg Val Gln
            20                  25                  30

caa caa gaa gat gaa atc act gtg cta aag gcg gct ttg gct gat gtt     144
Gln Gln Glu Asp Glu Ile Thr Val Leu Lys Ala Ala Leu Ala Asp Val
        35                  40                  45

ttg agg cgt ctt gca atc tct gaa gat cat gtg gcc tca gtg aaa aaa     192
Leu Arg Arg Leu Ala Ile Ser Glu Asp His Val Ala Ser Val Lys Lys
    50                  55                  60

tca gtc tca agt aaa ggc caa cca agc cct cga gca gtt att ccc atg     240
Ser Val Ser Ser Lys Gly Gln Pro Ser Pro Arg Ala Val Ile Pro Met
65                  70                  75                  80

tcc tgt ata acc aat gga agt ggt gca aac aga aaa cca agt cat acc     288
Ser Cys Ile Thr Asn Gly Ser Gly Ala Asn Arg Lys Pro Ser His Thr
                85                  90                  95

agt gct gtc tca att gca gga aaa gaa act ctt tca tct gct gct aaa     336
Ser Ala Val Ser Ile Ala Gly Lys Glu Thr Leu Ser Ser Ala Ala Lys
            100                 105                 110

agt ggt aca gaa aaa aag aaa gaa aaa cca caa gga cag aga gaa aaa     384
```

Ser Gly Thr Glu Lys Lys Lys Glu Lys Pro Gln Gly Gln Arg Glu Lys
115 120 125

aaa gag gaa tct cat tct aat gat caa agt cca caa att cga gca tca 432
Lys Glu Glu Ser His Ser Asn Asp Gln Ser Pro Gln Ile Arg Ala Ser
130 135 140

cct tct ccc cag ccc tct tca caa cct ctc caa ata cac aga caa act 480
Pro Ser Pro Gln Pro Ser Ser Gln Pro Leu Gln Ile His Arg Gln Thr
145 150 155 160

cca gaa agc aag aat gct act ccc acc aaa agc ata aaa cga cca tca 528
Pro Glu Ser Lys Asn Ala Thr Pro Thr Lys Ser Ile Lys Arg Pro Ser
165 170 175

cca gct gaa aag tca cat aat tct tgg gaa aat tca gat gat agc cgt 576
Pro Ala Glu Lys Ser His Asn Ser Trp Glu Asn Ser Asp Asp Ser Arg
180 185 190

aat aaa ttg tcg aaa ata cct tca aca ccc aaa tta ata cca aaa gtt 624
Asn Lys Leu Ser Lys Ile Pro Ser Thr Pro Lys Leu Ile Pro Lys Val
195 200 205

acc aaa act gca gac aag cat aaa gat gtc atc atc aac caa gca aaa 672
Thr Lys Thr Ala Asp Lys His Lys Asp Val Ile Ile Asn Gln Ala Lys
210 215 220

atg tca act cgc gaa aaa aac agc caa gtg tac cgc cgg aag cac cag 720
Met Ser Thr Arg Glu Lys Asn Ser Gln Val Tyr Arg Arg Lys His Gln
225 230 235 240

gag ctg caa gcc atg cag atg gag ctg cag agc cct gag tac aag ctg 768
Glu Leu Gln Ala Met Gln Met Glu Leu Gln Ser Pro Glu Tyr Lys Leu
245 250 255

agc aag ctc cgc acc tcg acc atc atg acc gac tac aac ccc aac tac 816
Ser Lys Leu Arg Thr Ser Thr Ile Met Thr Asp Tyr Asn Pro Asn Tyr
260 265 270

tgc ttt gct ggc aag acc tcc tcc atc agt gac ctg aag gag gtg ccg 864
Cys Phe Ala Gly Lys Thr Ser Ser Ile Ser Asp Leu Lys Glu Val Pro
275 280 285

cgg aaa aac atc acc ctc att cgg ggt ctg ggc cat ggc gcc ttt ggg 912
Arg Lys Asn Ile Thr Leu Ile Arg Gly Leu Gly His Gly Ala Phe Gly
290 295 300

gag gtg tat gaa ggc cag gtg tcc gga atg ccc aac gac cca agc ccc 960
Glu Val Tyr Glu Gly Gln Val Ser Gly Met Pro Asn Asp Pro Ser Pro
305 310 315 320

ctg caa gtg gct gtg aag acg ctg cct gaa gtg tgc tct gaa cag gac 1008
Leu Gln Val Ala Val Lys Thr Leu Pro Glu Val Cys Ser Glu Gln Asp
325 330 335

gaa ctg gat ttc ctc atg gaa gcc ctg atc atc agc aaa ttc aac cac 1056
Glu Leu Asp Phe Leu Met Glu Ala Leu Ile Ile Ser Lys Phe Asn His
340 345 350

cag aac att gtt cgc tgc att ggg gtg agc ctg caa tcc ctg ccc cgg 1104
Gln Asn Ile Val Arg Cys Ile Gly Val Ser Leu Gln Ser Leu Pro Arg
355 360 365

ttc atc ctg ctg gag ctc atg gcg ggg gga gac ctc aag tcc ttc ctc 1152
Phe Ile Leu Leu Glu Leu Met Ala Gly Gly Asp Leu Lys Ser Phe Leu
370 375 380

cga gag acc cgc cct cgc ccg agc cag ccc tcc tcc ctg gcc atg ctg 1200
Arg Glu Thr Arg Pro Arg Pro Ser Gln Pro Ser Ser Leu Ala Met Leu
385 390 395 400

gac ctt ctg cac gtg gct cgg gac att gcc tgt ggc tgt cag tat ttg 1248
Asp Leu Leu His Val Ala Arg Asp Ile Ala Cys Gly Cys Gln Tyr Leu
405 410 415

gag gaa aac cac ttc atc cac cga gac att gct gcc aga aac tgc ctc 1296
Glu Glu Asn His Phe Ile His Arg Asp Ile Ala Ala Arg Asn Cys Leu
420 425 430

ttg acc tgt cca ggc cct gga aga gtg gcc aag att gga gac ttc ggg 1344
Leu Thr Cys Pro Gly Pro Gly Arg Val Ala Lys Ile Gly Asp Phe Gly
435 440 445

atg gcc cga gac atc tac agg gcg agc tac tat aga aag gga ggc tgt 1392

Met Ala Arg Asp Ile Tyr Arg Ala Ser Tyr Tyr Arg Lys Gly Gly Cys
450 455 460

gcc atg ctg cca gtt aag tgg atg ccc cca gag gcc ttc atg gaa gga 1440
Ala Met Leu Pro Val Lys Trp Met Pro Pro Glu Ala Phe Met Glu Gly
465 470 475 480

ata ttc act tct aaa aca gac aca tgg tcc ttt gga gtg ctg cta tgg 1488
Ile Phe Thr Ser Lys Thr Asp Thr Trp Ser Phe Gly Val Leu Leu Trp
485 490 495

gaa atc ttt tct ctt gga tat atg cca tac ccc agc aaa agc aac cag 1536
Glu Ile Phe Ser Leu Gly Tyr Met Pro Tyr Pro Ser Lys Ser Asn Gln
500 505 510

gaa gtt ctg gag ttt gtc acc agt gga ggc cgg atg gac cca ccc aag 1584
Glu Val Leu Glu Phe Val Thr Ser Gly Gly Arg Met Asp Pro Pro Lys
515 520 525

aac tgc cct ggg cct gta tac cgg ata atg act cag tgc tgg caa cat 1632
Asn Cys Pro Gly Pro Val Tyr Arg Ile Met Thr Gln Cys Trp Gln His
530 535 540

cag cct gaa gac agg ccc aac ttt gcc atc att ttg gag agg att gaa 1680
Gln Pro Glu Asp Arg Pro Asn Phe Ala Ile Ile Leu Glu Arg Ile Glu
545 550 555 560

tac tgc acc cag gac ccg gat gta atc aac acc gct ttg ccg ata gaa 1728
Tyr Cys Thr Gln Asp Pro Asp Val Ile Asn Thr Ala Leu Pro Ile Glu
565 570 575

tat ggt cca ctt gtg gaa gag gaa gag aaa gtg cct gtg agg ccc aag 1776
Tyr Gly Pro Leu Val Glu Glu Glu Glu Lys Val Pro Val Arg Pro Lys
580 585 590

gac cct gag ggg gtt cct cct ctc ctg gtc tct caa cag gca aaa cgg 1824
Asp Pro Glu Gly Val Pro Pro Leu Leu Val Ser Gln Gln Ala Lys Arg
595 600 605

gag gag gag cgc agc cca gct gcc cca cca cct ctg cct acc acc tcc 1872
Glu Glu Glu Arg Ser Pro Ala Ala Pro Pro Pro Leu Pro Thr Thr Ser
610 615 620

tct ggc aag gct gca aag aaa ccc aca gct gca gag gtc tct gtt cga 1920
Ser Gly Lys Ala Ala Lys Lys Pro Thr Ala Ala Glu Val Ser Val Arg
625 630 635 640

gtc cct aga ggg ccg gcc gtg gaa ggg gga cac gtg aat atg gca ttc 1968
Val Pro Arg Gly Pro Ala Val Glu Gly Gly His Val Asn Met Ala Phe
645 650 655

tct cag tcc aac cct cct tcg gag ttg cac aag gtc cac gga tcc aga 2016
Ser Gln Ser Asn Pro Pro Ser Glu Leu His Lys Val His Gly Ser Arg
660 665 670

aac aag ccc acc agc ttg tgg aac cca acg tac ggc tcc tgg ttt aca 2064
Asn Lys Pro Thr Ser Leu Trp Asn Pro Thr Tyr Gly Ser Trp Phe Thr
675 680 685

gag aaa ccc acc aaa aag aat aat cct ata gca aag aag gag cca cac 2112
Glu Lys Pro Thr Lys Lys Asn Asn Pro Ile Ala Lys Lys Glu Pro His
690 695 700

gac agg ggt aac ctg ggg ctg gag gga agc tgt act gtc cca cct aac 2160
Asp Arg Gly Asn Leu Gly Leu Glu Gly Ser Cys Thr Val Pro Pro Asn
705 710 715 720

gtt gca act ggg aga ctt ccg ggg gcc tca ctg ctc cta gag ccc tct 2208
Val Ala Thr Gly Arg Leu Pro Gly Ala Ser Leu Leu Leu Glu Pro Ser
725 730 735

tcg ctg act gcc aat atg aag gag gta cct ctg ttc agg cta cgt cac 2256
Ser Leu Thr Ala Asn Met Lys Glu Val Pro Leu Phe Arg Leu Arg His
740 745 750

ttc cct tgt ggg aat gtc aat tac ggc tac cag caa cag ggc ttg ccc 2304
Phe Pro Cys Gly Asn Val Asn Tyr Gly Tyr Gln Gln Gln Gly Leu Pro
755 760 765

tta gaa gcc gct act gcc cct gga gct ggt cat tac gag gat acc att 2352
Leu Glu Ala Ala Thr Ala Pro Gly Ala Gly His Tyr Glu Asp Thr Ile
770 775 780

ctg aaa agc aag aat agc atg aac cag cct ggg ccc tga 2391

Leu Lys Ser Lys Asn Ser Met Asn Gln Pro Gly Pro
785 790 795

<210> 108
<211> 796
<212> PRT
<213> Homo sapiens

<400> 108

Met Asp Gly Phe Ala Gly Ser Leu Asp Asp Ser Ile Ser Ala Ala Ser
1 5 10 15

Thr Ser Asp Val Gln Asp Arg Leu Ser Ala Leu Glu Ser Arg Val Gln
20 25 30

Gln Gln Glu Asp Glu Ile Thr Val Leu Lys Ala Ala Leu Ala Asp Val
35 40 45

Leu Arg Arg Leu Ala Ile Ser Glu Asp His Val Ala Ser Val Lys Lys
50 55 60

Ser Val Ser Ser Lys Gly Gln Pro Ser Pro Arg Ala Val Ile Pro Met
65 70 75 80

Ser Cys Ile Thr Asn Gly Ser Gly Ala Asn Arg Lys Pro Ser His Thr
85 90 95

Ser Ala Val Ser Ile Ala Gly Lys Glu Thr Leu Ser Ser Ala Ala Lys
100 105 110

Ser Gly Thr Glu Lys Lys Lys Glu Lys Pro Gln Gly Gln Arg Glu Lys
115 120 125

Lys Glu Glu Ser His Ser Asn Asp Gln Ser Pro Gln Ile Arg Ala Ser
130 135 140

Pro Ser Pro Gln Pro Ser Ser Gln Pro Leu Gln Ile His Arg Gln Thr
145 150 155 160

Pro Glu Ser Lys Asn Ala Thr Pro Thr Lys Ser Ile Lys Arg Pro Ser
165 170 175

Pro Ala Glu Lys Ser His Asn Ser Trp Glu Asn Ser Asp Asp Ser Arg
180 185 190

Asn Lys Leu Ser Lys Ile Pro Ser Thr Pro Lys Leu Ile Pro Lys Val
195 200 205

Thr Lys Thr Ala Asp Lys His Lys Asp Val Ile Ile Asn Gln Ala Lys
210 215 220

Met Ser Thr Arg Glu Lys Asn Ser Gln Val Tyr Arg Arg Lys His Gln
225 230 235 240

Glu Leu Gln Ala Met Gln Met Glu Leu Gln Ser Pro Glu Tyr Lys Leu
245 250 255

Ser Lys Leu Arg Thr Ser Thr Ile Met Thr Asp Tyr Asn Pro Asn Tyr
260 265 270

Cys Phe Ala Gly Lys Thr Ser Ser Ile Ser Asp Leu Lys Glu Val Pro
275 280 285

Arg Lys Asn Ile Thr Leu Ile Arg Gly Leu Gly His Gly Ala Phe Gly

290 295 300

Glu Val Tyr Glu Gly Gln Val Ser Gly Met Pro Asn Asp Pro Ser Pro
305 310 315 320

Leu Gln Val Ala Val Lys Thr Leu Pro Glu Val Cys Ser Glu Gln Asp
325 330 335

Glu Leu Asp Phe Leu Met Glu Ala Leu Ile Ile Ser Lys Phe Asn His
340 345 350

Gln Asn Ile Val Arg Cys Ile Gly Val Ser Leu Gln Ser Leu Pro Arg
355 360 365

Phe Ile Leu Leu Glu Leu Met Ala Gly Gly Asp Leu Lys Ser Phe Leu
370 375 380

Arg Glu Thr Arg Pro Arg Pro Ser Gln Pro Ser Ser Leu Ala Met Leu
385 390 395 400

Asp Leu Leu His Val Ala Arg Asp Ile Ala Cys Gly Cys Gln Tyr Leu
405 410 415

Glu Glu Asn His Phe Ile His Arg Asp Ile Ala Ala Arg Asn Cys Leu
420 425 430

Leu Thr Cys Pro Gly Pro Gly Arg Val Ala Lys Ile Gly Asp Phe Gly
435 440 445

Met Ala Arg Asp Ile Tyr Arg Ala Ser Tyr Tyr Arg Lys Gly Gly Cys
450 455 460

Ala Met Leu Pro Val Lys Trp Met Pro Pro Glu Ala Phe Met Glu Gly
465 470 475 480

Ile Phe Thr Ser Lys Thr Asp Thr Trp Ser Phe Gly Val Leu Leu Trp
485 490 495

Glu Ile Phe Ser Leu Gly Tyr Met Pro Tyr Pro Ser Lys Ser Asn Gln
500 505 510

Glu Val Leu Glu Phe Val Thr Ser Gly Gly Arg Met Asp Pro Pro Lys
515 520 525

Asn Cys Pro Gly Pro Val Tyr Arg Ile Met Thr Gln Cys Trp Gln His
530 535 540

Gln Pro Glu Asp Arg Pro Asn Phe Ala Ile Ile Leu Glu Arg Ile Glu
545 550 555 560

Tyr Cys Thr Gln Asp Pro Asp Val Ile Asn Thr Ala Leu Pro Ile Glu
565 570 575

Tyr Gly Pro Leu Val Glu Glu Glu Glu Lys Val Pro Val Arg Pro Lys
580 585 590

Asp Pro Glu Gly Val Pro Pro Leu Leu Val Ser Gln Gln Ala Lys Arg
595 600 605

Glu Glu Glu Arg Ser Pro Ala Ala Pro Pro Pro Leu Pro Thr Thr Ser
610 615 620

Ser Gly Lys Ala Ala Lys Lys Pro Thr Ala Ala Glu Val Ser Val Arg

625 630 635 640

Val Pro Arg Gly Pro Ala Val Glu Gly Gly His Val Asn Met Ala Phe
645 650 655

Ser Gln Ser Asn Pro Pro Ser Glu Leu His Lys Val His Gly Ser Arg
660 665 670

Asn Lys Pro Thr Ser Leu Trp Asn Pro Thr Tyr Gly Ser Trp Phe Thr
675 680 685

Glu Lys Pro Thr Lys Lys Asn Asn Pro Ile Ala Lys Lys Glu Pro His
690 695 700

Asp Arg Gly Asn Leu Gly Leu Glu Gly Ser Cys Thr Val Pro Pro Asn
705 710 715 720

Val Ala Thr Gly Arg Leu Pro Gly Ala Ser Leu Leu Leu Glu Pro Ser
725 730 735

Ser Leu Thr Ala Asn Met Lys Glu Val Pro Leu Phe Arg Leu Arg His
740 745 750

Phe Pro Cys Gly Asn Val Asn Tyr Gly Tyr Gln Gln Gln Gly Leu Pro
755 760 765

Leu Glu Ala Ala Thr Ala Pro Gly Ala Gly His Tyr Glu Asp Thr Ile
770 775 780

Leu Lys Ser Lys Asn Ser Met Asn Gln Pro Gly Pro
785 790 795

<210> 109
<211> 2358
<212> DNA
<213> . Homo sapiens

<220>
<221> CDS
<222> (1)..(2355)

<400> 109

```
atg gac ggt ttc gcc ggc agt ctc gat gat agt att tct gct gca agt        48
Met Asp Gly Phe Ala Gly Ser Leu Asp Asp Ser Ile Ser Ala Ala Ser
1               5                   10                  15

act tct gat gtt caa gat cgc ctg tca gct ctt gag tca cga gtt cag        96
Thr Ser Asp Val Gln Asp Arg Leu Ser Ala Leu Glu Ser Arg Val Gln
            20                  25                  30

caa caa gaa gat gaa atc act gtg cta aag gcg gct ttg gct gat gtt       144
Gln Gln Glu Asp Glu Ile Thr Val Leu Lys Ala Ala Leu Ala Asp Val
        35                  40                  45

ttg agg cgt ctt gca atc tct gaa gat cat gtg gcc tca gtg aaa aaa       192
Leu Arg Arg Leu Ala Ile Ser Glu Asp His Val Ala Ser Val Lys Lys
    50                  55                  60

tca gtc tca agt aaa ggc caa cca agc cct cga gca gtt att ccc atg       240
Ser Val Ser Ser Lys Gly Gln Pro Ser Pro Arg Ala Val Ile Pro Met
65                  70                  75                  80

tcc tgt ata acc aat gga agt ggt gca aac aga aaa cca agt cat acc       288
Ser Cys Ile Thr Asn Gly Ser Gly Ala Asn Arg Lys Pro Ser His Thr
                85                  90                  95

agt gct gtc tca att gca gga aaa gaa act ctt tca tct gct gct aaa       336
Ser Ala Val Ser Ile Ala Gly Lys Glu Thr Leu Ser Ser Ala Ala Lys
            100                 105                 110

agt ggt aca gaa aaa aag aaa gaa aaa cca caa gga cag aga gaa aaa       384
Ser Gly Thr Glu Lys Lys Lys Glu Lys Pro Gln Gly Gln Arg Glu Lys
```

115 120 125

aaa gag gaa tct cat tct aat gat caa agt cca caa att cga gca tca 432
Lys Glu Glu Ser His Ser Asn Asp Gln Ser Pro Gln Ile Arg Ala Ser
130 135 140

cct tct ccc cag ccc tct tca caa cct ctc caa ata cac aga caa act 480
Pro Ser Pro Gln Pro Ser Ser Gln Pro Leu Gln Ile His Arg Gln Thr
145 150 155 160

cca gaa agc aag aat gct act ccc acc aaa agc ata aaa cga cca tca 528
Pro Glu Ser Lys Asn Ala Thr Pro Thr Lys Ser Ile Lys Arg Pro Ser
165 170 175

cca gct gaa aag tca cat aat tct tgg gaa aat tca gat gat agc cgt 576
Pro Ala Glu Lys Ser His Asn Ser Trp Glu Asn Ser Asp Asp Ser Arg
180 185 190

aat aaa ttg tcg aaa ata cct tca aca ccc aaa tta ata cca aaa gtt 624
Asn Lys Leu Ser Lys Ile Pro Ser Thr Pro Lys Leu Ile Pro Lys Val
195 200 205

acc aaa act gca gac aag cat aaa gat gtc atc atc aac caa gtg tac 672
Thr Lys Thr Ala Asp Lys His Lys Asp Val Ile Ile Asn Gln Val Tyr
210 215 220

cgc cgg aag cac cag gag ctg caa gcc atg cag atg gag ctg cag agc 720
Arg Arg Lys His Gln Glu Leu Gln Ala Met Gln Met Glu Leu Gln Ser
225 230 235 240

cct gag tac aag ctg agc aag ctc cgc acc tcg acc atc atg acc gac 768
Pro Glu Tyr Lys Leu Ser Lys Leu Arg Thr Ser Thr Ile Met Thr Asp
245 250 255

tac aac ccc aac tac tgc ttt gct ggc aag acc tcc tcc atc agt gac 816
Tyr Asn Pro Asn Tyr Cys Phe Ala Gly Lys Thr Ser Ser Ile Ser Asp
260 265 270

ctg aag gag gtg ccg cgg aaa aac atc acc ctc att cgg ggt ctg ggc 864
Leu Lys Glu Val Pro Arg Lys Asn Ile Thr Leu Ile Arg Gly Leu Gly
275 280 285

cat ggc gcc ttt ggg gag gtg tat gaa ggc cag gtg tcc gga atg ccc 912
His Gly Ala Phe Gly Glu Val Tyr Glu Gly Gln Val Ser Gly Met Pro
290 295 300

aac gac cca agc ccc ctg caa gtg gct gtg aag acg ctg cct gaa gtg 960
Asn Asp Pro Ser Pro Leu Gln Val Ala Val Lys Thr Leu Pro Glu Val
305 310 315 320

tgc tct gaa cag gac gaa ctg gat ttc ctc atg gaa gcc ctg atc atc 1008
Cys Ser Glu Gln Asp Glu Leu Asp Phe Leu Met Glu Ala Leu Ile Ile
325 330 335

agc aaa ttc aac cac cag aac att gtt cgc tgc att ggg gtg agc ctg 1056
Ser Lys Phe Asn His Gln Asn Ile Val Arg Cys Ile Gly Val Ser Leu
340 345 350

caa tcc ctg ccc cgg ttc atc ctg ctg gag ctc atg gcg ggg gga gac 1104
Gln Ser Leu Pro Arg Phe Ile Leu Leu Glu Leu Met Ala Gly Gly Asp
355 360 365

ctc aag tcc ttc ctc cga gag acc cgc cct cgc ccg agc cag ccc tcc 1152
Leu Lys Ser Phe Leu Arg Glu Thr Arg Pro Arg Pro Ser Gln Pro Ser
370 375 380

tcc ctg gcc atg ctg gac ctt ctg cac gtg gct cgg gac att gcc tgt 1200
Ser Leu Ala Met Leu Asp Leu Leu His Val Ala Arg Asp Ile Ala Cys
385 390 395 400

ggc tgt cag tat ttg gag gaa aac cac ttc atc cac cga gac att gct 1248
Gly Cys Gln Tyr Leu Glu Glu Asn His Phe Ile His Arg Asp Ile Ala
405 410 415

gcc aga aac tgc ctc ttg acc tgt cca ggc cct gga aga gtg gcc aag 1296
Ala Arg Asn Cys Leu Leu Thr Cys Pro Gly Pro Gly Arg Val Ala Lys
420 425 430

att gga gac ttc ggg atg gcc cga gac atc tac agg gcg agc tac tat 1344
Ile Gly Asp Phe Gly Met Ala Arg Asp Ile Tyr Arg Ala Ser Tyr Tyr
435 440 445

aga aag gga ggc tgt gcc atg ctg cca gtt aag tgg atg ccc cca gag 1392
Arg Lys Gly Gly Cys Ala Met Leu Pro Val Lys Trp Met Pro Pro Glu

450 455 460

gcc ttc atg gaa gga ata ttc act tct aaa aca gac aca tgg tcc ttt 1440
Ala Phe Met Glu Gly Ile Phe Thr Ser Lys Thr Asp Thr Trp Ser Phe
465 470 475 480

gga gtg ctg cta tgg gaa atc ttt tct ctt gga tat atg cca tac ccc 1488
Gly Val Leu Leu Trp Glu Ile Phe Ser Leu Gly Tyr Met Pro Tyr Pro
485 490 495

agc aaa agc aac cag gaa gtt ctg gag ttt gtc acc agt gga ggc cgg 1536
Ser Lys Ser Asn Gln Glu Val Leu Glu Phe Val Thr Ser Gly Gly Arg
500 505 510

atg gac cca ccc aag aac tgc cct ggg cct gta tac cgg ata atg act 1584
Met Asp Pro Pro Lys Asn Cys Pro Gly Pro Val Tyr Arg Ile Met Thr
515 520 525

cag tgc tgg caa cat cag cct gaa gac agg ccc aac ttt gcc atc att 1632
Gln Cys Trp Gln His Gln Pro Glu Asp Arg Pro Asn Phe Ala Ile Ile
530 535 540

ttg gag agg att gaa tac tgc acc cag gac ccg gat gta atc aac acc 1680
Leu Glu Arg Ile Glu Tyr Cys Thr Gln Asp Pro Asp Val Ile Asn Thr
545 550 555 560

gct ttg ccg ata gaa tat ggt cca ctt gtg gaa gag gaa gag aaa gtg 1728
Ala Leu Pro Ile Glu Tyr Gly Pro Leu Val Glu Glu Glu Glu Lys Val
565 570 575

cct gtg agg ccc aag gac cct gag ggg gtt cct cct ctc ctg gtc tct 1776
Pro Val Arg Pro Lys Asp Pro Glu Gly Val Pro Pro Leu Leu Val Ser
580 585 590

caa cag gca aaa cgg gag gag gag cgc agc cca gct gcc cca cca cct 1824
Gln Gln Ala Lys Arg Glu Glu Glu Arg Ser Pro Ala Ala Pro Pro Pro
595 600 605

ctg cct acc acc tcc tct ggc aag gct gca aag aaa ccc aca gct gca 1872
Leu Pro Thr Thr Ser Ser Gly Lys Ala Ala Lys Lys Pro Thr Ala Ala
610 615 620

gag gtc tct gtt cga gtc cct aga ggg ccg gcc gtg gaa ggg gga cac 1920
Glu Val Ser Val Arg Val Pro Arg Gly Pro Ala Val Glu Gly Gly His
625 630 635 640

gtg aat atg gca ttc tct cag tcc aac cct cct tcg gag ttg cac aag 1968
Val Asn Met Ala Phe Ser Gln Ser Asn Pro Pro Ser Glu Leu His Lys
645 650 655

gtc cac gga tcc aga aac aag ccc acc agc ttg tgg aac cca acg tac 2016
Val His Gly Ser Arg Asn Lys Pro Thr Ser Leu Trp Asn Pro Thr Tyr
660 665 670

ggc tcc tgg ttt aca gag aaa ccc acc aaa aag aat aat cct ata gca 2064
Gly Ser Trp Phe Thr Glu Lys Pro Thr Lys Lys Asn Asn Pro Ile Ala
675 680 685

aag aag gag cca cac gac agg ggt aac ctg ggg ctg gag gga agc tgt 2112
Lys Lys Glu Pro His Asp Arg Gly Asn Leu Gly Leu Glu Gly Ser Cys
690 695 700

act gtc cca cct aac gtt gca act ggg aga ctt ccg ggg gcc tca ctg 2160
Thr Val Pro Pro Asn Val Ala Thr Gly Arg Leu Pro Gly Ala Ser Leu
705 710 715 720

ctc cta gag ccc tct tcg ctg act gcc aat atg aag gag gta cct ctg 2208
Leu Leu Glu Pro Ser Ser Leu Thr Ala Asn Met Lys Glu Val Pro Leu
725 730 735

ttc agg cta cgt cac ttc cct tgt ggg aat gtc aat tac ggc tac cag 2256
Phe Arg Leu Arg His Phe Pro Cys Gly Asn Val Asn Tyr Gly Tyr Gln
740 745 750

caa cag ggc ttg ccc tta gaa gcc gct act gcc cct gga gct ggt cat 2304
Gln Gln Gly Leu Pro Leu Glu Ala Ala Thr Ala Pro Gly Ala Gly His
755 760 765

tac gag gat acc att ctg aaa agc aag aat agc atg aac cag cct ggg 2352
Tyr Glu Asp Thr Ile Leu Lys Ser Lys Asn Ser Met Asn Gln Pro Gly
770 775 780

ccc tga 2358
Pro

785

<210> 110
<211> 785
<212> PRT
<213> Homo sapiens

<400> 110

Met Asp Gly Phe Ala Gly Ser Leu Asp Asp Ser Ile Ser Ala Ala Ser
1               5                   10                  15

Thr Ser Asp Val Gln Asp Arg Leu Ser Ala Leu Glu Ser Arg Val Gln
            20                  25                  30

Gln Gln Glu Asp Glu Ile Thr Val Leu Lys Ala Ala Leu Ala Asp Val
        35                  40                  45

Leu Arg Arg Leu Ala Ile Ser Glu Asp His Val Ala Ser Val Lys Lys
    50                  55                  60

Ser Val Ser Ser Lys Gly Gln Pro Ser Pro Arg Ala Val Ile Pro Met
65                  70                  75                  80

Ser Cys Ile Thr Asn Gly Ser Gly Ala Asn Arg Lys Pro Ser His Thr
                85                  90                  95

Ser Ala Val Ser Ile Ala Gly Lys Glu Thr Leu Ser Ser Ala Ala Lys
            100                 105                 110

Ser Gly Thr Glu Lys Lys Lys Glu Lys Pro Gln Gly Gln Arg Glu Lys
        115                 120                 125

Lys Glu Glu Ser His Ser Asn Asp Gln Ser Pro Gln Ile Arg Ala Ser
130 135 140

Pro Ser Pro Gln Pro Ser Ser Gln Pro Leu Gln Ile His Arg Gln Thr
145 150 155 160

Pro Glu Ser Lys Asn Ala Thr Pro Thr Lys Ser Ile Lys Arg Pro Ser
165 170 175

Pro Ala Glu Lys Ser His Asn Ser Trp Glu Asn Ser Asp Asp Ser Arg
180 185 190

Asn Lys Leu Ser Lys Ile Pro Ser Thr Pro Lys Leu Ile Pro Lys Val
195 200 205

Thr Lys Thr Ala Asp Lys His Lys Asp Val Ile Ile Asn Gln Val Tyr
210 215 220

Arg Arg Lys His Gln Glu Leu Gln Ala Met Gln Met Glu Leu Gln Ser
225 230 235 240

Pro Glu Tyr Lys Leu Ser Lys Leu Arg Thr Ser Thr Ile Met Thr Asp
245 250 255

Tyr Asn Pro Asn Tyr Cys Phe Ala Gly Lys Thr Ser Ser Ile Ser Asp
260 265 270

Leu Lys Glu Val Pro Arg Lys Asn Ile Thr Leu Ile Arg Gly Leu Gly
275 280 285

His Gly Ala Phe Gly Glu Val Tyr Glu Gly Gln Val Ser Gly Met Pro
290 295 300

Asn Asp Pro Ser Pro Leu Gln Val Ala Val Lys Thr Leu Pro Glu Val
305 310 315 320

Cys Ser Glu Gln Asp Glu Leu Asp Phe Leu Met Glu Ala Leu Ile Ile
325 330 335

Ser Lys Phe Asn His Gln Asn Ile Val Arg Cys Ile Gly Val Ser Leu
340 345 350

Gln Ser Leu Pro Arg Phe Ile Leu Leu Glu Leu Met Ala Gly Gly Asp
355 360 365

Leu Lys Ser Phe Leu Arg Glu Thr Arg Pro Arg Pro Ser Gln Pro Ser
370 375 380

Ser Leu Ala Met Leu Asp Leu Leu His Val Ala Arg Asp Ile Ala Cys
385 390 395 400

Gly Cys Gln Tyr Leu Glu Glu Asn His Phe Ile His Arg Asp Ile Ala
405 410 415

Ala Arg Asn Cys Leu Leu Thr Cys Pro Gly Pro Gly Arg Val Ala Lys
420 425 430

Ile Gly Asp Phe Gly Met Ala Arg Asp Ile Tyr Arg Ala Ser Tyr Tyr
435 440 445

Arg Lys Gly Gly Cys Ala Met Leu Pro Val Lys Trp Met Pro Pro Glu
450 455 460

Ala Phe Met Glu Gly Ile Phe Thr Ser Lys Thr Asp Thr Trp Ser Phe
465 470 475 480

Gly Val Leu Leu Trp Glu Ile Phe Ser Leu Gly Tyr Met Pro Tyr Pro
485 490 495

Ser Lys Ser Asn Gln Glu Val Leu Glu Phe Val Thr Ser Gly Gly Arg
500 505 510

Met Asp Pro Pro Lys Asn Cys Pro Gly Pro Val Tyr Arg Ile Met Thr
515 520 525

Gln Cys Trp Gln His Gln Pro Glu Asp Arg Pro Asn Phe Ala Ile Ile
530 535 540

Leu Glu Arg Ile Glu Tyr Cys Thr Gln Asp Pro Asp Val Ile Asn Thr
545 550 555 560

Ala Leu Pro Ile Glu Tyr Gly Pro Leu Val Glu Glu Glu Glu Lys Val
565 570 575

Pro Val Arg Pro Lys Asp Pro Glu Gly Val Pro Pro Leu Leu Val Ser
580 585 590

Gln Gln Ala Lys Arg Glu Glu Glu Arg Ser Pro Ala Ala Pro Pro Pro
595 600 605

Leu Pro Thr Thr Ser Ser Gly Lys Ala Ala Lys Lys Pro Thr Ala Ala
610 615 620

Glu Val Ser Val Arg Val Pro Arg Gly Pro Ala Val Glu Gly Gly His
625 630 635 640

Val Asn Met Ala Phe Ser Gln Ser Asn Pro Pro Ser Glu Leu His Lys
645 650 655

Val His Gly Ser Arg Asn Lys Pro Thr Ser Leu Trp Asn Pro Thr Tyr
660 665 670

Gly Ser Trp Phe Thr Glu Lys Pro Thr Lys Lys Asn Asn Pro Ile Ala
675 680 685

Lys Lys Glu Pro His Asp Arg Gly Asn Leu Gly Leu Glu Gly Ser Cys
690 695 700

Thr Val Pro Pro Asn Val Ala Thr Gly Arg Leu Pro Gly Ala Ser Leu
705 710 715 720

Leu Leu Glu Pro Ser Ser Leu Thr Ala Asn Met Lys Glu Val Pro Leu
725 730 735

Phe Arg Leu Arg His Phe Pro Cys Gly Asn Val Asn Tyr Gly Tyr Gln
740 745 750

Gln Gln Gly Leu Pro Leu Glu Ala Ala Thr Ala Pro Gly Ala Gly His
755 760 765

Tyr Glu Asp Thr Ile Leu Lys Ser Lys Asn Ser Met Asn Gln Pro Gly
770 775 780

Pro
785

<210> 111
<211> 21
<212> DNA/RNA
<213> Artificial

<220>
<223> Description of Artificial Sequence: an artificial synthesized sense strand of siRNA-1

<220>
<221> misc_feature
<222> (1).. (13)
<223> RNA

<220>
<221> misc_feature
<222> (14).. (21)
<223> DNA

<400> 111
ugggaaagga ccuaaagtgt a 21

<210> 112
<211> 21
<212> DNA/RNA
<213> Artificial

<220>
<223> Description of Artificial Sequence: an artificial synthesized antisense strand of siRNA-1

<220>
<221> misc_feature
<222> (1) .. (6)
<223> DNA

<220>
<221> misc_feature
<222> (7).. (21)
<223> RNA

<400> 112
cactttaggu ccuuucccag g 21

<210> 113
<211> 21
<212> DNA/RNA
<213> Artificial

<220>
<223> Description of Artificial Sequence: an artificial synthesized sense strand of siRNA-2

<220>
<221> misc_feature
<222> (1).. (13)
<223> RNA

<220>
<221> misc_feature
<222> (14).. (21)

<223> DNA

<400> 113
gggaaaggac cuaaagtgta c 21

<210> 114
<211> 21
<212> DNA/RNA
<213> Artificial

<220>
<223> Description of Artificial Sequence: an artificial synthesized antisense strand of siRNA-2

<220>
<221> misc_feature
<222> (1).. (6)
<223> DNA

<220>
<221> misc_feature
<222> (7) .. (21)
<223> RNA

<400> 114
acacttuagg uccuuuccca g 21

<210> 115
<211> 21
<212> DNA/RNA
<213> Artificial

<220>
<223> Description of Artificial Sequence: an artificial synthesized sense strand of siRNA-3

<220>
<221> misc_feature
<222> (1)... (13)
<223> RNA

<220>
<221> misc_feature
<222> (14) .. (21)
<223> DNA

<400> 115
ggaccuaaag uguaccgccg g 21

<210> 116
<211> 21
<212> DNA/RNA
<213> Artificial

<220>
<223> Description of Artificial Sequence: an artificial synthesized antisense strand of siRNA-3

<220>
<221> misc_feature
<222> (1).. (6)

<223> DNA

<220>
<221> misc_feature
<222> (7).. (21)
<223> RNA

<400> 116
ggcggtacac uuuagguccu u 21

<210> 117
<211> 21
<212> DNA/RNA
<213> Artificial

<220>
<223> Description of Artificial Sequence: an artificial synthesized sense strand of siRNA-4

<220>
<221> misc_feature
<222> (1).. (13)
<223> RNA

<220>
<221> misc_feature
<222> (14) .. (21)
<223> DNA

<400> 117
ccuaaagugu accgccggaa g 21

<210> 118
<211> 21
<212> DNA/RNA
<213> Artificial

<220>
<223> Description of Artificial Sequence: an artificial synthesized antisense strand of siRNA-4

<220>
<221> misc_feature
<222> (1).. (6)
<223> DNA

<220>
<221> misc_feature
<222> (7) .. (21)
<223> RNA ...

<400> 118
tccggcggua cacuuuaggu c 21

<210> 119
<211> 21
<212> DNA/RNA
<213> Artificial

<220>

<223> Description of Artificial Sequence: an artificial synthesized sense strand of siRNA-5

<220>
<221> misc_feature
<222> (1).. (13)
<223> RNA

<220>
<221> misc_feature
<222> (14).. (21)
<223> DNA

<400> 119
aaaguguacc gccggaagca c 21

<210> 120
<211> 21
<212> DNA/RNA
<213> Artificial

<220>
<223> Description of Artificial Sequence: an artificial synthesized antisense strand of siRNA-5

<220>
<221> misc_feature
<222> (1) .. (6)
<223> DNA

<220>
<221> misc_feature
<222> (7).. (21)
<223> RNA

<400> 120
gcttccggcg guacacuuua g 21

<210> 121
<211> 21
<212> DNA/RNA
<213> Artificial

<220>
<223> Description of Artificial Sequence: an artificial synthesized sense strand of siRNA-6

<220>
<221> misc_feature
<222> (1) .. (13)
<223> RNA

<220>
<221> misc_feature
<222> (14) .. (21)
<223> DNA

<400> 121
aaguguaccg ccggaagcac c 21

<210> 122

<211> 21
<212> DNA/RNA
<213> Artificial

<220>
<223> Description of Artificial Sequence: an artificial synthesized antisense strand of siRNA-6

<220>
<221> mist_feature
<222> (1).. (6)
<223> DNA

<220>
<221> misc_feature
<222> (7).. (21)
<223> RNA

<400> 122
tgcttccggc gguacacuuu a 21

<210> 123
<211> 21
<212> DNA/RNA
<213> Artificial

<220>
<223> Description of Artificial Sequence: an artificial synthesized sense strand of siRNA-7

<220>
<221> misc_feature
<222> (1).. (13)
<223> RNA

<220>
<221> misc_feature
<222> (14) .. (21)
<223> DNA

<400> 123
ggccuguaua ccggataatg a 21

<210> 124
<211> 21
<212> DNA/RNA
<213> Artificial

<220>
<223> Description of Artificial Sequence: an artificial synthesized antisense strand of siRNA-7

<220>
<221> misc_feature
<222> (1).. (6)
<223> DNA

<220>
<221> misc_feature
<222> (7).. (21)
<223> RNA

<400> 124
attatccggu auacaggccc a 21

<210> 125
<211> 21
<212> RNA
<213> Artificial

<220>
<223> Description of Artificial Sequence: an artificial synthesized sense strand of siRNA-8

<400> 125
ggccuguaua ccggauaaug a 21

<210> 126
<211> 21
<212> RNA
<213> Artificial

<220>
<223> Description of Artificial Sequence: an artificial synthesized antisense strand of siRNA-8

<400> 126
auuauccggu auacaggccc a 21

<210> 127
<211> 21
<212> DNA/RNA
<213> Artificial

<220>
<223> Description of Artificial Sequence: an artificial synthesized sense strand of siRNA-9

<220>
<221> misc_feature
<222> (1).. (13)
<223> RNA

<220>
<221> misc_feature
<222> (14).. (21)
<223> DNA

<400> 127
cggcugcaau cgattgatag c 21

<210> 128
<211> 21
<212> DNA/RNA
<213> Artificial

<220>
<223> Description of Artificial Sequence: an artificial synthesized antisense strand of siRNA-9

<220>
<221> misc_feature
<222> (1).. (6)
<223> DNA

<220>
<221> misc_feature
<222> (7) .. (21)
<223> RNA

<400> 128
tatcaaucga uugcagccga a 21

<210> 129
<211> 2391
<212> DNA
<213> Homo sapiens

<220>
<221> CDS
<222> (1).. (2388)

<400> 129

```
atg gac ggt ttc gcc ggc agt ctc gat gat agt att tct gct gca agt      48
Met Asp Gly Phe Ala Gly Ser Leu Asp Asp Ser Ile Ser Ala Ala Ser
1               5                   10                  15

act tct gat gtt caa gat cgc ctg tca gct ctt gag tca cga gtt cag      96
Thr Ser Asp Val Gln Asp Arg Leu Ser Ala Leu Glu Ser Arg Val Gln
            20                  25                  30

caa caa gaa gat gaa atc act gtg cta aag gcg gct ttg gct gat gtt     144
Gln Gln Glu Asp Glu Ile Thr Val Leu Lys Ala Ala Leu Ala Asp Val
        35                  40                  45

ttg agg cgt ctt gca atc tct gaa gat cat gtg gcc tca gtg aaa aaa     192
Leu Arg Arg Leu Ala Ile Ser Glu Asp His Val Ala Ser Val Lys Lys
    50                  55                  60
```

tca gtc tca agt aaa ggc caa cca agc cct cga gca gtt att ccc atg 240
Ser Val Ser Ser Lys Gly Gln Pro Ser Pro Arg Ala Val Ile Pro Met
65 70 75 80

tcc tgt ata acc aat gga agt ggt gca aac aga aaa cca agt cat acc 288
Ser Cys Ile Thr Asn Gly Ser Gly Ala Asn Arg Lys Pro Ser His Thr
85 90 95

agt gct gtc tca att gca gga aaa gaa act ctt tca tct gct gct aaa 336
Ser Ala Val Ser Ile Ala Gly Lys Glu Thr Leu Ser Ser Ala Ala Lys
100 105 110

agt ggt aca gaa aaa aag aaa gaa aaa cca caa gga cag aga gaa aaa 384
Ser Gly Thr Glu Lys Lys Lys Glu Lys Pro Gln Gly Gln Arg Glu Lys
115 120 125

aaa gag gaa tct cat tct aat gat caa agt cca caa att cga gca tca 432
Lys Glu Glu Ser His Ser Asn Asp Gln Ser Pro Gln Ile Arg Ala Ser
130 135 140

cct tct ccc cag ccc tct tca caa cct ctc caa ata cac aga caa act 480
Pro Ser Pro Gln Pro Ser Ser Gln Pro Leu Gln Ile His Arg Gln Thr
145 150 155 160

cca gaa agc aag aat gct act ccc acc aaa agc ata aaa cga cca tca 528
Pro Glu Ser Lys Asn Ala Thr Pro Thr Lys Ser Ile Lys Arg Pro Ser
165 170 175

cca gct gaa aag tca cat aat tct tgg gaa aat tca gat gat agc cgt 576
Pro Ala Glu Lys Ser His Asn Ser Trp Glu Asn Ser Asp Asp Ser Arg
180 185 190

aat aaa ttg tcg aaa ata cct tca aca ccc aaa tta ata cca aaa gtt 624
Asn Lys Leu Ser Lys Ile Pro Ser Thr Pro Lys Leu Ile Pro Lys Val
195 200 205

acc aaa act gca gac aag cat aaa gat gtc atc atc aac caa gca aaa 672
Thr Lys Thr Ala Asp Lys His Lys Asp Val Ile Ile Asn Gln Ala Lys
210 215 220

atg tca act cgc aaa aaa aac agc caa gtg tac cgc cgg aag cac cag 720

Met Ser Thr Arg Lys Lys Asn Ser Gln Val Tyr Arg Arg Lys His Gln
225 230 235 240

gag ctg caa gcc atg cag atg gag ctg cag agc cct gag tac aag ctg 768
Glu Leu Gln Ala Met Gln Met Glu Leu Gln Ser Pro Glu Tyr Lys Leu
245 250 255

agc aag ctc cgc acc tcg acc atc atg acc gac tac aac ccc aac tac 816
Ser Lys Leu Arg Thr Ser Thr Ile Met Thr Asp Tyr Asn Pro Asn Tyr
260 265 270

tgc ttt gct ggc aag acc tcc tcc atc agt gac ctg aag gag gtg ccg 864
Cys Phe Ala Gly Lys Thr Ser Ser Ile Ser Asp Leu Lys Glu Val Pro
275 280 285

cgg aaa aac atc acc ctc att cgg ggt ctg ggc cat gga gcc ttt ggg 912
Arg Lys Asn Ile Thr Leu Ile Arg Gly Leu Gly His Gly Ala Phe Gly
290 295 300

gag gtg tat gaa ggc cag gtg tcc gga atg ccc aac gac cca agc ccc 960
Glu Val Tyr Glu Gly Gln Val Ser Gly Met Pro Asn Asp Pro Ser Pro
305 310 315 320

ctg caa gtg gct gtg aag acg ctg cct gaa gtg tgc tct gaa cag gac 1008
Leu Gln Val Ala Val Lys Thr Leu Pro Glu Val Cys Ser Glu Gln Asp
325 330 335

gaa ctg gat ttc ctc atg gaa gcc ctg atc atc agc aaa ttc aac cac 1056
Glu Leu Asp Phe Leu Met Glu Ala Leu Ile Ile Ser Lys Phe Asn His
340 345 350

cag aac att gtt cgc tgc att ggg gtg agc ctg caa tcc ctg ccc cgg 1104
Gln Asn Ile Val Arg Cys Ile Gly Val Ser Leu Gln Ser Leu Pro Arg
355 360 365

ttc atc ctg ctg gag ctc atg gcg ggg gga gac ctc aag tcc ttc ctc 1152
Phe Ile Leu Leu Glu Leu Met Ala Gly Gly Asp Leu Lys Ser Phe Leu
370 375 380

cga gag acc cgc cct cgc ccg agc cag ccc tcc tcc ctg gcc atg ctg 1200
Arg Glu Thr Arg Pro Arg Pro Ser Gln Pro Ser Ser Leu Ala Met Leu
385 390 395 400

gac ctt ctg cac gtg gct cgg gac att gcc tgt ggc tgt cag tat ttg 1248
Asp Leu Leu His Val Ala Arg Asp Ile Ala Cys Gly Cys Gln Tyr Leu
405 410 415

gag gaa aac cac ttc atc cac cga gac att gct gcc aga aac tgc ctc 1296
Glu Glu Asn His Phe Ile His Arg Asp Ile Ala Ala Arg Asn Cys Leu
420 425 430

ttg acc tgt cca ggc cct gga aga gtg gcc aag att gga gac ttc ggg 1344
Leu Thr Cys Pro Gly Pro Gly Arg Val Ala Lys Ile Gly Asp Phe Gly
435 440 445

atg gcc cga gac atc tac agg gcg agc tac tat aga aag gga ggc tgt 1392
Met Ala Arg Asp Ile Tyr Arg Ala Ser Tyr Tyr Arg Lys Gly Gly Cys
450 455 460

gcc atg ctg cca gtt aag tgg atg ccc cca gag gcc ttc atg gaa gga 1440
Ala Met Leu Pro Val Lys Trp Met Pro Pro Glu Ala Phe Met Glu Gly
465 470 475 480

ata ttc act tct aaa aca gac aca tgg tcc ttt gga gtg ctg cta tgg 1488
Ile Phe Thr Ser Lys Thr Asp Thr Trp Ser Phe Gly Val Leu Leu Trp
485 490 495

gaa atc ttt tct ctt gga tat atg cca tac ccc agc aaa agc aac cag 1536
Glu Ile Phe Ser Leu Gly Tyr Met Pro Tyr Pro Ser Lys Ser Asn Gln
500 505 510

gaa gtt ctg gag ttt gtc acc agt gga ggc cgg atg gac cca ccc aag 1584
Glu Val Leu Glu Phe Val Thr Ser Gly Gly Arg Met Asp Pro Pro Lys
515 520 525

aac tgc cct ggg cct gta tac cgg ata atg act cag tgc tgg caa cat 1632
Asn Cys Pro Gly Pro Val Tyr Arg Ile Met Thr Gln Cys Trp Gln His
530 535 540

cag cct gaa gac agg ccc aac ttt gcc atc att ttg gag agg att gaa 1680
Gln Pro Glu Asp Arg Pro Asn Phe Ala Ile Ile Leu Glu Arg Ile Glu
545 550 555 560

tac tgc acc cag gac ccg gat gta atc aac acc gct ttg ccg ata gaa 1728

Tyr Cys Thr Gln Asp Pro Asp Val Ile Asn Thr Ala Leu Pro Ile Glu
565 570 575

tat ggt cca ctt gtg gaa gag gaa gag aaa gtg cct gtg agg ccc aag 1776
Tyr Gly Pro Leu Val Glu Glu Glu Glu Lys Val Pro Val Arg Pro Lys
580 585 590

gac cct gag ggg gtt cct cct ctc ctg gtc tct caa cag gca aaa cgg 1824
Asp Pro Glu Gly Val Pro Pro Leu Leu Val Ser Gln Gln Ala Lys Arg
595 600 605

gag gag gag cgc agc cca gct gcc cca cca cct ctg cct acc acc tcc 1872
Glu Glu Glu Arg Ser Pro Ala Ala Pro Pro Pro Leu Pro Thr Thr Ser
610 615 620

tct ggc aag gct gca aag aaa ccc aca gct gca gag gtc tct gtt cga 1920
Ser Gly Lys Ala Ala Lys Lys Pro Thr Ala Ala Glu Val Ser Val Arg
625 630 635 640

gtc cct aga ggg ccg gcc gtg gaa ggg gga cac gtg aat atg gca ttc 1968
Val Pro Arg Gly Pro Ala Val Glu Gly Gly His Val Asn Met Ala Phe
645 650 655

tct cag tcc aac cct cct tcg gag ttg cac agg gtc cac gga tcc aga 2016
Ser Gln Ser Asn Pro Pro Ser Glu Leu His Arg Val His Gly Ser Arg
660 665 670

aac aag ccc acc agc ttg tgg aac cca acg tac ggc tcc tgg ttt aca 2064
Asn Lys Pro Thr Ser Leu Trp Asn Pro Thr Tyr Gly Ser Trp Phe Thr
675 680 685

gag aaa ccc acc aaa aag aat aat cct ata gca aag aag gag cca cac 2112
Glu Lys Pro Thr Lys Lys Asn Asn Pro Ile Ala Lys Lys Glu Pro His
690 695 700

gag agg ggt aac ctg ggg ctg gag gga agc tgt act gtc cca cct aac 2160
Glu Arg Gly Asn Leu Gly Leu Glu Gly Ser Cys Thr Val Pro Pro Asn
705 710 715 720

gtt gca act ggg aga ctt ccg ggg gcc tca ctg ctc cta gag ccc tct 2208
Val Ala Thr Gly Arg Leu Pro Gly Ala Ser Leu Leu Leu Glu Pro Ser
725 730 735

tcg ctg act gcc aat atg aag gag gta cct ctg ttc agg cta cgt cac 2256
Ser Leu Thr Ala Asn Met Lys Glu Val Pro Leu Phe Arg Leu Arg His
740 745 750

ttc cct tgt ggg aat gtc aat tac ggc tac cag caa cag ggc ttg ccc 2304
Phe Pro Cys Gly Asn Val Asn Tyr Gly Tyr Gln Gln Gln Gly Leu Pro
755 760 765

tta gaa gcc gct act gcc cct gga gct ggt cat tac gag gat acc att 2352
Leu Glu Ala Ala Thr Ala Pro Gly Ala Gly His Tyr Glu Asp Thr Ile
770 775 780

ctg aaa agc aag aat agc atg aac cag cct ggg ccc tga 2391
Leu Lys Ser Lys Asn Ser Met Asn Gln Pro Gly Pro
785 790 795

<210> 130
<211> 796
<212> PRT
<213> Homo sapiens

<400> 130

Met Asp Gly Phe Ala Gly Ser Leu Asp Asp Ser Ile Ser Ala Ala Ser
1 5 10 15

Thr Ser Asp Val Gln Asp Arg Leu Ser Ala Leu Glu Ser Arg Val Gln
20 25 30

Gln Gln Glu Asp Glu Ile Thr Val Leu Lys Ala Ala Leu Ala Asp Val
35 40 45

Leu Arg Arg Leu Ala Ile Ser Glu Asp His Val Ala Ser Val Lys Lys
50 55 60

Ser Val Ser Ser Lys Gly Gln Pro Ser Pro Arg Ala Val Ile Pro Met

65 70 75 80

Ser Cys Ile Thr Asn Gly Ser Gly Ala Asn Arg Lys Pro Ser His Thr
85 90 95

Ser Ala Val Ser Ile Ala Gly Lys Glu Thr Leu Ser Ser Ala Ala Lys
100 105 110

Ser Gly Thr Glu Lys Lys Lys Glu Lys Pro Gln Gly Gln Arg Glu Lys
115 120 125

Lys Glu Glu Ser His Ser Asn Asp Gln Ser Pro Gln Ile Arg Ala Ser
130 135 140

Pro Ser Pro Gln Pro Ser Ser Gln Pro Leu Gln Ile His Arg Gln Thr
145 150 155 160

Pro Glu Ser Lys Asn Ala Thr Pro Thr Lys Ser Ile Lys Arg Pro Ser
165 170 175

Pro Ala Glu Lys Ser His Asn Ser Trp Glu Asn Ser Asp Asp Ser Arg
180 185 190

Asn Lys Leu Ser Lys Ile Pro Ser Thr Pro Lys Leu Ile Pro Lys Val
195 200 205

Thr Lys Thr Ala Asp Lys His Lys Asp Val Ile Ile Asn Gln Ala Lys
210 215 220

Met Ser Thr Arg Lys Lys Asn Ser Gln Val Tyr Arg Arg Lys His Gln
225 230 235 240

Glu Leu Gln Ala Met Gln Met Glu Leu Gln Ser Pro Glu Tyr Lys Leu
245 250 255

Ser Lys Leu Arg Thr Ser Thr Ile Met Thr Asp Tyr Asn Pro Asn Tyr
260 265 270

Cys Phe Ala Gly Lys Thr Ser Ser Ile Ser Asp Leu Lys Glu Val Pro
275 280 285

Arg Lys Asn Ile Thr Leu Ile Arg Gly Leu Gly His Gly Ala Phe Gly
290 295 300

Glu Val Tyr Glu Gly Gln Val Ser Gly Met Pro Asn Asp Pro Ser Pro
305 310 315 320

Leu Gln Val Ala Val Lys Thr Leu Pro Glu Val Cys Ser Glu Gln Asp
325 330 335

Glu Leu Asp Phe Leu Met Glu Ala Leu Ile Ile Ser Lys Phe Asn His
340 345 350

Gln Asn Ile Val Arg Cys Ile Gly Val Ser Leu Gln Ser Leu Pro Arg
355 360 365

Phe Ile Leu Leu Glu Leu Met Ala Gly Gly Asp Leu Lys Ser Phe Leu
370 375 380

Arg Glu Thr Arg Pro Arg Pro Ser Gln Pro Ser Ser Leu Ala Met Leu
385 390 395 400

Asp Leu Leu His Val Ala Arg Asp Ile Ala Cys Gly Cys Gln Tyr Leu

405 410 415

Glu Glu Asn His Phe Ile His Arg Asp Ile Ala Ala Arg Asn Cys Leu
420 425 430

Leu Thr Cys Pro Gly Pro Gly Arg Val Ala Lys Ile Gly Asp Phe Gly
435 440 445

Met Ala Arg Asp Ile Tyr Arg Ala Ser Tyr Tyr Arg Lys Gly Gly Cys
450 455 460

Ala Met Leu Pro Val Lys Trp Met Pro Pro Glu Ala Phe Met Glu Gly
465 470 475 480

Ile Phe Thr Ser Lys Thr Asp Thr Trp Ser Phe Gly Val Leu Leu Trp
485 490 495

Glu Ile Phe Ser Leu Gly Tyr Met Pro Tyr Pro Ser Lys Ser Asn Gln
500 505 510

Glu Val Leu Glu Phe Val Thr Ser Gly Gly Arg Met Asp Pro Pro Lys
515 520 525

Asn Cys Pro Gly Pro Val Tyr Arg Ile Met Thr Gln Cys Trp Gln His
530 535 540

Gln Pro Glu Asp Arg Pro Asn Phe Ala Ile Ile Leu Glu Arg Ile Glu
545 550 555 560

Tyr Cys Thr Gln Asp Pro Asp Val Ile Asn Thr Ala Leu Pro Ile Glu
565 570 575

Tyr Gly Pro Leu Val Glu Glu Glu Glu Lys Val Pro Val Arg Pro Lys
580 585 590

Asp Pro Glu Gly Val Pro Pro Leu Leu Val Ser Gln Gln Ala Lys Arg
595 600 605

Glu Glu Glu Arg Ser Pro Ala Ala Pro Pro Pro Leu Pro Thr Thr Ser
610 615 620

Ser Gly Lys Ala Ala Lys Lys Pro Thr Ala Ala Glu Val Ser Val Arg
625 630 635 640

Val Pro Arg Gly Pro Ala Val Glu Gly Gly His Val Asn Met Ala Phe
645 650 655

Ser Gln Ser Asn Pro Pro Ser Glu Leu His Arg Val His Gly Ser Arg
660 665 670

Asn Lys Pro Thr Ser Leu Trp Asn Pro Thr Tyr Gly Ser Trp Phe Thr
675 680 685

Glu Lys Pro Thr Lys Lys Asn Asn Pro Ile Ala Lys Lys Glu Pro His
690 695 700

Glu Arg Gly Asn Leu Gly Leu Glu Gly Ser Cys Thr Val Pro Pro Asn
705 710 715 720

Val Ala Thr Gly Arg Leu Pro Gly Ala Ser Leu Leu Leu Glu Pro Ser
725 730 735

Ser Leu Thr Ala Asn Met Lys Glu Val Pro Leu Phe Arg Leu Arg His

740 745 750

Phe Pro Cys Gly Asn Val Asn Tyr Gly Tyr Gln Gln Gln Gly Leu Pro
755 760 765

Leu Glu Ala Ala Thr Ala Pro Gly Ala Gly His Tyr Glu Asp Thr Ile
770 775 780

Leu Lys Ser Lys Asn Ser Met Asn Gln Pro Gly Pro
785 790 795

<210> 131
<211> 24
<212> DNA
<213> Homo sapiens

<400> 131 24
taccagtgct gtctcaattg cagg 24

<210> 132
<211> 16
<212> DNA
<213> Homo sapiens

<400> 132 16
cacagacaaa ctccag 16

<210> 133
<211> 16
<212> DNA
<213> Homo sapiens

<400> 133
cgaccatcac cagctg 16

<210> 134
<211> 17
<212> DNA
<213> Homo sapiens

<400> 134
agaatgctac tcccacc 17

<210> 135
<211> 17
<212> DNA
<213> Homo sapiens

<400> 135
cttcacaacc tctccaa 17

<210> 136
<211> 18
<212> DNA
<213> Homo sapiens

<400> 136
aaccacaagg acagagag 18

<210> 137
<211> 18
<212> DNA
<213> Homo sapiens

<400> 137
aagtccacaa attcgagc 18

<210> 138
<211> 19
<212> DNA.
<213> Homo sapiens

<400> 138
gaatctcatt ctaatgatc 19

<210> 139
<211> 19
<212> DNA
<213> Homo sapiens

<400> 139
ttcgagcatc accttctcc 19

<210> 140
<211> 20
<212> DNA
<213> Homo sapiens

<400> 140
aaagtcacat aattcttggg 20

<210> 141
<211> 20
<212> DNA
<213> Homo sapiens

<400> 141
cccaccaaaa gcataaaacg 20

## Description

BACKGROUND OF THE INVENTION

Field of the Invention

The present invention relates to a polypeptide as a novel fusion protein, a method for detecting the fusion protein or a polynucleotide encoding the polypeptide and related materials.

Background Art

Several cancer-related genes have been known so far. In particular, tyrosine kinase genes, which encode important enzymes directly regulating cell growth, have been known to be activated even by substitution or deletion in amino acid sequences and thereby bring about carcinogenesis.

For example, NPM-ALK, fusion genes encoding NPM fused with ALK tyrosine kinase are observed in more than half of the cases of anaplastic large-cell lymphoma (ALCL) and the activation of ALK kinase has been shown to be important for tumor cell growth by NPM-ALK (Non-Patent Documents 25 and 14).

The presence of a new type of abnormal kinase has been reported to be found in approximately 10% of lung cancer cases, and this report, however, has made no reference to specific molecules (Non-Patent Document 2).

In 2000, EML4 (echinoderm microtubule-associated protein like protein 4) has been reported (Non-Patent Document 3). The EML4 protein has a basic region at the amino terminus, and further has carboxyl-terminal WD domains (Non-Patent Document 8). The physiological functions of EML4 have been little known.

On the other hand, ALK (Anaplastic Lymphoma Kinase) (Non-Patent Document 4) is receptor tyrosine kinase (Non-Patent Document 5).

Full-length ALK expression has been reported so far in some cancer cells of ectodermal origin, such as neuroblastoma, glioblastoma, breast cancer, and melanoma (the full-length ALK expression has not been observed in cancer cells of endodermal and mesodermal origins) (Non-Patent Document 13). Full-length ALK is expressed in many neuroblastoma cell lines. However, the autophosphorylation of ALK is not observed in these neuroblastoma cell lines. Moreover, ALK expression has been reported, from the cohort analysis of neuroblastoma patients, to be weakly associated with cancer. It has been suggested that ALK expression in neuroblastoma may reflect its expression in normal neural differentiation, rather than its association with cancer (Non-Patent Document 10). On the other hand, in reported cases, ligands such as pleiotrophin and midkine as well as the gene amplification of ALK itself increase the autophosphorylation of ALK and mobilize intracellular signals. It has also been reported that ALK may contribute to cancer cell growth (Non-Patent Document 12).

In some cases of human malignant lymphoma and inflammatory myofibroblastic tumor, the ALK gene has been reported to be fused with other genes (NPM, CLTCL, TFG, CARS, SEC31L1, etc.) as a result of chromosomal translocation or inversion and thereby form a fusion type of tyrosine kinase (Non-Patent Documents 9, 11, 14 to 19 and 26 to 28). Moreover, a method for identifying a protein as a fusion partner for ALK using ALK antibodies has been reported (Non-Patent Document 6). On the other hand, a fusion gene of EML4 and ALK has not been reported. Since most partner molecules have a complex formation domain, the fusion protein itself has been thought to form a complex. This complex formation has been considered to cause loss of control of the tyrosine kinase activity of ALK and induce carcinogenesis with abnormally activated intracellular signals (Non-Patent Document 10). Indeed, it has been reported that the use of ALK shRNA or ALK kinase-inhibiting compound for lymphoma cells expressing ALK fusion proteins can induce cell growth inhibition and cell death. Therefore, it has been suggested that the ALK fusion protein may serve as a therapeutic target for lymphoma and inflammatory myofibroblastic tumor (Non-Patent Documents 20 to 22). It has also been suggested that ALK may serve as a therapeutic target for other cancers whose growth involves ALK as described above (Non-Patent Documents 21 to 22).

Marzec et al. have reported that WHI-P131 and WHI-P154 (both, EMD Biosciences), which have been utilized as JAK3 tyrosine kinase-inhibiting substances, inhibit the activity of NPM-ALK (Non-Patent Document 22). Another group has reported that low-molecular-weight ALK inhibitor induces the cell death of NPM-ALK-expressing lymphoma cell lines (Non-Patent Document 21). In addition, plural low-molecular-weight compounds having an inhibitory activity against ALK have been reported so far (Non-Patent Documents 23 and 24 and Patent Documents 1 to 3).

[Patent Document 1] Pamphlet of WO 2004/080980 [Patent Document 2] Pamphlet of WO 2005/009389
[Patent Document 3] Pamphlet of WO 2005/016894
[Non-Patent Document 1] "The New England journal of medicine", (US), 2005, Vol. 353, p. 172-187
[Non-Patent Document 2] Proceedings of the 65th Annual Meeting of the Japanese Cancer Association, O-324 (issued on Aug. 28, 2006)

[Non-Patent Document 3] GenBank accession Number: NM_019063
[Non-Patent Document 4] GenBank accession Number: AB209477
[Non-Patent Document 5] Oncogene. 1997 Jan 30; 14 (4): 439-49
[Non-Patent Document 6] PNAS 2006 103, 7402-7407
[Non-Patent Document 7] "Seminars in oncology", (US), 1993, Vol. 20, p. 105-127
[Non-Patent Document 8] "Genomics", (US), 2000, Vol. 68, p. 348-350
[Non-Patent Document 9] Oncogene 9: 1567-1574, 1994
[Non-Patent Document 10] "Cellular and molecular life sciences", (Switzerland), 2004, Vol. 61, p. 2939-2953
[Non-Patent Document 11] Am J Pathol 160: 1487-1494, 2002
[Non-Patent Document 12] "Journal of cellular physiology", (US), 2004, Vol. 199, p. 330-358
[Non-Patent Document 13] "International journal of cancer", (US), 2002, Vol. 100, p. 49-56
[Non-Patent Document 14] "Science", (US), 1994, Vol. 263, p. 1281-1284
[Non-Patent Document 15] "Blood", (US), 1995, Vol. 86, p. 1954-1960
[Non-Patent Document 16] "Blood", (US), 2000, Vol. 95, p. 3204-3207
[Non-Patent Document 17] "Blood", (US), 1999, Vol. 94, p. 3265-3268
[Non-Patent Document 18] "Laboratory investigation; a journal of technical methods and pathology", (US), 2003, Vol. 83, p. 1255-1265
[Non-Patent Document 19] "International journal of cancer", (US), 2006, Vol. 118, p. 1181-1186
[Non-Patent Document 20] "Blood", (US), 2006, Vol. 107, p. 689-697
[Non-Patent Document 21] "Blood", (US), 2006, Vol. 107, p. 1617-1623
[Non-Patent Document 22] "Laboratory investigation; a journal of technical methods and pathology", (US), 2005, Vol. 85, p. 1544-1554
[Non-Patent Document 23] "Journal of medicinal chemistry", (US), 2006, Vol. 49, p. 1006-1015
[Non-Patent Document 24] J Comb Chem. 8: 401-409, 2006
[Non-Patent Document 25] "Science", (US), 1997, Vol. 278, p. 1309-1312
[Non-Patent Document 26] Am J Pathol 157: 377-384, 2000
[Non-Patent Document 27] Blood 90: 2901-2910, 1997
[Non-Patent Document 28] Am J Pathol. 2000 Mar; 156 (3): 781-9

SUMMARY OF THE INVENTION

The present inventors successfully isolated, from samples obtained from lung cancer patients, the cDNA and genomic DNA of a novel fusion polynucleotide of a partial EML4 gene fused with a partial ALK gene as kinase ( hereinafter, referred to as an EML4-ALK fusion polynucleotide v1), which is produced by chromosomal inversion (Examples 1, 2, and 4(1)). The present inventors also successfully isolated the cDNA and genomic DNA of a novel fusion polynucleotide (hereinafter, referred to as an EML4-ALK fusion polynucleotide v2, EML4-ALK fusion polynucleotide v3) whose fused regions are different from those in the EML4-ALK fusion polynucleotide v1 (Examples 4(1), 3(3), 11(1) and (7)). Analysis using clinical samples showed that the EML4-ALK fusion polynucleotide v1, EML4-ALK fusion polynucleotide v2 or EML4-ALK fusion polynucleotide v3 is present in some lung cancer patients (Example 3 and 11(1)). On the other hand, since the EML4-ALK fusion polynucleotide is an oncogene that exhibits tumorigenicity depending on its kinase activity (Example 6,10(3) and 11(2)), it was revealed that the EML4-ALK fusion polypeptide is a tool for screening a therapeutic agent for cancer that is shown to be positive for the fusion polynucleotide. Based on these findings, the present inventors constructed a method for detecting the fusion polynucleotide or fusion protein in a sample obtained from a test subject (Examples 3, 4(2), 5(2), 9, and 11(3)) and, subsequently, a method for screening an inhibitor of the fusion polynucleotide and/or the fusion polypeptide (i.e., a therapeutic agent for cancer that is shown to be positive for the fusion polynucleotide) causative of cancer (Examples 7, 10(2), 11(4), 11(5) and 11(8)), and confirmed that compounds obtained by screening exhibit an anti-tumor effect (Examples 8(3), 8(7), 8(8),11(6) and 11(9)). As a result, a test subject from which the fusion polynucleotide has been detected can receive cancer treatment using the inhibitor of the fusion polynucleotide and/or the polypeptide encoded thereby. According to the detection method, subjects to which the therapeutic agent is applicable can be selected. As a result, tailor-made medical care expected as highly effective treatment using the inhibitor can be carried out.

Based on these findings, the present inventors disclose herein a novel polynucleotide and polypeptide useful as screening tools, a screening method, and a pharmaceutical composition for treatment of cancer that is shown to be positive for the fusion gene of EML4 gene and ALK gene. The present inventors further disclose a detection method useful in the detection of cancer that is shown to be positive for the fusion gene of EML4 gene and ALK gene.

Specifically, the present invention relates to: methods, kits and primer sets as defined in the claims.

None of above-mentioned documents have reported the formation of a fusion gene by EML4 gene and ALK gene, let alone the expression of the fusion gene of EML4 gene and ALK gene in some cancer patients. The formation of a fusion gene by EML4 gene and ALK gene and the expression of this fusion gene in some cancer patients were found for the first time by the present inventors. The screening method using a fusion gene of EML4 gene and ALK gene is an invention that was made for the first time by the present inventors. Moreover, the method for detecting the fusion gene useful in the detection of cancer that is shown to be positive for the fusion gene , the primer set useful in this detection, and the kit for detection are inventions that were provided for the first time by the findings of the present inventors. Various ALK inhibitors (Patent Documents 3 to 4 and Non-Patent Documents 20 to 22 and 34) including 5-chloro-*N*4-[2-(isopropylsulfonyl)phenyl]-*N*2-{2-methoxy-4-[4-(4-methylpiperazin-1-yl)piperidin-1-yl]phenyl}pyrimidine-2,4-diamine and 2-[(5-bromo-2-{[2-methoxy-4-(4-methylpiperazin-1-yl)phenyl]amino) pyrimidin-4-yl)amino]-*N*-methylbenzenesulfonamide have been reported. Furthermore ALK inhibitors have been reported to induce growth inhibition and cell death in lymphoma cells expressing NPM-ALK fusion proteins (Non-Patent Documents 20 to 22) and inhibit lymphoma (PNAS, 2007, Jan 2, 104 (1), 270-275 Epub2006 Dec). However, it has totally been unknown that these ALK inhibitors have therapeutic applications for cancer (particularly, lung cancer) that is shown to be positive for a fusion gene of EML4 gene and ALK gene. The pharmaceutical composition for treatment of cancer (particularly, lung cancer) that is shown to be positive for a fusion gene of EML4 gene and ALK gene is a disclosure that was provided for the first time by the findings of the present inventors.

The polypeptide, polynucleotide, expression vector, and cell of the present disclosure can be used in the screening of a substance inhibiting the polypeptide of the present disclosure (particularly, a therapeutic agent for lung cancer that is shown to be positive for a fusion gene of EML4 gene and ALK gene). Subjects for which a fusion gene of EML4 gene and ALK gene is positive (particularly, lung cancer patients) can be detected by using the presence of the polypeptide and/or polynucleotide of the present disclosure as an index. According to the screening method of the present disclosure a therapeutic agent for cancer (particularly, a therapeutic agent for lung cancer) that is shown to be positive for a fusion gene of EML4 gene and ALK gene can be screened. The primer and kit for detection of the present invention can be utilized for detecting a cancer that is shown to be positive for a fusion gene of EML4 gene and ALK gene.. The detection method of the present invention can be utilized as a method for detecting cancer (particularly, lung cancer) that is shown to be positive for the fusion gene of EML4 gene and ALK gene of the present disclosure. Moreover, according to the detection method of the present invention, whether or not the therapeutic agent of the present disclosure is applicable to subjects can be determined. The substance inhibiting the polypeptide of the present disclosure is useful as a therapeutic agent for cancer, particularly lung cancer, that is shown to be positive for a fusion gene of EML4 gene and ALK gene.

BRIEF DESCRIPTION OF THE DRAWINGS

FIG. 1 shows the results of PCR. The left lane (46, XX) shows the result when the genomic DNA from a normal healthy subject was used as a substrate, and the right lane (ID #33) shows the result when the genomic DNA from a cancer patient was used as a substrate;

FIG. 2 shows the results of the screening for EML4-ALK fusion polynucleotide in specimens of lung cancer patients. Lane "46, XX" shows the result of using peripheral monocytes of a normal healthy female subject, and "ID #2" to "ID #42" show the result of using samples obtained from excised specimens from lung cancer patients. In addition, lane "NTC" shows the result without added substrate cDNA. Lane "marker" is the lane where the size marker DNA was electrophoresed (upper section). The results of amplification of GAPDH cDNA are shown in the lower section. Sex (M, male; F, female), pathology (S, squamous cell carcinoma; A, adenocarcinoma; AS, adenosquamous carcinoma; B, bronchiolo-aleveolar carcinoma) and the presence or absence of EGFR mutation and the presence or absence of smoking history are shown in the upper part of the figure;

FIG. 3 shows tumorgenicity of the genes. The upper section of the figure (3T3) shows 3T3 fibroblast cells when a blank vector (Vector), and expression plasmid such as full length ALK/pMXS (ALK), EML4-ALKv1/pMXS (EML4-ALK) or EML4-ALK (K589M)/pMXS were introduced The scale bar represents 100 $\mu$m. The lower section of the figure (Nude mice) shows the result of the inoculation of each 3T3 fibroblast cell line to nude mice;

FIG. 4 shows the inhibitory effect of a EML4-ALK fusion polypeptide inhibitor (compound A) on intracellular autophosphorylation. "KM" indicates when EML4-ALK (K589M) expressing cells were used, and "EA" shows when v1 expressing BA/F3 cells were used. "$\alpha$p-ALK" (upper panel) shows the result of the immunoblotting when anti-phosphorylated ALK antibody was used, and "$\alpha$FLAG" (lower panel) shows the result of the immunoblotting when anti-FLAG antibody was used;

FIG. 5 shows the growth potential of cells which express CD8 protein only (CD8), or co-express CD8 and ALK (ALK), CD8 and EML4-ALK fusion polypeptide v1 (EA) or CD8 and EML4-ALK (K589M) (KM) in the presence (+IL-3) or absence (-IL-3) of IL-3. The horizontal axis of the figure is time course (Days) and the vertical axis is the cell number;

FIG. 6(a) shows time dependent change of cell number when respective concentrations of compound A were added to BA/F3 cells expressing only CD8 and cultured in the presence of IL-3. (b) shows time dependent change of cell number when v1 expressing BA/F3 cells were cultured with respective concentration of compound A in the absence of IL-3. The horizontal axis of the figure is time course (Days) and the vertical axis is the cell number; and

FIG. 7 shows siRNAI-siRNA9.

## DETAILED DESCRIPTION OF THE PREFERRED EMBODIMENTS

Hereinafter, the present invention will be described in detail. Gene manipulation techniques described herein can be practiced according to techniques known in the art, such as "Molecular Cloning", Sambrook, J et al., Cold Spring Harbor Laboratory Press, 1989, unless otherwise specified. Protein manipulation techniques described herein can be practiced according to techniques known in the art, such as "Experimental Protocol on Proteins", Shujunsha Co. Ltd. 1997, unless otherwise specified.

The phrase "the polypeptide of the present disclosure is inhibited" described herein encompasses both the phrases "the expression of the polypeptide of the present disclosure is inhibited" and "the activity of the polypeptide of the present disclosure is inhibited". A "substance inhibiting the polypeptide of the present disclosure" encompasses both a "substance inhibiting the expression of the polypeptide of the present disclosure" and a "substance inhibiting the activity of the polypeptide of the present disclosure".

<Polypeptide, polynucleotide, expression vector, transformed cell, and methods for producing polypeptide of the present disclosure>

The polypeptide of the present disclosure encompasses:

(1) a polypeptide consisting of the amino acid sequence represented by SEQ ID NO: 2 , 7 or 130;

(2)

(a) a polypeptide comprising the amino acid sequence represented by SEQ ID NO: 2 and having a kinase activity, or a polypeptide comprising an amino acid sequence derived from the amino acid sequence represented by SEQ ID NO: 2 with the substitution, deletion, and/or insertion of 1 to 10 amino acids and having a kinase activity (hereinafter, referred to as a v1 functionally equivalent modified polypeptide);

(b) a polypeptide comprising the amino acid sequence represented by SEQ ID NO: 7 and having a kinase activity, or a polypeptide comprising an amino acid sequence derived from the amino acid sequence represented by SEQ ID NO: 7 with the substitution, deletion, and/or insertion of 1 to 10 amino acids and having a kinase activity (hereinafter, referred to as a v2 functionally equivalent modified polypeptide); and

(c) a polypeptide comprising the amino acid sequence represented by SEQ ID NO: 130 and having a kinase activity, or a polypeptide comprising an amino acid sequence derived from the amino acid sequence represented by SEQ ID NO: 130 with the substitution, deletion, and/or insertion of 1 to 10 amino acids and having a kinase activity (hereinafter, referred to as a v3 functionally equivalent modified polypeptide);

(hereinafter, the v1 functionally equivalent modified polypeptide, v2 functionally equivalent modified polypeptide, and v3 functionally equivalent modified polypeptide are collectively referred to as a functionally equivalent modified polypeptide); and

(3)

(a) a polypeptide comprising an amino acid sequence with 90% or higher identity to the amino acid sequence represented by SEQ ID NO: 2 and having a kinase activity (hereinafter, referred to as a v1 homologous polypeptide);

(b) a polypeptide comprising an amino acid sequence with 90% or higher identity to the amino acid sequence represented by SEQ ID NO: 7 and having a kinase activity (hereinafter, referred to as a v2 homologous polypeptide); and

(c) a polypeptide comprising an amino acid sequence with 90% or higher identity to the amino acid sequence represented by SEQ ID NO: 130 and having a kinase activity (hereinafter, referred to as a v3 homologous polypeptide);

(hereinafter the v1 homologous polypeptide, v2homologous polypeptide and v3 homologous polypeptide are collectively referred to as a homologous polypeptide).

The "functionally equivalent modified polypeptide" is, preferably, the "polypeptide comprising an amino acid sequence derived from the amino acid sequence represented by SEQ ID NO: 2, 7 or 130 with the substitution, deletion, and/or insertion of 1 to 10, preferably 1 to several, more preferably 1 to 7, most preferably 1 to 5 amino acids and having a kinase activity". More preferably polypeptide is the "polypeptide comprising the amino acid sequence represented by SEQ ID NO: 2, 7 or 130 and having a kinase activity".

The "homologous polypeptide" is a "polypeptide comprising an amino acid sequence with 90% or higher identity to the amino acid sequence represented by SEQ ID NO: 2, 7 or 130 and having a kinase activity". Preferably, the homologous polypeptide has an amino acid sequence with 95% or higher, more preferably 98% or higher identity thereto.

The "identity" described herein means a value Identity obtained by NEEDLE program (J Mol Biol 1970; 48: 443-453) search using parameters prepared as defaults. The parameters are as follows:

Gap penalty = 10
Extend penalty = 0.5
Matrix = EDNAFULL

The phrase "having a kinase activity" means having an activity as an enzyme phosphorylating tyrosine. Whether a certain polypeptide "has a kinase activity" is confirmed by a method of Example 7(2). More preferably, functionally equivalent modified polypeptide and homologous polypeptide have combined kinase activity and tumorigenicity. Whether a certain polypeptide "has a tumorigenicity " is confirmed by a method of Example 6(1).

Up to this point, the polypeptide of the present disclosure has been described. Hereinafter, the polypeptide consisting of the amino acid sequence represented by SEQ ID NO: 2, 7 or 130, the functionally equivalent modified polypeptide, and the homologous polypeptide are collectively referred to as a "polypeptide of the present disclosure". Of the polypeptides of the present disclosure, the polypeptide consisting of the amino acid sequence represented by SEQ ID NO: 2, the v1 functionally equivalent modified polypeptide, and the v1 homologous polypeptide are collectively referred to as a "polypeptide type v1 of the present disclosure". The polypeptide consisting of the amino acid sequence represented by SEQ ID NO: 7, the v2 functionally equivalent modified polypeptide, and the v2 homologous polypeptide are collectively referred to as a "polypeptide type v2 of the present disclosure". The polypeptide consisting of the amino acid sequence represented by SEQ ID NO: 130, the v3 functionally equivalent modified polypeptide, and the v3 homologous polypeptide are collectively referred to as a "polypeptide type v3 of the present disclosure". A protein as the polypeptide consisting of the amino acid sequence represented by SEQ ID NO: 2 is referred to as an "EML4-ALK fusion polypeptide v1". A protein as the polypeptide consisting of the amino acid sequence represented by SEQ ID NO: 7 is referred to as an "EML4-ALK fusion polypeptide v2". A protein as the polypeptide consisting of the amino acid sequence represented by SEQ ID NO: 130 is referred to as an "EML4-ALK fusion polypeptide v3". The "EML4-ALK fusion polypeptide v1 ", the "EML4-ALK fusion polypeptide v2" and the "EML4-ALK fusion polypeptide v3 "are collectively referred to as an "EML4-ALK fusion polypeptide".

The polypeptide of the present disclosure is, preferably, the "polypeptide comprising the amino acid sequence represented by SEQ ID NO: 2, 7 or 130 and having a kinase activity ", more preferably, the "polypeptide comprising the amino acid sequence represented by SEQ ID NO: 2, 7 or 130 and having a kinase activity and tumorigenicity ", most preferably, the "polypeptide consisting of the amino acid sequence represented by SEQ ID NO: 2, 7 or 130".

A polynucleotide encoding the polypeptide of the present disclosure (hereinafter, referred to as a "polynucleotide of the present disclosure") is a polynucleotide represented by a nucleotide sequence encoding the EML4-ALK fusion polypeptide, functionally equivalent modified polypeptide, or homologous polypeptide. The polynucleotide of the present disclosure is, preferably, a polynucleotide consisting of a nucleotide sequence encoding the amino acid sequence represented by SEQ ID NO: 2, 7 or 130, more preferably, a polynucleotide consisting of the nucleotide sequence represented by SEQ ID NO: 1 (particularly preferably, positions 271 to 3447 in SEQ ID NO: 1), 6 or 129.

Of the polynucleotides of the present disclosure, a gene encoding the polypeptide type v1 of the present invention is referred to as a "polynucleotide type v1 of the present disclosure". A gene encoding the polypeptide type v2 of the present disclosure is referred to as a "polynucleotide type v2 of the present disclosure". A gene encoding the polypeptide type v3 of the present disclosure is referred to as a "polynucleotide type v3 of the present disclosure".

A "fusion gene of EML4 gene and ALK gene" described herein refers to the polynucleotide of the present disclosure. A gene encoding the EML4-ALK fusion polypeptide v1, which is a polynucleotide type v1 of the present disclosure, is referred to as an "EML4-ALK fusion polynucleotide v1". A gene encoding the EML4-ALK fusion polypeptide v2, which is a polynucleotide type v2 of the present disclosure, is referred to as an "EML4-ALK fusion polynucleotide v2". A gene encoding the EML4-ALK fusion polypeptide v3, which is a polynucleotide type v3 of the present disclosure, is referred to as an "EML4-ALK fusion polynucleotide v3". The EML4-ALK fusion polynucleotide v1, the EML4-ALK fusion polynucleotide v2 and the EML4-ALK fusion polynucleotide v3 are collectively referred to as an "EML4-ALK fusion polynucleotide".

The phrase "cancer that is shown to be positive for a fusion gene of EML4 gene and ALK gene" described herein means cancer positive for the polynucleotide of the present disclosure (i.e., the polynucleotide of the present disclosure is present) and, preferably, means cancer positive for the EML4-ALK fusion polynucleotide (i.e., the EML4-ALK fusion polynucleotide-positive cancer) (i.e., the EML4-ALK fusion polynucleotide is present).

Methods for producing the polynucleotide of the present disclosure include, but not particularly limited to, (1) a method using polymerase chain reaction (PCR), (2) a method using a standard genetic engineering approach (i.e., a method comprising selecting a transformed strain comprising desired amino acid sequence from strains transformed with a cDNA library), and (3) a chemical synthesis method. Each method can be practiced in the same way as in WO 01/34785. However, the "novel protein of the present invention" described therein can be interpreted as a protein consisting of the polypeptide of the present disclosure described herein, and the "gene of the present invention" described therein can be interpreted as the polynucleotide of the present disclosure described herein.

In the method using PCR, the polynucleotide of the present disclosure can be produced, for example, according to procedures described in 1) Production method of protein gene, a) First production method in "Embodiments of the Invention" of the patent document. mRNA is extracted from cells or tissues having the ability to produce the protein of the present disclosure, for example, from lung tissues derived from a human patient with lung cancer. Subsequently, this mRNA can be subjected to reverse transcriptase reaction in the presence of random primers or oligo dT primers to synthesize single-stranded cDNA. The obtained single-stranded cDNA can be subjected to PCR using 2 primers interposing a partial region of the gene of interest to obtain the polynucleotide of the present disclosure or a portion thereof. More specifically, the polynucleotide of the present disclosure can be produced, for example, by a method described in Example 4(1), 11 (1) or 11 (7).

Alternatively, the polynucleotide of the present disclosure may be produced by artificially synthesizing the polynucleotide of the present disclosure as separated fragments by reverse transcription (RT)-PCR and then fusing these obtained fragments. For example, (a) 1489 bases located from exon 1 to exon 13 in EML4 (for the polynucleotide type v1 of the present invention) or 2242 bases located from exon 1 to exon 20 in EML4 (for the polynucleotide type v2 of the present invention) are amplified by RT-PCR using, as a template, mRNA extracted from cells (e.g., HeLa cells) or tissues endogenously expressing EML4 and using 2 primers interposing the gene region of interest. On the other hand, for example, (b) 1691 bases located from exon 21 to exon 30 in ALK (for both the polynucleotide type v1 of the present disclosure and the polynucleotide type v2 of the present disclosure) are amplified by RT-PCR using, as a template, mRNA extracted from cells (e.g., Rh30 or U-87MG cells) or tissues endogenously expressing ALK and using 2 primers interposing the gene region of interest. The amplified PCR products of (a) and (b) can be fused to obtain the polynucleotide of the present disclosure. This fusion can be practiced by devising the primers used in the RT-PCR of (a) and (b). For example, a primer is created by adding approximately 10 bases of the 5'-terminal antisense nucleotide sequence of exon 21 in ALK to the 5' terminus of an antisense primer for the RT-PCR amplification of the fragment of (a), and this created primer is used as an antisense primer to amplify the fragment of (a). Moreover, a primer is created by adding approximately 10 bases of the 3'-terminal sense nucleotide sequence of exon 13 in EML4 (for the polynucleotide type v1 of the present disclosure) or approximately 10 bases of the 3'-terminal sense nucleotide sequence of exon 20 in EML4 (for the polynucleotide type v2 of the present disclosure) to the 5' terminus of a sense primer for the RT-PCR amplification of the fragment of (b), and this created primer is used as a sense primer to amplify the fragment of (b). The polynucleotide of the present disclosure can be obtained by PCR using, as templates, these 2 kinds of PCR products obtained and using a sense primer comprising the initiation codon of EML4 and an antisense primer comprising the stop codon of ALK. Alternatively, the polynucleotide of the present disclosure can also be obtained by annealing and extension reactions using only these 2 kinds of PCR products obtained without using the sense primer comprising the initiation codon of EML4 and the antisense primer comprising the stop codon of ALK.

The expression vector of the present disclosure, transformed cell of the present disclosure, and method for producing polypeptide of the present disclosure can be practiced, for example, according to procedures described in 2) Methods for the production of the vector of the invention, the host cell of the invention and the recombinant protein of the invention in " Mode for Carrying Out the Invention " of WO 01/34785. The isolated polynucleotide of the present disclosure can be incorporated again into appropriate vector DNA to thereby transform a eukaryotic or prokaryotic host cell therewith. Alternatively, an appropriate promoter and a sequence involved in phenotypic expression may be introduced to the vector to thereby cause each host cell transformed therewith to express the polynucleotide.

The expression vector of the present disclosure is not particularly limited as long as it comprises the polynucleotide of the present disclosure and it expresses the polypeptide of the present disclosure. Examples thereof can include an expression vector obtained by inserting the polynucleotide of the present disclosure into an expression vector known in the art, which is appropriately selected according to a host cell used.

Likewise, the cell of the present disclosure is not particularly limited as long as it comprises the polynucleotide of the present disclosure as a result of nucleic acid transfer by transfection or infection with the expression vector of the present disclosure. For example, the cell of the present disclosure is a host cell comprising the polynucleotide of the present disclosure incorporated in the chromosome or is a cell comprising the expression vector comprising the polynucleotide of the present disclosure. The cell of the present disclosure is obtained, for example, by transfecting or infecting a desired cell with the expression vector of the present disclosure More specifically, for example, the expression vector comprising the polynucleotide of the present disclosure and a plasmid for packaging (e.g., pGP or pE-eco) can be introduced into a BOSC23 cell by use of a commercially available transfection reagent Lipofectamine, as described in Example 1, to thereby produce an expression retrovirus. A BA/F3 cell can be infected with this retrovirus to thereby produce the transformed cell of the present disclosure.

The desired transformed cell thus obtained can be cultured according to a standard method. A protein consisting of the polypeptide of the present disclosure is produced by this culture. A medium used in the culture can be selected appropriately according to a host cell used from among a variety of routine media. For example, an RPMI1640 medium supplemented with serum components such as fetal bovine serum (FBS) is used for the BA/F3 cell.

The polypeptide of the present disclosure thus produced by the transformed cell can be separated and purified by a variety of separation operation techniques know in the art using the physical or biochemical properties of the polypeptide.

The polypeptide of the present disclosure can be fused in frame with a marker sequence and expressed to thereby achieve the confirmation of expression of the protein as well as the purification of the protein.

<Primer set of the present invention>

The present invention encompasses a primer set useful in the detection of the presence of the polynucleotide of the present invention.

Specifically, the present invention encompasses:

(1) a primer set for detecting the polynucleotide of the present disclosure, comprising an antisense primer consisting of nucleic acid molecules with at least 16 consecutive bases hybridizing under stringent conditions (preferably, more stringent conditions) to i) a polynucleotide type v1 of the present disclosure (preferably, EML4-ALK fusion polynucleotide v1, more preferably, a polynucleotide consisting of the nucleotide sequence represented by SEQ ID NO: 1), a polynucleotide type v2 of the present disclosure (preferably, EML4-ALK fusion polynucleotide v2, more preferably, a polynucleotide consisting of the nucleotide sequence represented by SEQ ID NO: 6), a polynucleotide type v3 of the present disclosure (preferably, EML4-ALK fusion polynucleotide v3, more preferably, a polynucleotide consisting of the nucleotide sequence represented by SEQ ID NO: 129), ii) a polynucleotide consisting of the nucleotide sequence represented by SEQ ID NO: 4, which is a genomic sequence comprising the fusion point of a polynucleotide belonging to EML4-ALK fusion polynucleotide v1, and/or iii) a polynucleotide consisting of the nucleotide sequence represented by SEQ ID NO: 5, which is one of sequences comprising the fusion point of a polynucleotide belonging to EMK4-ALK fusion polynucleotide v2, and a sense primer consisting of a nucleic acid molecule with at least 16 consecutive bases hybridizing under stringent conditions (preferably, more stringent conditions) to complementary strands to the any of above i) to iii);

(2) a primer set of a sense primer comprising an oligonucleotide with at least any 16 consecutive bases located at base Nos. 1 to 1759 (preferably, base Nos. 271 to 1759) in SEQ ID NO: 1 and an antisense primer comprising an oligonucleotide complementary to an oligonucleotide with at least any 16 consecutive bases located at base Nos. 1760 to 3926 (preferably, base Nos. 1760 to 3447) in SEQ ID NO: 1, or a primer set consisting of complementary strands thereof, wherein the spacing between the selected positions of the sense and antisense primers in SEQ ID NO: 1 is 1 kb or less, or wherein the sense and antisense primers give amplification products of 1 kb or less in size;

(3) a primer set of a sense primer comprising an oligonucleotide with at least any 16 consecutive bases located at base Nos. 1 to 2242 in SEQ ID NO: 6 and an antisense primer comprising an oligonucleotide complementary to an oligonucleotide with at least any 16 consecutive bases located at base Nos. 2243 to 3933 in SEQ ID NO: 6, or a primer set consisting of complementary strands thereof, wherein the spacing between the selected positions of the sense and antisense primers in SEQ ID NO: 6 is 1 kb or less, or wherein the sense and antisense primers give amplification products of 1 kb or less in size;

(4) a primer set of a sense primer comprising an oligonucleotide with at least any 16 consecutive bases located at base Nos. 1 to 3629 in SEQ ID NO: 4 and an antisense primer comprising an oligonucleotide complementary to an oligonucleotide with at least any 16 consecutive bases located at base Nos. 3630 to 3979 in SEQ ID NO: 4, or a primer set consisting of complementary strands thereof, wherein the spacing between the selected positions of the sense and antisense primers in SEQ ID NO: 4 is 1 kb or less, or wherein the sense and antisense primers give amplification products of 1 kb or less in size;

(5) a primer set of a sense primer comprising an oligonucleotide with at least any 16 consecutive bases located at base Nos. 1 to 579 in SEQ ID NO: 5 and an antisense primer comprising an oligonucleotide complementary to an oligonucleotide with at least any 16 consecutive bases located at base Nos. 580 to 853 in SEQ ID NO: 5, or a primer set consisting of complementary strands thereof; and

(6) a primer set of a sense primer comprising an oligonucleotide with at least any 16 consecutive bases located at base Nos. 1 to 700 in SEQ ID NO: 129 and an antisense primer comprising an oligonucleotide complementary to an oligonucleotide with at least any 16 consecutive bases located at base Nos. 701 to 1691 in SEQ ID NO: 129, or a primer set consisting of complementary strands thereof, wherein the sense and antisense primers give amplification products of 1 kb or less in size. Examples of a preferable primer set include:

(7) the primer set or the primer set consisting of complementary strands thereof according to any of (3) to (6), wherein the sense and antisense primers are represented by (i) SEQ ID NOs: 8 and 9, (ii) SEQ ID NOs: 15 and 16, 17 and 18, 19 and 20, 21 and 22, 23 and 24, 25 and 26, 27 and 28, 29 and 30, 31 and 32, or 33 and 34, (iii) SEQ ID NOs: 35 and 36, 37 and 38, 39 sand 18, 41 and 20, 43 and 22, 45 and 24, 47 and 26, 49 and 28, 51 and 52, 53 and 54, or 55 and 34, (iv) SEQ ID NOs: 61 and 62, 63 and 64, 65 and 66, 67 and 68, 69 and 70, 71 and 72, 73 and 74, 75 and 76, 77 and 78, or 79 and 80, (v) SEQ ID NOs: 81 and 62, 83 and 84, 85 and 68, 87 and 88, 89 and 70, 91 and 92, 93 and 74, 95 and 96, 97 and 98, or 99 and 100, or (vi) SEQ ID NOs: 131 and 82, 132 and 62, 133 and 64, 134 and 66, 135 and 68, 136 and 70, 137 and 72, 138 and 74, 139 and 76, 140 and 78, or 141 and 80.

The primer sets (7) (i) (in Example 3(1)), (7) (ii) (in Example 3(2)), (7) (iii) (in Example 3(3)), and (7) (iv) and (v) (in Example 4(2)), and (7) (vi) (in Example 11(3)) were used for detecting the presence of the polynucleotide of the present disclosure.

Fusion point in this specification means a point where a portion derived from EML4 gene and a portion derived from ALK gene are fused. Fusion point in SEQ ID No: 1 is the point where a nucleotide of base position 1759 and a nucleotide of base position 1760 are fused. Fusion point in SEQ ID No: 6 is the point where a nucleotide of base position 2242 and a nucleotide of base position 2243 are fused. Fusion point in SEQ ID No: 129 is the point where a nucleotide of base position 700 and a nucleotide of base position 701 are fused. Fusion point in SEQ ID No: 4 is the point where a nucleotide of base position 3629 and a nucleotide of base position 3630 are fused. Fusion point in SEQ ID No: 5 is the point where a nucleotide of base position 579 and a nucleotide of base position 580 are fused.

The "stringent conditions" in this specification comprise hybridization conditions on the order of "5 x SSPE, 5 $\times$ Denhardt's solution, 0.5% SDS, 50% formamide, 200 $\mu$g/ml salmon sperm DNA, 42°C overnight" and washing conditions on the order of "0.5 $\times$ SSC, 0.1 % SDS, 42°C". The "more stringent conditions" comprise hybridization conditions on the order of "5 x SSPE, 5 $\times$ Denhardt's solution, 0.5% SDS, 50% formamide, 200 $\mu$g/ml salmon sperm DNA, 42°C overnight" and washing conditions on the order of "0.2 x SSC, 0.1% SDS, 65°C".

The primer sets of the present invention can be utilized as primer sets for amplifying and detecting the polynucleotide of the present disclosure. For primer use, its chain length is usually 15 to 40 bases, preferably 16 to 24 bases, more preferably 18 to 24 bases, particularly preferably 20 to 24 bases.

Methods for producing primers of the present invention are not particularly limited. The primers of the present invention can be produced by the chemical synthesis method used for the method for producing the polynucleotide of the present disclosure.

<Detection method and kit for detection of the present invention>

The present invention encompasses a method for detecting the polynucleotide of the present disclosure and a method for detecting a fusion protein consisting of the polypeptide of the present disclosure. Specifically, an aspect comprising the step below is exemplified. Specifically, the method for detecting the polynucleotide of the present disclosure comprises the step of

(1) detecting the presence of the polynucleotide of the present disclosure in a sample obtained from a test subject.

A sample collected from a test subject (a sample separated from the body), specifically, any collected body fluid (preferably, blood), bronchoalveolar lavage fluid, biopsied sample, or sputum sample is used as the sample obtained from a test subject. Preferably, a biopsy sample of the affected part of the lung of the test subject or a sputum sample of the test subject is used. Genomic DNA extracted from the sample or a transcription product (product as a result of transcription and translation of the genome; e.g., mRNA, cDNA, or a protein) thereof can be used. Particularly preferably, mRNA or cDNA is prepared for use.

In the method for detecting the polynucleotide of the present disclosure, the "step of detecting the presence of the polynucleotide" may be practiced by detecting the presence of the polynucleotide represented by SEQ ID NO: 4 (genomic sequence comprising the fusion point) or SEQ ID NO:5 (genomic sequence comprising the fusion point) in the genome of the sample obtained from a test subject or detecting the presence of mRNA or DNA corresponding to the polynucleotide type v1 of the present disclosure (preferably, the EML4-ALK fusion polynucleotide v1), the polynucleotide type v2 of the present disclosure (preferably, the EML4-ALK fusion polynucleotide v2) or the polynucleotide type v3 of the present disclosure (preferably, the EML4-ALK fusion polynucleotide v3) by preparing a transcription product (e.g., mRNA or cDNA) of genomic DNA extracted from the sample obtained from a test subject.

The genomic DNA extraction can be performed by a method known in the art and can be performed conveniently with a commercially available DNA extraction kit.

The detection step at the step (1) can be practiced according to a gene analysis method known in the art (e.g., PCR commonly used as a gene detection method, LCR (Ligase chain reaction), SDA (Strand displacement amplification), NASBA (Nucleic acid sequence-based amplification), ICAN (Isothermal and chimeric primer-initiated amplification of nucleic acids), LAMP (Loop-mediated isothermal amplification), TMA (Gen-Probe's TMA system), and well known methods such as a microarray). For example, a hybridization technique using, as a probe, a nucleic acid hybridizing to the polynucleotide of the present invention or a gene amplification technique using, as primers, DNAs hybridizing to the polynucleotide of the present invention is utilized. Specifically, a nucleic acid, for example, mRNA, derived from the sample obtained from a test subject is used for measurement. The mRNA level is measured by a gene amplification reaction method using primers designed to specifically amplify the polynucleotide sequence of the present disclosure. Primers used in the detection method of the present invention or primers contained in the kit for detection of the present invention are not particularly limited as long as they specifically amplify the polynucleotide sequence of the present invention. These primers are designed on the basis of the nucleotide sequence of the polynucleotide of the present invention. Primer design for a PCR amplification monitoring method can be achieved by utilizing primer design software Primer Express (PE Biosystems). PCR products with a large size reduce amplification efficiency. Therefore, it is appropriate that sense and antisense primers should be designed to give amplification products of I kb or less in size in the amplification of mRNA or cDNA. More specifically, a sense primer (5'-primer) and an antisense primer (3'-primer) are designed from an EML4-encoding portion (e.g., any portion within the EML4 gene region of the EML4-ALK fusion polynucleotide (particularly, cDNA)) and from an ALK-encoding portion (e.g., any portion within the ALK gene region of the EML4-ALK fusion polynucleotide (particularly, cDNA)), respectively. Preferably, primers contained in the kit for detection of the present invention, more preferably, most suitable primers contained in the kit for detection of the present invention, are used. Whether the gene of interest (the whole sequence or its specific portion) is amplified or not can be confirmed by a method suitable for each amplification technique. For example, for PCR, the PCR products can be subjected to analysis by agarose gel electrophoresis and ethidium bromide staining to thereby confirm whether an amplification fragment with the size of interest is obtained or not. If the amplification fragment with the size of interest is obtained, then the polynucleotide of the present invention is present in the sample obtained from a test subject. In this way, the presence of the polynucleotide of the present disclosure can be detected.

Detection using the hybridization technique is performed, for example, by a northern hybridization, dot-blot, or DNA microarray method. A probe comprising a nucleic acid molecule with at least 32 consecutive bases hybridizing under stringent conditions (preferably, more stringent conditions) to a polynucleotide of the present disclosure; or complementary strands thereof, and comprising positions 1744 to 1775 of the nucleotide sequence represented by SEQ ID NO: 1, positions 2227 to 2258 of the nucleotide sequence represented by SEQ ID NO: 6, positions 685 to 716 of the nucleotide sequence represented by SEQ ID NO: 129, positions 3614 to 3645 of the nucleotide sequence represented by SEQ ID NO: 4, positions 564 to 595 of the nucleotide sequence represented by SEQ ID NO: 5; or complementary strands thereof can be used for detecting the polynucleotide in the hybridization. Furthermore, a gene amplification technique such as RT-PCR can be utilized. In the RT-PCR method, the presence of the polynucleotide of the present disclosure can be analyzed more quantitatively by using a PCR amplification monitoring (real-time PCR) method (Genome Res., 6 (10), 986, 1996) in the gene amplification process. For example, ABI PRISM 7900 (PE Biosystems) can be used as the PCR amplification monitoring method. The real-time

PCR is a method known in the art and can be performed conveniently by utilizing a commercially available apparatus and a kit for real-time PCR.

A method for detecting a fusion protein encoded by the polynucleotide of the present disclosure comprises the step of

(2) detecting the presence of the polypeptide of the present disclosure in a sample obtained from a test subject.

Such a detection step can be practiced by preparing a solubilized solution derived from a sample obtained from a test subject (e.g., a cancer tissue or cell obtained from the test subject) and detecting the polypeptide of the present disclosure (particularly, the EML4-ALK fusion polypeptide v1) contained therein by an immunological measurement or enzyme activity measurement method using anti-EML4 and anti-ALK antibodies in combination. Preferably, an approach using a monoclonal or polyclonal antibody specific to the polypeptide of the present disclosure (particularly, the EML4-ALK fusion polypeptide v1) can be used, such as enzyme immunoassay, two-antibody sandwich ELISA, fluoroimmunoassay, radioimmunoassay, or western blotting.

More preferably, the presence of the polypeptide of the present disclosure can be detected, as shown in Example 9, by subjecting cell extracts from a cell likely to have the polypeptide of the present disclosure to immunoprecipitation with an anti-EML4 antibody and performing detection using an anti-ALK antibody for the precipitates. In the method of Example 9, immunoprecipitation with the anti-ALK antibody and detection with the anti-EML4 antibody may also be used. After the immunoprecipitation and detection thus performed, it is preferred to further confirm that the protein detected by the detection antibody has the size of the polypeptide of the present disclosure of interest. The antibodies used in this detection may be any antibody that can specifically bind to a polypeptide sequence from exon 1 to exon 20 (preferably, from exon 1 to exon 13; more preferably, from exon 1 to exon 6) of EML4 or a polypeptide sequence from exon 21 to exon 30 of ALK, and may be monoclonal or polyclonal antibodies.

If the polynucleotide of the present disclosure or the polypeptide of the present disclosure is detected from the sample obtained from a test subject, the test subject is a target (patient) having cancer that is shown to be positive for the polynucleotide of the present disclosure and serves as a target to which the pharmaceutical composition of the present disclosure is applicable.

The kit for detection of the present invention comprises at least sense and antisense primers designed to specifically amplify the polynucleotide of the present disclosure. The set of sense and antisense primers are a set of polynucleotides that function as primers for amplification of the polynucleotide of the present disclosure. Examples thereof include the primer sets (1) to (7) described in the paragraph <Primer set of the present invention>. Preferable primer sets are the primer sets (2) to (7). More preferable primer sets are the primer sets (7).

Examples of other reagents that can be contained in the kit for detection of the present invention can include reagents (e.g., Taq polymerase, nucleotide substrates, and buffer solutions) necessary for PCR.

<Screening method of the present disclosure>

The screening method of the present disclosure encompasses [1] a method for screening a substance inhibiting the polypeptide of the present disclosure and [2] a method for screening a therapeutic agent for cancer (preferably, lung cancer) that is shown to be positive for the polynucleotide of the present disclosure.

[1] Method for screening a substance inhibiting the polypeptide of the present disclosure (inhibiting the activity and/or expression of the polypeptide of the present disclosure).

The method for screening a substance inhibiting the polypeptide of the present disclosure is not particularly limited as long as it comprises the following steps (i) to (iii):

(i) bringing test substances into contact with the polypeptide of the present disclosure or a cell expressing the polypeptide of the present disclosure;
(ii) analyzing whether the polypeptide is inhibited or not, and
(iii) selecting a substance inhibiting the polypeptide.

Preferably, the substance inhibiting the polypeptide of the present disclosure can be screened by methods described in Examples 7(3), 7(4), 8(3), 8(5) - 8(9), 10(3), 11(4) - (6), 11(8), and 11(9).

The screening method [1] of the present disclosure encompasses the following methods (a), (b), or (c) :

(a) *In vitro* screening method;
a method for screening a substance inhibiting the activity of the polypeptide of the present disclosure, comprising the steps of (1) bringing test substances into contact with the polypeptide of the present disclosure, (2) analyzing whether the activity of the polypeptide is inhibited or not, and (3) selecting a substance inhibiting the activity of the polypeptide;
(b) Cell-based screening method;
a method for screening a substance inhibiting the activity of the polypeptide of the present disclosure, comprising the steps of (1) bringing test substances into contact with a cell expressing the polypeptide of the present disclosure, (2) analyzing whether the activity of the polypeptide is inhibited or not, and (3) selecting a substance inhibiting the activity of the polypeptide; and
(c) Expression inhibition-based screening method;
a method for screening a substance inhibiting the expression of the polypeptide of the present disclosure, comprising the steps of (1) bringing test substances into contact with a cell expressing the polypeptide of the present disclosure, (2) analyzing whether the expression of the polypeptide is inhibited or not, and (3) selecting a substance inhibiting the expression of the polypeptide.

Each screening method (a)-(c) will be described below. The cell expressing the polypeptide of the present disclosure, comprises a cell naturally expressing the polypeptide of the present disclosure, (e.g., NCI-H2228) and a cell caused to express the polypeptide of the present disclosure, by its transformation with a vector comprising the polynucleotide of the present disclosure. Preferable cell is the cell caused to express the polypeptide of the present disclosure by its transformation with a vector comprising the polynucleotide of the present disclosure.

(a) *In vitro* screening method

The *in vitro* screening method comprises: bringing test substances into contact with the purified polypeptide of the present invention by addition (contact step); analyzing whether the activity of the polypeptide of the present disclosure is inhibited or not by the test substance(s), by comparison with the activity of the polypeptide of the present disclosure not brought into contact with the test substances (analysis step); and selecting a substance inhibiting the activity of the polypeptide of the present disclosure (i.e., a therapeutic agent for cancer, particularly, a therapeutic agent for lung cancer).

In the screening method of the present disclosure, each step can specifically be practiced, for example, as described below. Test substances are brought into contact with the purified polypeptide of the present disclosure, by addition. After the addition of ATP, the activity of the polypeptide is measured. Solvents (e.g., DMSO) for the test substances are brought as a control into contact with the purified polypeptide by mixing. After the addition of ATP, the activity of the polypeptide is measured. A condition without the addition of ATP can be set as a background control. Whether the activity of the polypeptide of the present disclosure is inhibited or not by the test substance(s) is analyzed. Whether the activity (i.e., phosphorylating activity) of the polypeptide of the present disclosure is inhibited or not by the test substance(s) can be determined by analyzing a test substance-induced change in the tyrosine phosphorylation level of the polypeptide of the present disclosure. Specifically, when the addition (i.e., contact) of a test substance inhibits the activity (i.e., phosphorylating activity) of the polypeptide of the present disclosure as compared with the addition (i.e., contact) of the solvent control, this test substance is selected as a substance inhibiting the activity of the polypeptide of the present disclosure, (i.e., a therapeutic agent for cancer, particularly, a therapeutic agent for lung cancer). *In vitro* screening method of the present disclosure comprises a screening method carried our in a manner similar as above except in the above steps, the peptide substrate is added and mixed before the addition of ATP, and activity of the polypeptide of present disclosure is determined by analyzing phosphorylation activity on the peptide substrate by the polypeptide of the present disclosure (i.e., analyzing a change in the phosphorylation level on the peptide substrate by the polypeptide of the present disclosure in order to determine whether a test substance inhibits the activity of the polypeptide of the present disclosure). Of the screening methods of the present disclosure, preferably, the *in vitro* screening method is practiced under the conditions described in Example 7(3) or 11(4). A substance that can inhibit 50% or more activity by this method at a concentration of 10 μM or lower, preferably 1 μM or lower, more preferably 0.1 μM or lower is selected as a substance inhibiting the activity of the polypeptide of the present disclosure.

(b) Cell-based screening method

The cell-based screening method comprises: bringing test substances into contact with a cell expressing the polypeptide of the present disclosure by mixing (i.e., addition) (contact step); analyzing whether the activity of the polypeptide of the present disclosure is inhibited or not by the test substance(s), by comparison with the activity of the polypeptide of the present disclosure not brought into contact with the test substances (analysis step); and selecting a substance inhibiting the activity of the polypeptide of the present disclosure (i.e., a therapeutic agent for cancer, particularly, a therapeutic agent for lung cancer). This screening method can specifically be practiced, for example, as described below.

First, test substances or solvent controls (e.g., DMSO) are brought into contact with a cell expressing the polypeptide of the present disclosure. The cells are cultured for a given time. The activity (i.e., autophosphorylating activity) of the polypeptide of the present disclosure is measured using cell lysates prepared from the cultured cells by dissolution, by SDS electrophoresis known in the art and immunoblotting using an anti-phosphorylated ALK antibody (e.g., Cell Signaling Technology) to thereby analyze whether the activity of the polypeptide of the present disclosure is inhibited or not by the test substance(s). Whether the activity of the polypeptide of the present disclosure is inhibited or not by the test substance(s) can be determined by analyzing a test substance-induced change in the tyrosine phosphorylation (i.e., autophosphorylation) level of the polypeptide of the present disclosure Specifically, when the addition (i.e., contact) of a test substance inhibits the activity of the polypeptide of the present disclosure as compared with the addition (i.e., contact) of the solvent control, this test substance is selected as a substance inhibiting the activity of the polypeptide of the present disclosure (i.e., a therapeutic agent for cancer, particularly, a therapeutic agent for lung cancer). Of the screening methods of the present disclosure, preferably, the cell-based screening method is practiced under the conditions described in Example 7(4), 10(3), 11(5) or 11(8). A substance that can inhibit 50% or more activity by this method at a concentration of 10 μM or lower, preferably 1 μM or lower, more preferably 0.1 μM or lower is selected.

(c) Expression inhibition-based screening method

The expression inhibition-based screening method comprises: bringing test substances into contact with a cell expressing the polypeptide of the present disclosure by mixing (i.e., addition) (contact step); analyzing whether the expression of the polypeptide of the present disclosure is inhibited or not by the test substance(s), by comparison with the expression of the polypeptide of the present disclosure not brought into contact with the test substances (analysis step); and selecting a substance inhibiting the expression of the polypeptide of the present disclosure (i.e., a therapeutic agent for cancer, particularly, a therapeutic agent for lung cancer, that is shown to be positive for the polynucleotide of the present disclosure). This screening method can specifically be practiced, for example, as described below.

Test substances or solvent controls (e.g., DMSO) are brought into contact with any cell expressing the polypeptide of the present disclosure. After culture, extracts are prepared from the cells and subsequently used to analyze whether the expression of the polypeptide of the present disclosure is inhibited or not by the test substance(s). Whether the expression of the polypeptide of the present disclosure is inhibited or not can be analyzed by analyzing whether the mRNA or protein expression of the polypeptide of the present disclosure is inhibited or not. More specifically, the mRNA or protein level of the polypeptide of the present disclosure present in the cell extracts is identified by an expression level analysis method known in the art, for example, northern blotting, quantitative PCR, immunoblotting, or ELISA. More specifically, the inhibition of the mRNA or protein expression of the polypeptide of the present disclosure can be analyzed by a method described in Example 8(5) or 8(6). Whether the expression of the polypeptide of the present disclosure is inhibited or not by the test substance(s) can be determined by analyzing a test substance-induced change in the expression level of the polypeptide of the present disclosure. Specifically, when the contact of a test substance inhibits the expression level (i.e., mRNA or protein level) of the polypeptide of the present disclosure as compared with the contact of the solvent control, this test substance is selected as a substance inhibiting the expression of the polypeptide of the present disclosure (i.e., a therapeutic agent for cancer, particularly, a therapeutic agent for lung cancer). Of the screening methods of the present disclosure, preferably, the expression inhibition-based screening method is practiced under the conditions described in Example 8(5) or 8(6). A substance that can inhibit 50% or more activity by this method at a concentration of 10 μM or lower, preferably 1 μM or lower, more preferably 0.1 μM or lower is selected. Preferably, the selected test substance has an inhibitory activity on all cells used. However, a test substance having an inhibitory activity on one cell can also be selected.

[2] Method for screening a therapeutic agent for cancer that is shown to be positive for the polynucleotide of the present disclosure

It was revealed that the EML4-ALK fusion polynucleotide is an oncogene (Example 6 and 11 (2)) and the presence of the EML4-ALK fusion polynucleotide was detected in some lung cancer patients. Further it was revealed that the anchorage-independent cell growth was inhibited (i.e., an anti-cancer effect is exhibited) by inhibiting the activity and/or expression of the polypeptide of the present disclosure (Example 8, 11). It was shown that the substance inhibiting the polypeptide of the present disclosure (inhibiting the activity and/or expression of the polypeptide of the present disclosure) has a therapeutic effect on cancer. Specifically, the method for screening a substance inhibiting the polypeptide of the present disclosure (inhibiting the activity and/or expression of the polypeptide of the present disclosure), aforementioned above [1], can be utilized as a method for screening a therapeutic agent for cancer (preferably, lung cancer) that is shown to be positive for the polynucleotide of the present disclosure.

The screening method [2] of the present invention further comprises, in addition to analyzing whether the polypeptide of the present disclosure is inhibited or not and selecting a substance inhibiting the polypeptide of the present disclosure the step of confirming that the selected test substance has a therapeutic activity against cancer (particularly, lung cancer) that is shown to be positive for the polynucleotide of the present disclosure.

<u>The step of confirming that the selected test substance has a therapeutic activity against cancer</u>

Examples of the step of confirming that the selected substance has a therapeutic activity against cancer (particularly, lung cancer) that is shown to be positive for the polynucleotide of the present disclosure include a step of practicing an evaluation method known in the art or a modified method thereof, for example, a method comprising analyzing the therapeutic activity of the selected substance against cancer (particularly, lung cancer) by treating, with the substance, cultured cells or tumor model animals expressing the polypeptide of the present disclosure (Clinical Oncology, 2nd ed., Cancer and Chemotherapy Publishers, Inc.).

The step of confirming that the selected test substance has a therapeutic activity against cancer (particularly, lung cancer) that is shown to be positive for the polynucleotide of the present disclosure is, preferably, the step of confirming that the selected test substance exhibits the growth-inhibiting effect and/or cell death-inducing effect on the cells using human cancer-derived (particularly, lung cancer-derived) cancer cells endogenously expressing the polypeptide of the present disclosure (e.g., NCI-H2228), the step of confirming that the selected test substance has the inhibitory effect on the anchorage-independent growth of cells transformed by the expression of the polypeptide of the present disclosure, and/or the step of confirming that the selected test substance has the inhibitory effect on the growth of tumor formed in nude mouse inoculated with a cell expressing the polynucleotide of the present disclosure.

A cancer-bearing model animal serving as a tumor model animal can be used in which the cancer cells endogenously expressing the polypeptide of the present disclosure (e.g., NCI-H2228) or the cells transformed by the expression of the polypeptide of the present disclosure are transplanted subcutaneously, intradermally, or intraperitoneally or into each organ (e.g., a nude mouse in which NIH3T3 cells caused to express the polypeptide of the present invention are transplanted). Furthermore, an animal caused to overexpress the EML4-ALK fusion polynucleotide can also be used.

Examples of the test substances used in the screening method of the present disclosure can include, but not particularly limited to, commercially available compounds (including peptides), a variety of compounds (including peptides) known in the art and registered in chemical files, compound groups obtained by a combinatorial chemistry technique (N. Terrett et al., Drug Discov. Today, 4 (1): 41, 1999), microorganism culture supernatants, plant- or marine organism-derived natural components, animal tissue extracts, double-stranded nucleic acids, antibodies or antibody fragments, and compounds (including peptides) chemically or biologically modified from compounds (including peptides) selected by the screening method of the present disclosure.

< Use for the manufacture of pharmaceutical composition for treatment of cancer that is shown to be positive for the polynucleotide of the present disclosure, and double-stranded nucleic acid>

The present disclosure encompasses a pharmaceutical composition for treatment of cancer that is shown to be positive for the polynucleotide of the present disclosure, comprising, as an active ingredient, a substance obtained by the screening method of the present disclosure, The present disclosure encompasses use of a substance inhibiting the polypeptide of the present disclosure (e.g., a substance obtained by the screening method of the present disclosure [e.g., a double-stranded nucleic acid (including siRNA), protein (including an antibody or antibody fragment), peptide, or other compounds]) for the manufacture of a pharmaceutical composition for treatment of cancer that is shown to be positive for the polynucleotide of the present disclosure.

The active ingredient in the pharmaceutical composition can be selected by the screening method of the present disclosure. Examples of the substance selected by the screening method of the present disclosure can include compounds A to D and a double-stranded nucleic acid described in Examples 7 and 8 below respectively. Alternatively, a compound selected by the screening method of the present disclosure from a low-molecular-weight compound with an inhibitory activity against ALK (ALK inhibitor) known in the art can be used as an active ingredient in the pharmaceutical composition. The ALK inhibitor can be exemplified by ALK inhibitors described in WO 2005/097765 and WO 2005/016894 (particularly, 2,4-pyrimidinediamine derivatives). Particularly, a compound described in Wan W et al., Blood 107: 1617-1623, 2006 as well as WHI-P131 (4-(4'-Hydroxyphenyl)amino-6,7-dimethoxyquinazoline) and WHI-P154 (4-[(3'-Bromo-4'-hydroxyphenyl)amino]-6,7-dimethoxyquinazoline); referred to as a compound A; Marzec M et al., Lab Invest 85: 1544-1554, 2005) (both, EMD Biosciences ) can be used. Alternatively, *N*-[2-(3-chlorophenyl)ethyl]-2-[({[4-(trifluoromethoxy)phenoxy]acetyl}amino)methyl]-1,3-thiazole-4-carboxamide (WO 2005/097765; hereinafter, referred to as a compound B), 5-chloro-$N^4$-[2-(isopropylsulfonyl)phenyl]-$N^2$-{2-methoxy-4-[4-(4-methylpiperazin-1-yl)piperidin-1-yl]phenyl}pyrimidine-2,4-diamine (WO 2005/016894; hereinafter, referred to as a compound C), or 2-[(5-bromo-2-{[2-methoxy-4-(4-methylpiperazin-1-yl)phenyl]amino}pyrimidin-4-yl)amino]-*N*-methylbenzenesulfonamide (WO 2005/016894; hereinafter, referred to as a compound D) can be used as an ALK inhibitor.

The double-stranded nucleic acid exemplified as an active ingredient in the pharmaceutical composition comprises a double-stranded nucleic acid (RNA or DNA) portion and, preferably, 3'-terminal overhangs of the sense and antisense strands and induces RNAi. The RNAi is an evolutionarily conserved phenomenon, which occurs via a double-stranded nucleic acid with 21 to 23 bases produced by RNase III endonuclease (Genes Dev. 15, 485-490, 2001). The 3'-terminal overhangs are respectively any nucleic acid with 1 or 2 bases, preferably 2 bases. The number of bases (21 to 23 bases) described above is the number of bases of the sense or antisense strand including its overhang. The sense and antisense strands can have the same number of bases or a different number of bases and, preferably, have the same number of bases.

For example, U (uridine), A (adenosine), G (guanosine), or C (cytidine) can be used as ribonucleic acids constituting the 3'-terminal overhangs of the double-stranded nucleic acid. For example, dT (deoxythymidine), dA (deoxyadenosine), dG (deoxyguanosine), or dC (deoxycytidine) can be used as deoxyribonucleic acids constituting the 3'-terminal overhangs thereof.

The double-stranded nucleic acid that can be used as an active ingredient in the pharmaceutical composition comprises a double-stranded portion designed on the basis of bases at positions 1743 to 1761, 1744 to 1762, 1750 to 1768, 1753 to 1771, 1756 to 1774, or 1757 to 1775 in SEQ ID NO: 1 and has an inhibitory activity on the expression of the polypeptide of the present disclosure (hereinafter, referred to as a double-stranded nucleic acid of the present disclosure). Examples of preferable aspects of such a double-stranded nucleic acid include siRNA-1 to siRNA-6 described in Example 8 (i.e., a double-stranded nucleic acid, one strand of which consists of the nucleotide sequence represented by SEQ ID NO: 111 and the other strand of which consists of the nucleotide sequence represented by SEQ ID NO: 112; a double-stranded nucleic acid, one strand of which consists of the nucleotide sequence represented by SEQ ID NO:

113 and the other strand of which consists of the nucleotide sequence represented by SEQ ID NO: 114; a double-stranded nucleic acid, one strand of which consists of the nucleotide sequence represented by SEQ ID NO: 115 and the other strand of which consists of the nucleotide sequence represented by SEQ ID NO: 116; a double-stranded nucleic acid, one strand of which consists of the nucleotide sequence represented by SEQ ID NO: 117 and the other strand of which consists of the nucleotide sequence represented by SEQ ID NO: 118; a double-stranded nucleic acid, one strand of which consists of the nucleotide sequence represented by SEQ ID NO: 119 and the other strand of which consists of the nucleotide sequence represented by SEQ ID NO: 120; and a double-stranded nucleic acid, one strand of which consists of the nucleotide sequence represented by SEQ ID NO: 121 and the other strand of which consists of the nucleotide sequence represented by SEQ ID NO: 122). The double-stranded nucleic acid of the present invention can be produced by standard methods (e.g., J. Am. Chem. Soc., 120, 11820-11821, 1998; and Methods, 23, 206-217, 2001). Alternatively, a contract manufacturer for double-stranded nucleic acids (e.g., RNAi Co., Ltd.) is well known by those skilled in the art and can be utilized in the production of the double-stranded nucleic acid. The target sequences of the siRNA-1 to siRNA-6 were confirmed by an siRNA sequence design system (commercial version siDirect (registered trademark), RNAi Co., Ltd.) to be specific to the polypeptide of the present disclosure.

The double-stranded nucleic acid of the present invention can be designed on the basis of DNA nucleotide sequences (bases at positions 1743 to 1761, 1744 to 1762, 1750 to 1768, 1753 to 1771, 1756 to 1774, or 1757 to 1775 in SEQ ID NO: 1) targeted by the siRNA-1 to siRNA-6. Such a double-stranded nucleic acid inhibits the polypeptide of the present disclosure, as with the siRNA-1 to siRNA-6. For example, siRNA can be designed which has a double-stranded portion consisting of an RNA nucleotide sequence directly converted from the whole target DNA nucleotide sequence. Alternatively, chimeric DNA-RNA double-stranded nucleic acid (which comprises both RNA and DNA in the identical strand) can be designed which has an RNA sequence converted from any portion of the target DNA nucleotide sequence. Furthermore a hybrid double-stranded nucleic acid, one strand of which is DNA and the other strand of which is RNA can be designed and produced for use. The conversion of the RNA nucleotide sequence from the DNA nucleotide sequence described herein means that dT in the DNA nucleotide sequence is converted to U and other bases, that is, dA, dG, and dC are converted to A, G, and C, respectively.

The double-stranded nucleic acid of the present disclosure exhibited an inhibitory effect on the anchorage-independent growth of a cell expressing the polypeptide of the present disclosure (Example 8(7)). Therefore, the double-stranded nucleic acid of the present disclosure can be utilized for manufacture of the pharmaceutical composition for treatment of cancer that is shown to be positive for the polynucleotide of the present disclosure.

A therapeutic effect on cancer that is shown to be positive for the polynucleotide of the present disclosure can be confirmed by use of a method generally known by those skilled in the art or a modified method thereof (see "The step of confirming that the selected test substance has a therapeutic activity against cancer").

A preparation comprising, as an active ingredient, a substance inhibiting the polypeptide of the present disclosure (e.g., a substance [e.g., a double-stranded nucleic acid, protein (including an antibody or antibody fragment), peptide, or other compounds] obtained by the screening method of the present disclosure) can be prepared as a pharmaceutical composition using pharmacologically acceptable carriers, excipients, and/or other additives usually used in the preparation production according to the type of the active ingredient.

Examples of administration can include: oral administration using tablets, pills, capsules, granules, subtle granules, powders, or oral liquid agents; and parenteral administration using injections for intravenous injection (including intravenous drip), intramuscular injections, or subcutaneous injection, suppositories, percutaneous administration agents, or transmucosal administration agent. Particularly, parenteral administration such as intravenous injection is preferable for peptides that are digested in the stomach.

To prepare a solid composition for oral administration, 1 or more active substances can be mixed with at least one inactive diluent, for example, lactose, mannitol, glucose, microcrystalline cellulose, hydroxypropylcellulose, starch, polyvinyl pyrrolidone, or magnesium aluminometasilicate. The composition can contain additives other than the inactive diluent, for example, lubricants, disintegrants, stabilizers, or solvents or solubilizers according to a standard method. Tablets or pills can be coated, if necessary, with a sugar coating or with a film such as a gastrosoluble or enteric substance.

A liquid composition for oral administration can comprise, for example, an emulsion, solution, suspension, syrup, or elixir and can contain an inactive diluent generally used, for example, purified water or ethanol. The composition can contain additives other than the inactive diluent, for example, moisturizers, suspensions, sweeteners, flavors, or antiseptics.

A parenteral injection can comprise an aseptic aqueous or non-aqueous solution, suspension, or emulsion. A water-soluble solution or suspension can contain, for example, distilled water or saline for injection, as a diluent. A water-insoluble solution or suspension can contain, for example, propylene glycol, polyethylene glycol, plant oil (e.g., olive oil), alcohols (e.g., ethanol), or polysorbate 80 as a diluent. The composition can further contain moisturizers, emulsifiers, dispersants, stabilizers, solvents or solubilizers, or antiseptics. The composition can be sterilized, for example, by filtration using a bacterium-impermeable filter, formulation of germicides thereinto, or irradiation. Alternatively, an aseptic solid composition can be produced and dissolved in aseptic water or other aseptic media for injection for use.

A dose can be determined appropriately in consideration of the activity intensity of the active ingredient, that is, the substance obtained by the screening method of the present disclosure, conditions, the age or gender of a subject receiving administration, and so on. Preferably, the dose can be calculated according to a route as an amount that gives a serum concentration around tumor or intratumoral concentration 3 to 30 timers, for example, 10 times, higher than a drug concentration inhibiting 50% of the activity or expression of the polypeptide of the present invention. For example, the dose in oral administration in adult (60 kg in body weight) is usually approximately 0.1 to 100 mg/day, preferably, 0.1 to 50 mg/day. The dose in parenteral administration is 0.01 to 50 mg/day, preferably, 0.01 to 10 mg/day, in terms of an injection.

A therapeutic target by the pharmaceutical composition is a test subject from which the presence of the polynucleotide of the present disclosure and/or the polypeptide of the present disclosure has been detected. The substance inhibiting the polypeptide of the present disclosure kills cells that have transformed due to the EML4-ALK fusion polynucleotide. Therefore, the substance inhibiting the polypeptide of the present disclosure serves as an effective therapeutic agent for cancer (particularly, lung cancer) that is shown to be positive for the polynucleotide of the present invention.

[Examples]

The present invention will be described in detail below by Examples, but the present inventions are not limited by these Examples. Further, unless otherwise stated the process of the present invention can be carried out according to publicly known methods. Also, commercially available reagents and kits can be used in accordance with the instructions of the commercial products.

A full length ALK cDNA was kindly supplied by Dr. Steve Morris of St. Jude Children's Research Hospital. Further, this research project was approved by the ethics committee for gene analysis study at Jichi Medical University.

Anti-phosphorylated ALK antibody and anti-ALK antibody used were produced by Cell Signaling Technology Inc. and NEOMARKERS Inc., respectively.

[Example 1]

Isolation of EML4-ALK fusion polynucleotide v1

(1) Construction of cDNA library

Using a RNA purification kit (RNeasy Mini Column; Qiagen Inc.), RNA was extracted from a resected specimen of lung adenocarcinoma of a 62 year old male who gave informed consent and cDNA was synthesized using reverse transcriptase (Power Script Reverse Transcriptase) and primers (an oligonucleotide of SEQ ID NO: 42 and CDS primer IIA) (all from Clontech Inc.). After selectively amplifying the full length cDNA by polymerase chain reaction (PCR) (17 cycles of 98°C for 10 seconds and 68°C for 6 minutes) using a primer (5'-PCR primer IIA; Clontech Inc.) and a polymerase (primeSTAR HSDNA polymerase, Takara Bio Inc.), a BstX1 adapter (Invitrogen Co.) was attached to the both ends of cDNA. The cDNA thus obtained was ligated to a retrovirus plasmid, and a retrovirus plasmid library was constructed by introducing this plasmid to E. coli DH10B (Invitrogen Inc.). As the result, the plasmid library containing clones more than 1,500,000 colony forming units in total has been successfully constructed.

(2) Focus formation assay

2 μg of the plasmid of the library described above and 0.5 μg of a plasmid for packaging (pGP, and pE-eco, both of which were obtained from Takara Bio Inc.) were transfected to BOSC23 packaging cells using a transfection reagent. Two days after the transfection, the culture supernatant was recovered as a solution of recombinant retrovirus library, mixed with polybrene (Sigama Inc.) at a concentration of 4 μg/ml, and the mixture was added to mouse 3T3 cells at MOI (multiplicity of infection) of 0.1 concentration. Two days later, the culture supernatant of 3T3 cells was changed to DMEM-F12 medium (Invitrogen Inc.) supplemented with 5% bovine serum (Invitrogen Inc.) and 2 mM L-glutamine, and cells were cultured 2 more weeks to obtain 10 or more kinds of transformed foci. After isolating each 3T3 cell clone, the culturing of the clones was continued separately, and the genomic DNA of each clone was extracted. The viral cDNA integrated in each 3T3 clone was amplified and recovered by carrying out PCR (30 cycles of 98°C for 10 seconds and 68°C for 6 minutes) using 10 ng of the genomic DNA as a template, 5'-PCR primer IIA primer and DNA polymerase (PrimeStar HS DNA polynerase; Takara Bio Inc.), and cloned in pT7Blue-2 vector.

One of the cDNA thus obtained was 3926 base pair long (SEQ ID NO: 1) and had a single open reading frame (from the 271st to 3447th base of SEQ ID NO: 1) coding for a protein having 1059 amino acid residues (SEQ ID NO: 2 ; a novel full-length sequence). About half of the amino-terminus of SEQ ID NO: 2 (1-496 amino acid residues of SEQ ID NO: 2) was perfectly matched to 1-496 amino acid residues of echinoderm microtubule associated protein like-4;

EML4 (GenBank accession No. NM_019063), and on the other hand, about half of the carboxyl terminus (497-1059 amino acid residues of SEQ ID NO: 2) was perfectly matched to the amino acid sequence of anaplastic lymphoma kinase; ALK (GenBank accession No. AB209477). Also, in the nucleotide sequence of SEQ ID NO: 1, 99.9% of the 35-1759 base matched the 1-1725 base of the reported human EML4 cDNA, and the 1760-3677 base of SEQ ID NO: 1 matched the 3613-5530 base of human ALK cDNA by 99.9%. Although respective sequences are a little different from the reported base sequences, any of these differences do not lead to amino acid replacement, and therefore it may be possible that they are gene sequence polymorphism. From the above results, the present cDNA was believed to be a fused cDNA between a part of EML4 cDNA and a part of ALK cDNA. Further, the obtained cDNA (SEQ ID NO: 1) contained a domain of ALK tyrosine kinase.

[Example 2]

Confirmation of EML4-ALK fusion polynucleotide v1

The EML4 gene and ALK gene in human are both mapped in the short arm of the second chromosome in opposite directions (head to head direction). For the EML4-ALK fusion polynucleotide found in Example 1 to be produced, respective genomes are needed to be cut in the intron downstream from the exon 13 of the EML4 gene and the intron upstream from the exon 21 of the Alk gene, and re-ligated with the one gene in the reverse direction. To prove this directly, PCR (after 94°C for 1 minute, 40 cycles of 98°C for 20 seconds and 68°C for 9 minutes) was carried out using primers having the base sequences of SEQ ID NO: 40 (a sequence designed in the sense strand of the 3' terminus of the exon 13 of the EML4 gene) and SEQ ID NO: 36 (a sequence designed in the anti-sense strand of the exon 21 of the ALK gene), templates of the genomic DNA of a patient (ID 33) and the control genomic DNA (the genomic DNA of peripheral monocytes of normal healthy female (46, XX)) and DNA polymerase (LA Taq polymerase; Takara Bio Inc.).

As the result, as shown in FIG. 1, a PCR product having about 4 kbp length was obtained only by the genomic DNA of the present patient. That is, it was confirmed as expected that in the genomic level the EML4 gene and the ALK gene were cut at the introns and re-ligated in the reverse direction. Further, this PCR product of about 4 kbp was cloned into a pT7Blue-2 vector (Novagen Inc.) and the total base sequence was determined. It was made clear that the cuts were located approximately 3.6 kbp downstream from the exon 13 of the EML4 gene and 297 bp upstream from the exon 21 of the ALK gene. The base sequence is shown in SEQ ID NO: 4. SEQ ID NO: 4 is a genomic sequence including the fusion point of the EML4 gene and ALK gene.

[Example 3]

Screening for EML4-ALK fusion polynucleotide in clinical specimens

(1) Detection of EML4-ALK fusion polynucleotide using cDNA

cDNAs were synthesized from 33 cases of clinical specimens (resected specimens of non-small cell lung cancer) including the case (ID 33) used in the present Example 1 and 2 and from peripheral monocytes of one case of a normal healthy subject (46, XX).

To detect the cDNA of EML4-ALK fusion polynucleotide v1, PCR (50 cycles of 94°C for 15 seconds, 60°C for 30 seconds and 72°C for 1 minute) was carried out using a quantitative PCR kit (QuantiTect SYBR Green; Qiagen Inc.), the cDNAs as substrates prepared from the clinical specimens and the normal healthy subject described above and oligonucleotides of SEQ ID NO: 8 and 9 as primers. Using the same specimens, PCR amplifications of the glyceraldehyde-3-phosphate dehydrogenase (herein after GAPDH) cDNA was tried as a control. To detect the GAPDH cDNA, oligonucleotides consisting of the nucleotide sequences represented by SEQ ID NO: 10 and 11 were used as primers. Amplified respective samples were electrophoresed with a size marker DNA (Marker: 50 bp ladder, Invitrogen Inc.). As the result, as shown in the upper part of Fig 2, in the total 3 cases including ID 33, the cDNA of the EML4-ALK fusion polynucleotide v1 was detected. Further, in all the cases analyzed, an amplification of the GAPDH cDNA was confirmed clearly (Lower part of FIG. 2). In addition, the base sequence of the PCR products identified in the cases of ID 20 and ID 39 were analyzed and the result confirmed that the sequence was identical to that of ID 33 (the 247 bp including the fusion point of the EML4 gene and the ALK gene. SEQ ID NO: 14). That is, the result of the analyses of the 33 cases of non-small cell lung cancer confirmed that the fusion of the EML4 gene and the ALK gene occurs in 9.1% of the cases (3/33 cases).

Mutation in the EGFR gene has been known to be one of the causes of lung cancer. In the 33 specimens of the cases analyzed as described above, the analysis of the presence of an abnormality in the base sequence of the EGFR gene according to the known method confirmed a partial deletion of the exon 19 in 6 cases. The cases having the EGFR gene mutation and the cases positive for the EML4-ALK fusion polynucleotide belonged to different subgroups. That is, the existing therapeutic agents for lung cancer, which are therapeutic agent for the lung cancer caused by the mutation in the EGFR gene, are expected to be not effective for the lung cancer patients who are positive for the EML4-ALK fusion polynucleotide.

Also, the 33 cases analyzed as described above were subjected to the investigation whether the full length ALK gene existed, and it was found that it existed in 8 cases. The specimens of the 7 cases among these 8 cases did not contain the EML4-ALK fusion polynucleotide. (That is, the full length ALK gene did not exist in the 2 cases among the 3 cases where the EML4-ALK fusion polynucleotide was positive).

Further, analyses of 42 cases of other non-small cell lung cancer cases by a similar method as described above gave about 1 kbp PCR products in 4.8% of the cases (2/42 cases), which are larger than those detected in the cases of ID 33, ID 20 and ID 39 cases. These were cloned in pT7Blue-2 vector and the base sequence was analyzed. The results indicated that these were not the exon 13 of the EML4 gene but a fusion product of the exon 20 of the EML4 gene and the exon 21 of the ALK gene (SEQ ID NO: 3). That is, in these products the fused ALK gene fragment was the same, but the point of cleavage in the EML4 was different. The cDNA sequence of the fusion gene of the exon 1-20 of the EML4 gene and the exon 21-30 of the ALK gene, which contains the fusion point of the EML gene and the ALK gene found in SEQ ID NO: 3, is shown in SEQ ID NO: 6, and the amino acid sequence of the polypeptide coded thereby is shown in SEQ ID NO: 7. In the present description, the polynucleotide that codes for the protein consisiting of the polypeptide represented by SEQ ID NO: 7 is called the EML4-ALK fusion polynucleotide v2. The plasmid produced here in which a partial fragment of the EML4-ALK fusion polynucleotide v2 is cloned is designated as EML4-ALKv2 partial/pT7Blue-2.

(2) Detection of the EML4-ALK fusion polynucleotide v1 using genomic DNA

It turned out that the presence of the EML4-ALK fusion polynucleotide can be detected in samples obtained from the test subjects by PCR using the genomic DNA samples extracted from the clinical specimens (especially the lung tissue) of the test subjects as Example 2. Thus, as shown below, detection of EML4-ALK fusion polynucleotide v1 was tried using various primers. Using 1 ng of pT7Blue-2 vector as a template, in which the PCR product of about 4 kbp produced as described above was cloned and using a primer set [SEQ ID NO: 15 and SEQ ID NO: 16, SEQ ID NO: 17 and SEQ ID NO: 18, SEQ ID NO: 19 and SEQ ID NO: 20, SEQ ID NO: 21 and SEQ ID NO: 22, SEQ ID NO: 23 and SEQ ID NO: 24, SEQ ID NO: 25 and SEQ ID NO: 26, SEQ ID NO: 27 and SEQ ID NO: 28, SEQ ID NO: 29 and SEQ ID NO: 30, SEQ ID NO: 31 and SEQ ID NO: 32, or SEQ ID NO: 33 and SEQ ID NO: 34], PCR (30 cycles of 94°C for 15 seconds, 55°C for 30 seconds and 72°C for 1 minute) was carried out by a DNA polymerase (rTaq DNA polymerase; Takara Bio Inc.). The results indicated that a single DNA fragment having an expected size (from about 270 to 380 bp) was amplified in each PCR. It became clear from the above results that the detection of the EML4-ALK fusion polynucleotide v1 is possible by carrying out PCR using the genomic DNA extracted from the clinical specimens as a template and various primer sets which are thought to specifically detect the presence of the EML4-ALK fusion polynucleotide v1.

(3) Detection of the EML4-ALK fusion polynucleotide v2 using genomic DNA

An oligonucleotide having the base sequence represented by SEQ ID NO: 35 in the EML4 exon 20 and an oligonucleotide having the base sequence represented by SEQ ID NO: 36 in the antisense side of the ALK exon 21 were designed as a sense primer and antisense primer, respectively. Using these and using the genomic DNA of a patient (ID#KL-3121) as a template in which the cDNA of the EML4-ALK fusion polynucleotide v2 was detected, PCR (after 94°C for 1 minute, 40 cycles of 98°C for 20 seconds and 68°C for 6 minutes) was carried out with a DNA polymerase (LA Taq polymerase; Takara Bio Inc.). As the result, a PCR product of about 850 bp was obtained. The PCR product was cloned in pT7Blue-2 vector, the base sequence was determined and an 853 bp sequence shown in SEQ ID NO: 5 was obtained. The analyses of the sequence revealed that the 35 bp located at the 3' terminus of the EML4 exon 20 and the 544 bp of the intron sequence downstream from the EML4 exon 20 were linked to the 233 bp of the intron sequence upstream from the ALK exone 21 and the 41 bp located at the 5' terminus of the ALK exon 21. That is, it has become clear that the cleavage of the genome occurred at a location 544 bp downstream from the EML4 exon 20 and at a location 233 bp upstream from the ALK exon 21. Using 1 ng of pT7Blue-2 vector as a template, in which the 853 bp PCR product prepared as described above was cloned, and using a primer set [SEQ ID NO: 37 and SEQ ID NO: 38, SEQ ID NO: 39 and SEQ ID NO: 18, SEQ ID NO: 41 and SEQ ID NO: 20, SEQ ID NO: 43 and SEQ ID NO: 22, SEQ ID NO: 45 and SEQ ID NO: 24, SEQ ID NO: 47 and SEQ ID NO: 26, SEQ ID NO: 49 and SEQ ID NO: 28, SEQ ID NO: 51 and SEQ ID NO: 52, SEQ ID NO: 53 and SEQ ID NO: 54, or SEQ ID NO: 55 and SEQ ID NO: 34], PCR reaction was carried out under the same conditions as those in Example 3(2). As the result, a single DNA fragment having the expected size (from about 270 to 380 bp) was amplified in each PCR. It turned out from the above results that the detection of the presence of the EML4-ALK fusion polynucleotide v2 is possible by carrying out PCR using the genomic DNA extracted from the clinical specimens as a template and primer sets which are thought to specifically detect the EML4-ALK fusion polynucleotide v2.

[Example 4]

Method for detecting mRNA of the EML4-ALK fusion polynucleotide

(1) Construction of EML4-ALK fusion polypeptide v1 expression vector and cloning of EML4-ALK fusion polynucleotide v2

From the clone in which the EML4-ALK fusion polynucleotide v1 was cloned in the positive direction (designated as EML4-ALKv1/pT7Blue-2) the insert was taken out by digesting with restriction enzymes EcoRI and SalI and subcloned at the EcoRI-SalI sites of pMXS (JBC 275, 24945-24952, 2000). This was designated as EML4-ALKv1/pMS. Also, the cDNA of the 277-3450th base of the SEQ ID NO: 1 having the sites at the both ends which are recognized by restriction enzyme EcoRI was amplified by carrying out PCR (25 cycles of 98°C for 10 seconds, 68°C for 5 minutes) using EML4-ALKv1/pT7Blue-2 plasmid as a template and oligonucleotides consisting of the base sequence represented by SEQ ID NO: 59 and SEQ ID NO: 60 as primers and a DNA polymerase (PrimeStar HS DNA polymerase). After digesting with EcoRI, this was inserted at the EcoRI site of an expression vector pcDNA3, which is modified so that the insert can be expressed with an FLAG tag added to the N-terminus, to produce an expression plasmid (FLAG-EML4-ALKv1/pcDNA3) for EML4-ALK fusion polypeptide v1 having the FLAG tag at the N-terminus (hereinafter, FLAG-EML4-ALKv1). Further, the cDNA of FLAG-EML4-ALKv1 was taken out from this vector by digesting with restriction enzymes HindIII and XbaI, and after converting the both ends to blunt, an EcoRI-NotI-BamHI adaptor (Takara Bio Inc.) was ligated to both ends. The product was inserted at the EcoRI site of an expression vector, by which the inserted cDNA and cell surface antigen CD8 can be expressed at the same time (bicistronic vector pMX-iresCD8; J. Biol. Chem. 2001, vol. 276, p39012-39020), to produce an expression vector which expresses both FLAG-EML4-ALKv1 and CD8. This was designated as FLAG-EML4-ALKv1/pMX-iresCD8.

EML4-ALK fusion polynucleotide v2 was cloned as follows.

Using a full length polynucleotide of EML4 cloned in pT7Blue-2 according to "Genomics", (US), 2000, Vol. 68, p. 348-350 as a template for obtaining a polynucleotide fragment coding for EML4 of the EML4-ALK fusion polynucleotide v2, and using an oligonucleotide represented by SEQ ID NO: 57, in which an EcoRI cleavage sequence is attached to the 5'-terminus of the start codon ATG of the EML4 gene exon 1 and an oligonucleotide represented by SEQ ID NO: 58, the sequence of which consists of 10 bases of the 5' terminus of the antisense sequence of the ALK gene exon 21 fused to the 5' terminus of the antisense sequence to the 20 bases of 3' terminus of the EML4 gene exon 20, respectively as a sense and an antisense primers, and using a DNA polymerase (*Pyrobest* DNA polymerase; Takara Bio Inc.), PCR (25 cycles of 94°C for 20 seconds, 60°C for 30 seconds, 72°C for 1 minute) was carried out to obtain a PCR product of about 2260 bp. This product was designated as PCR product A.

While, using EML-ALKv1/pTBlue-2 produced in the present Example 4(1) as a template to obtain the ALK polynucleotide fragment of the EML4-ALK fusion polynucleotide v2, and using an oligonucleotide represented by SEQ ID NO: 101, the sequence of which consists of the 10 bases of the sense sequence at the 3' terminus of the EML4 gene exon 20 fused to the 5' terminus of the 20 bases of the sense sequence at the 5' terminus of the ALK gene exon 21 and an oligonucleotide represented by SEQ ID NO: 102, in which the Xba I cleavage sequence is attached to the 5' terminus of the antisense sequence area containing the stop codon present in the ALK gene exon 30 as a sense and an antisense primers, respectively, PCR was carried out under the same condition as that for obtaining PCR product A to obtain a PCR product of about 1700 bp. This was designated PCR product B.

PCR products A and B described above were mixed, and annealing and extension reactions (3 cycles of 94°C for 1 minute, 55°C for 30 seconds and 72°C for 2 minutes and 30 seconds) were carried out using a DNA polymerase (*Pyrobest* DNA polymerase; Takara Bio Inc.) to obtain a product of about 4000 bp. This product was TA-cloned into pCR2.1-TOPO vector using TOPO TA Cloning kit (Invitrogen Inc.), and the base sequence was analyzed. As shown in SEQ ID NO: 6 which consisted of 2242 bases from the start codon ATG of the EML4 gene to the exon 20 and 1691 bases from the ALK gene exon 21 to the stop codon of the exon 30 was obtained.

(2) Method for detecting mRNA of EML4-ALK fusion polynucleotide v1 and v2

Using 1 ng of FLAG-EML4-ALKv1/pMX-iresCD8 described in (1) as the template of EML4-ALK fusion polynucleotide v1 and 1 ng of EML4-ALKv2 partial/ pT7Blue-2 in Example 3 as the template for EML4-ALK fusion polynucleotide v2, and using a primer set [primer set for EML4-ALK fusion polynucleotide v1; (SEQ ID NO: 61 and SEQ ID NO: 62, SEQ ID NO: 63 and SEQ ID NO: 64, SEQ ID NO: 65 and SEQ ID NO: 66, SEQ ID NO: 67 and SEQ ID NO: 68, SEQ ID NO: 69 and SEQ ID NO: 70, SEQ ID NO: 71 and SEQ ID NO: 72, SEQ ID NO: 73 and SEQ ID NO: 74, SEQ ID NO: 75 and SEQ ID NO: 76, SEQ ID NO: 77 and SEQ ID NO: 78, or SEQ ID NO: 79 and SEQ ID NO: 80) and the primer set for EML4-ALK fusion polynucleotide v2; (SEQ ID NO: 81 and SEQ ID NO: 62, SEQ ID NO: 83 and SEQ ID NO: 84, SEQ ID NO: 85 and SEQ ID NO: 68, SEQ ID NO: 87 and SEQ ID NO: 88, SEQ ID NO: 89 and SEQ ID NO: 70, SEQ ID NO: 91 and SEQ ID NO: 92, SEQ ID NO: 93 and SEQ ID NO: 74, SEQ ID NO: 95 and SEQ ID NO: 96, SEQ ID NO:

97 and SEQ ID NO: 98, or SEQ ID NO: 99 and SEQ ID NO: 100)], PCR (30 cycles of 94°C for 15 seconds, 55°C for 30 seconds and 72°C for 1 minute) was carried out by a DNA polymerase (rTaq DNA polymerase; Takara Bio Inc.). As the result, in all the primer set, a single DNA fragment, each having the expected size (about from 260 to 350 bp), was amplified. From the above results, it was confirmed that detection of the presence of the EML4-ALK fusion polynucleotide v1 and/or v2 is possible by carrying out RT-PCR according to the present Example using mRNA extracted from the samples of test subjects as the template.

The results of Example 3 and 4(2) described above indicated that the presence of the EML4-ALK fusion polynucleotide v1 and v2 can be detected by using either cDNA or genomic DNA prepared from the clinical specimens obtained from test subjects. This fact suggests that patients having the EML4-ALK fusion polynucleotide can be selected and that a tailor-made treatment can be practiced by which the patients to be treated by the administration of inhibitors of the EML4-ALK fusion polynucleotide and/or polypeptide are selected beforehand and then treated.

[Example 5]

Detection of EML4-ALK fusion polynucleotide v1 in sputum

(1) Production of mouse BA/F3 cells expressing FLAG-EML4-ALKv1

A recombinant retrovirus was produced by a similar method as described before using FLAG-EML4-ALKv1/pMX-iresCD8 and a blank vector (pMX-iresCD8), and mouse lymphatic cell line BA/F3 cells were infected therewith. Cells expressing CD8 on the cell surface were simply purified using magnetic beads reagent for cell separation and a purification column (anti-CD8 monoclonal antibody bound magnetic beads and MiniMACS purification column, both Miltenyi Biotec Inc.).

(2) Detection of EML4-ALK fusion polynucleotide v1 in sputum

After mixing sputum samples of normal healthy subjects with the BA/F3 cells expressing EML4-ALK fusion polynucleotide v1 (hereinafter v1 expressing BA/F3 cells) at 0/mL, 10 cells/mL, 100 cells/mL, 1000 cells/mL and 10,000 cells/mL, cDNA was synthesized by the standard method. The presence of the EML4-ALK fusion polynucleotide v1 in sputum was examined by carrying out a PCR reaction (50°C for 2 minutes, 95 minutes for 15 minutes and further 40 cycles of the following reaction (94°C for 15 seconds, 60°C for 30 seconds and 72°C for 1 minute)) using the cDNA described above as a substrate, and oligonucleotides consisting of the base sequence represented by SEQ ID NO: 8 and SEQ ID NO: 9 as primers and a quantitative PCR kit (QuantiTect SYBR Green; Qiagen Inc.), and PCR products were obtained. As the results, in every case except at 0/mL, the presence of EML4-ALK fusion polynucleotide v1 could be confirmed.

Conventionally cytopathological examination using sputum samples has been an important diagnostic method for lung cancer diagnosis. This diagnosis for lung cancer is based on the presence of atypical cells in sputum but reliable diagnosis cannot be made unless many lung cancer cells exist in the sputum. However, most of the time, such cases were already in the advanced stage and it has been almost impossible to practice effective early diagnosis for lung cancer. According to the present invention, if the EML4-ALK fusion polynucleotide is present in sputum, it became clear that it can be detected by PCR even if a minute amount.

[Example 6]

Investigation of transformability and tumorgenicity of EML4-ALK fusion polypeptide v1.

(1) Analysis for the EML4-ALK fusion polypeptide v1

EML4-ALK (K589M)/pMXS, in which the 589th amino acid (ATP binding site), a lysine residue, of the EML4-ALK fusion polypeptide v1 was replaced with methionine was produced using EML4-ALKv1/pMXS as a substrate and using a mutation introducing kit (QuickChange Site-Directed Mutagenesis Kit; Stratagene Inc.). In the reaction, oligonucleotides of SEQ ID NO: 103 and SEQ ID NO: 104 were used. The ALK cDNA (Morris, SW et al, Science. 1994 Mar 4; 263 (5151):1281-4) was cloned to a retrovirus vector pMXS according to the standard method (designated as ALK/ pMXS).

EML4-ALKv1/pMXS described above, ALK/pMXS, a plasmid expressing EML4-ALK (K589M)/pMXS and a blank vector without inserted cDNA (pMXS) were transfected to 3T3 fibroblast cells by the phosphate calcium method and cultured for 21 days. As shown in the upper part of FIG. 3, many transformation foci were observed only when the EML4-ALK fusion polypeptide v1 expressing vector was transfected. The scale bar indicates 100 μm. Further, the same transfected 3T3 cells were inoculated subcutaneously to nude mice at 5 x $10^5$ cells/mouse and observed for 20 days.

It turned out also that tumor was formed only when EML4-ALK fusion polypeptide v1 expressing cells were inoculated. The tumor formation numbers (the number of inoculation sites of 3T3 cells and the number of tumor formation among them) are as follows. The tumor formation number of the full length ALK expression was 0 among 8, while the tumor formation number in the EML.4-ALK fusion polypeptide v1 expressing cells was 8 among 8. In addition the tumor formation number of EML4-ALK (K589M) expressing cells was 0 among 8. These results demonstrate that since the full length ALK polypeptide expression does not induce tumor but the EML4-ALK fusion polypeptide v1 is tumorgenic, the EML4-ALK fusion polynucleotide v1 is the causal gene of cancer. Also, since the tumorgenicity of EML4-ALK was not observed in EML4-ALK (K589M), it would appear that the tumorgenicity was dependent on the kinase activity. Hereinafter, the 3T3 cells in which EML4-ALK fusion polypeptide v1 is expressed are designated as the v1 expressing 3T3 cells.

(2) Analysis of various deletion mutants of EML4-ALK fusion polynucleotide v1

Various deletion mutants (ΔBasic deletion mutant (31-140th amino acids of the EML4-ALK fusion polypeptide v1 were deleted), ΔHELP deletion mutant (220-296th amino acids of the EML4-ALK fusion polypeptide v1 were deleted), ΔWD deletion mutant (305-475th amino acids of the EML4-ALK fusion polypeptide v1 were deleted)) of the EML4 part of the EML4-ALK fusion polynucleotide v1 was prepared by PCR reaction using FLAG-EML4-ALKv1/ pMX-iresCD8 as a template and using a cloning kit (ExSite PCR-based Site-Directed Mutagenesis; Stratagene Inc.). Using these deletion mutant plasmids, retrovirus solutions were prepared using a similar method to that of Example 1 to obtain infected 3T3 cells. These respective infected cells were inoculated subcutaneously to nude mice to investigate the tumorgenicity, and it was found that tumors were formed by 3T3 cells expressing ΔHELP deletion mutant and ΔWD deletion mutant The tumor forming number of respective 3T3 cells expressing ΔBasic deletion mutant, ΔHELP deletion mutant and ΔWD deletion mutant was 0 among 8, 7 among 8 and 8 among 8, respectively. Since no tumor formation was observed in the ΔBasic deletion mutant, it is demonstrated that the 31-140th amino acids of the EML4-ALK fusion polypeptide v1 are important in tumorgenesis. Since the EML4-ALK fusion polypeptide v2, like the EML4-ALK fusion polypeptide v1, appears to contain the aforementioned the 31-140th amino acids and the ALK kinase region, the EML4-ALK fusion polynucleotide v2, like the EML4-ALK fusion polynucleotide, is considered to be the causal gene of cancer which codes for the polypeptide having the transformability and tumorgenicity to 3T3 cells.

[Example 7]

Method for screening for the EML4-ALK fusion polypeptide inhibitors

(1) Preparation the EML4-ALK fusion polypeptide v1

The v1 expressing BA/F3 cells (Example 5(1)) were cultured in RPM1640 medium containing 10% of fetal bovine serum to obtain $2.7 \times 10^9$ cells. After washing 3 times with PBS, cells were lysed in a lysis solution (50 mM Tris-HCl (pH7.4), 150 mM NaCl, 1%Triton X100, 5 mM EDTA, 5 mM EGTA, 1 mM $NaVO_4$, 1 mM DTT and protease inhibitor cocktail complete). The EML4-ALK fusion polypeptide v1 present in the supernatant obtained after a centrifugation was purified using ANTI-FLAG M2 Affinity Gel (SIGMA-ALDRICH Inc) according to the method described in the product information document. For washing and elution, the washing solution (50 mM Tris-HCl (pH7.4), 250 mM NaCl, 0.05%Brij35, 1 mM EDTA, 1 mM EGTA, 1 mM $NaVO_4$, 1 mM DTT, complete) and the elution solution (50 mM Tris-HCl (pH7.4), 150 mM NaCl, 0.05%Brij35, 1 mM DTT, 0.5 mg/mL FLAG peptide) were used, respectively. Immunoblotting using anti-ALK antibody and anti FLAG M2 antibody (SIGMA-ALDRICH Inc.) and silver staining were carried out for the eluate to detect the EML4-ALK fusion polypeptide v1. It was demonstrated that the EML4-ALK fusion polypeptide v1 can be prepared by this method.

(2) Detection of the *in vitro* kinase activity of the EML4-ALK fusion polypeptide v1

The EML4-ALK fusion polypeptide v1 purified as described above was diluted in a reaction solution (15 mM Tris-HCl (pH7.4), 0.25 mM $MgCl_2$, 0.01% Tween-20, 2 mM DTT), and then ATP was not added or ATP 20 μM was added. The respective mixtures were reacted at room temperature for 1 hour. After the reaction, the auto phosphorylated EML4-ALK fusion polypeptide v1 and the EML4-ALK fusion polypeptide v1 were detected by immunoblotting using anti-phosphorylated ALK antibody, which recognizes specifically the product phosphorylated at the 1604th tyrosine residue of ALK, and anti-ALK antibody, and quantitated by an image analysis system (VersaDoc Imaging System; Bio-Rad Inc.). The amount of phosphorylation was calibrated by dividing the count of the autophosphorylated EML4-ALK fusion polypeptide v1 by the count of the EML4-ALK fusion polypeptide v1. As the result, the autophosphorylated EML4-ALK fusion polypeptide v1 band was detected at the location of about 130 kDa under the condition of ATP addition, and the amount of phosphorylation was increased by about 205 folds compared to no-ATP addition.

In addition, the phosphorylation activity toward a peptide substrate was investigated using a kinase activity detection kit (HTRF KinEASE-TK; Cisbio Inc.). Using TK substrate 1, which was included in the kit, as the substrate, and after adding no ATP or 100 μM ATP, the mixtures were reacted at room temperature for 1 hour, and the count of HTRF was detected as recommended by the Kits manufacturer. As the result it became clear that the count of HTRF (that is, phosphorylation of the peptide substrate) was increased by about 12 times by the addition of ATP compare to no addition of ATP. As shown above, *the in vitro* kinase activity of the EML4-ALK fusion polypeptide v1 can be detected using anti-phosphorylated ALK antibody and the kinase activity detection kit.

(3) Inhibitory effect of compounds against the *in vitro* kinase activity of the EML4-ALK fusion polypeptide v1

The inhibitory effect of compound A-D against the *in vitro* kinase activity of the EML4-ALK fusion polypeptide v1 was investigated using anti-phosphorylated ALK antibody and the kinase activity detection kit. Respective compounds were added to the reaction solution containing the EML4-ALK fusion polypeptide v1 at a final concentration of 10 μM or 10 nM, and then the reaction was carried out with or without the addition of ATP. The rest of the operations were carried out according to the method (2) described above. In the absence of the compound, the phosphorylation count without ATP addition and with ATP addition was assumed to be 100% inhibition and 0% inhibition, respectively. The inhibition (%) of the kinase activity of the EML4-ALK fusion polypeptide v1 by a compound was calculated by the following formula.

**[Kinase activity inhibition (%) by a compound] = (1-[phosphorylation count when the compound and ATP were added - phosphorylation count when the compound was not added and ATP was not added]/[phosphorylation count when the compound was not added and ATP was added - phosphorylation count when the compound was not added and ATP was not added]) × 100**

As the result, it was found that compound A-D inhibited the phosphorylation activity of the purified EML4-ALK fusion polypeptide v1 on itself and the peptide substrate (Table 1). Compound A and B could be selected as substances which inhibited the activity of the EML4-ALK fusion polypeptide v1 by 50% or more at a concentration of 10 μM or less, and compound C and D could be selected as substances which inhibited the activity of the EML4-ALK fusion polypeptide v1 by 50% or more at a concentration of 0.1 μM or less.

Table 1

| Compound | Final concentration | Inhibition on autophosphorylation | Inhibition on peptide substrate |
|---|---|---|---|
| A | 10 μM | 104% | 99% |
| B | 10 μM | 68% | 56% |
| C | 10 nM | 102% | 99% |
| D | 10 nM | 96% | 99% |

The above results indicated that screening (an *in vitro* type screening) for a substance which inhibits the activity of the polypeptide of the present invention could be performed by preparing the EML4-ALK fusion polypeptide and using the *in vitro* kinase activity as an index.

(4) Inhibitory effect of EML4-ALK fusion polypeptide v1 inhibitors on intracellular autophosphorylation

(4-1) BA/F3 cells

Compound A (1 μM, 5 μM and 10 μM) was added to the culture medium of v1 expressing BA/F3 cells (Example 5(1)) or not added, and cultured for 3 hours. In addition, BA/F3 cells expressing FLAG-EML4-ALKv1(K589M) were produced using pMX-iresCD8 vector in which EML4-ALK (K589M) was integrated so that FLAG can be added, and cultured. After culturing, cells were counted and the level of phosphorylation of tyrosine of EML4-ALK fusion polypeptide v1 was measured by immunoblotting using anti-phosphorylated ALK antibody. Further, for the same transfer membrane, immunoblotting analysis by anti-FLAG tag antibody (Eastman Kodak Inc.) was carried out, and total protein quantity of the FLAG attached EML4-ALK fusion polypeptide v1 was measured. As shown in the upper part of FIG. 4, tyrosine phosphorylation level of the EML4-ALK fusion polypeptide v1 was detected in v1 expressing BA/F3 cells, but no tyrosine phosphorylation was detected when EML4-ALK (K589M) was expressed. This fact indicates that the tyrosine phosphorylation of the EML4-ALK fusion polypeptide v1 detected in BA/F3 cells is an autophosphorylation by the EML4-ALK fusion polypeptide v1 itself. Also, it has been confirmed that compound A inhibits the intracellular autophosphorylation of the EML4-ALK fusion polypeptide v1 in a concentration-dependent manner. In addition, it has been shown that the amount of protein expression itself of the EML4-ALK fusion polypeptide v1 in all the samples has been almost constant (FIG. 4, lower part).

Inhibitory effect of compound B-D on the intracellular autophosphorylation was examined in a similar manner as described above. However, the culturing time after the addition of the compounds was 6 hours, and the total protein amount of the EML4-ALK fusion polypeptide v1 was measured using anti-ALK antibody. In addition, the amount of phosphorylation was calculated by quantitating as in Example 7(2). Also, for compound A, the amount of phosphorylation was calculated by quantitating in experiment under this condition. The rate of inhibition was calculated from the amount of phosphorylation when the compound was added, using the value when the compound was not added (the solvent of the compound, DMSO was added) as a control (0% inhibition). Every compound clearly inhibited the kinase activity of the EML4-ALK fusion polypeptide v1 in BA/F3 cells (Table 2). Compound A and B can be selected as the substance which inhibit the EML4-ALK fusion polypeptide v1 activity by 50% or more at a concentration of 10 μM or less, and compound C and D can be selected as the substance which inhibit the EML4-ALK fusion polypeptide v1 activity by 50% or more at a concentration of 0.1 μM or less, and it has been demonstrated that substances which inhibit the activity of the polypeptide of the present invention can be screened (cell-based screening).

(4-2) 3T3 cells

In a similar manner as in (4-1) except compound A-D were added to v1 expressing 3T3 cells (Example 6(1)) at 10 μM or 10 nM and cultured for 4 hours, the amount of phosphorylation of tyrosine of EML4-ALK fusion polypeptide v1 and the total protein amount of the EML4-ALK fusion polypeptide v1 were measured, and the inhibition rate of respective compounds on intracellular kinase activity was calculated. Each compound inhibited clearly the kinase activity of the EML4-ALK fusion polypeptide v1 in the v1 expressing 3T3 cells (Table 2). It became clear that various cells such as BA/F3 cells, 3T3 cells and the like can be used as cells expressing the polypeptide of the present invention in the cell-based screening method of the present invention.

Table 2

| Compound | Final concentration | Inhibition on autophosphorylation (BA/F3cells) | Inhibition on autophosphorylation (3T3cells) |
|---|---|---|---|
| A | 10 μM | 74% | 82% |
| B | 10 μM | 77% | 49% |
| C | 10 nM | 84% | 77% |
| D | 10 nM | 90% | 86% |

The above results indicated that the kinase activity inhibiting compound of the EML4-ALK fusion polypeptide v1 activity can be obtained by using as an index the autophosphorylation in the cells expressing the EML4-ALK fusion polypeptide v1.

[Example 8]

Growth inhibitory effect of the inhibitors of EML4-ALK fusion polypeptide on cells expressing the EML4-ALK fusion polynucleotide v1

(1) Growth potential of v1 expressing BA /F3

For growth of BA/F3 cells expressing only CD8 protein (Example 5(1)), BA/F3 cells expressing CD8 as well as ALK, the EML4-ALK fusion polypeptide v1 (Example 5(1)) or the EML4-ALK (K589M) defecting kinase activity, the change in the number of cells starting from $8 \times 10^5$ cells in the time course was counted in the presence or absence of a growth factor IL-3. The results are shown in FIG. 5. The v1 expressing BA/F3 cells can grow with or without IL-3. However BA/F3 cells expressing only CD8 did grow in the presence of IL-3 but died rapidly when IL-3 was removed. This fact indicates that the EML4-ALK fusion polynucleotide v1 has an activity as oncogene. Further, cells expressing the full length human ALK and BA/F3 cells expressing EML4-ALK (K589M) that defect kinase activity similarly died in the absence of IL-3. These results indicate that cells obtain the growth potential, even in the absence of the growth factor, by expressing the EML4-ALK fusion polypeptide v1 and that the growth potential is dependent on the kinase activity of the EML4-ALK fusion polypeptide v1. BA/F3 cells expressing the full length ALK was obtained according to Example 5 (1).

(2) Growth inhibitory effect of the inhibitors of the EML4-ALK fusion polypeptide on v1 expressing BA/F3 cells

Next, compound A, which is a substance that inhibits the EML4-ALK fusion polypeptide v1, was added to BA/F3 cells which obtained the IL-3 independent growth potential by expressing the EML4-ALK fusion polypeptide v1, and its effect on cell growth was investigated. When compound A was added at 1 μM, 5 μM or 10 μM, or not added (0 μM) to the control, CD8 expressing BA/F3 cells, which grow in the presence of IL-3 and the cell growth was measured, cells could grow although a slight growth inhibition was observed as shown in FIG. 6(a). On the other hand, when compound A was added to v1 expressing BA/F3 cells which were growing in the absence of IL-3, the cell growth was markedly inhibited by concentration-dependence of compound A and cell death was induced as shown in FIG. 6(b). That is, it was confirmed that cells, growing dependently on the EML4-ALK fusion polynucleotide (oncogene), were killed by an inhibitor of the EML4-ALK fusion polypeptide v1.

(3) Inhibitory effect of the inhibitors of EML4-ALK fusion polypeptide on anchorage independent growth of cells expressing the EML4-ALK fusion polypeptide v1 and the full length ALK polypeptide

Measurement for anchorage independent cell growth (colony method, etc) has been known to be a system for investigating an antitumor effect (pharmacologic effect) of compounds (Clinical Oncology, second edition, Cancer and Chemotherapy Publishers Inc.). In place of the colony method, there is a following method using spheroid plates for measuring the growth of non-attached cells.

The v1 expressing 3T3 cells (Example 6(1)) and one of the human glioma cells expressing the full length ALK polypeptide and not expressing EML4-ALK fusion polynucleotide endogenously, U-87 MG cells, were seeded to a 96 well spheroid plate (Sumilon Celltight Spheroid 96U, Sumitomo Bakelite Inc.) at 3000 cells per well in a medium containing 10% fetal bovine serum (DMEM for v1 expressing 3T3 cells and RPMI 1640 for U-87MG). Under 5% $CO_2$, cells were cultured overnight at 37°C, and then compound A or B was added to a final concentration of 10 μM, compound C or D was added to a final concentration of 10 nM and as a negative control the solvent of the compounds, DMSO, was added to make the same concentration as the compounds. At the same time, cells were counted before the addition of drugs (Day 0). Then, cells were cultured under 5% $CO_2$, at 37°C for 3 days, mixed with a reagent for measuring cell number (CellTiter-Glo™ Luminescent Cell Viability Assay; Promega Inc.) stirred for 20 minutes, and the measurements (day 3) were carried out using a luminometer (ML3000 microtiter plate luminometer; Dynatech Laboratories Inc.). The results show that all the compounds had growth inhibitory activity on v1 expressing 3T3 cells but almost no inhibitory activity on U-87MG cells. The inhibition rate of the compounds was calculated assuming the cell number at Day 0 and Day 3 were 100% inhibition and 0% inhibition, respectively (Table 3).

Table 3

| Compound | Final concentration | v1 expressing 3T3 cells | U-87MG cell |
|---|---|---|---|
| A | 10 μM | 106% | 15% |
| B | 10 μM | 91% | 34% |
| C | 10 nM | 131% | -2% |
| D | 10 nM | 135% | -2% |

Above results indicate that compound A-D inhibited the anchorage independent cell growth of v1 expressing 3T3 cells by inhibiting the kinase activity of the EML4-ALK fusion polypeptide v1. In addition, it became clear that these compounds could not inhibit the anchorage independent cell growth of U-87MG cells expressing the full length ALK polypeptide. These results indicate that the inhibitors of EML4-ALK fusion polypeptide can inhibit the growth of cancer cells and tumors which express the EML4-ALK fusion polypeptide.

(4) Preparation of siRNA to the EML4-ALK fusion polynucleotide- v1

siRNAs, which were composed of a sense strand consisting of the base sequences represented by SEQ ID NO: 111, 113, 115, 117, 119, or 121, and an antisense strand consisting of the base sequences represented by SEQ ID NO: 112, 114, 116, 118, 120, or 122, were prepared as the siRNA (siRNA-1 to siRNA-6) which have a 100% homology to the fusion area of the EML4-ALK fusion polypeptide v1 and is expected to have inhibitory activity on the expression of EML4-ALK fusion polypeptide v1. In addition, siRNAs composed of a sense strand consisting of the base sequences represented by SEQ ID NO: 123 or 125 and an antisense strand consisting of the base sequence represented by SEQ ID NO: 124 or 126 were designed and prepared, as the siRNA (siRNA-7, siRNA-8) which shows 100% homology to the ALK area of the EML4-ALK fusion polypeptide v1 and are expected to have an inhibitory effect on the expression of the ALK gene, using an siRNA sequence design system (Commercial siDirect (registered trade mark) RNAi Inc.). It has been confirmed by the siRNA sequence design system (Commercial siDirect (registered trade mark) RNAi Inc.) that the base sequences corresponding to siRNA-1 to siRN-8 do not show a 100% homology to the gene other than the EML4-ALK fusion polynucleotide and ALK genes. For control experiments to investigate the influence of non specific siRNA, siRNA composed of a sense strand consisting of a base sequence represented by SEQ ID NO: 127 and an antisense strand consisting of a base sequence represented by SEQ ID NO: 128 was prepared as siRNA (siRNA-9) corresponding to a base sequence not present in mammalian cells. siRNA-1 is a product of annealing SEQ ID NO: 111 (sense strand) and SEQ ID NO: 112 (antisense strand), and siRNA-2 and others are the same (FIG. 7).

(5) Inhibitory effect of siRNA on mRNA expression of the EML4-ALK fusion polynucleotide and ALK gene in cells expressing the EML4-ALK fusion polynucleotide v1 and full length ALK

The v1 expressing 3T3 cells and U-87MG cells were seeded to 12 well plates (IWAKI; Asahi techno glass corp.) at 50,000 cells and 150,000 cells, respectively. Four hours later, using a transfection reagent (Lipofectamine RNAiMax; Invitrogen Inc.), siRNA-1 to siRNA-9 were prepared to a final concentration of 20 nM and transfected to cells according to the attached instruction. In addition, as a control no siRNA transfection samples were prepared. After 72 hours, total RNA was extracted and cDNA was prepared.

The amount of expressed mRNA of the EML4-ALK fusion polynucleotide v1 in v1 expressing 3T3 cells and the amount of mRNA of the ALK gene in U-87MG cells were quantitated using a quantitative PCR reagent (Power SYBR Green PCR Master Mix; Applied Biosystems Inc.) The PCR reaction was carried out as follows: after 10 minutes incubation at 95°C, 45 cycles of 95°C for 15 seconds and 60°C for 60 seconds, and then one cycle of 95°C for 15 seconds, 60°C for 15 seconds and 95°C for 15 seconds to complete the reaction. In addition, to calibrate the amount of expression, the amount of expression of the mouse cyclophilin B gene and the human GAPDH gene was similarly quantitated for v1 expressing 3T3 cells and U-87MG cells, respectively. Analyses were carried out using a sequence detector (ABI PRISM 7900 Sequence Detection System; Perkin-Elmer Applied Biosystems Inc.).

Oligo nucleotides consisting of the base sequences represented by SEQ ID NO: 44 and 48, SEQ ID NO: 50 and 56, SEQ ID NO: 44 and 48 and SEQ ID NO: 12 and 13 were used as the primer sets that specifically recognize the EML4-ALK fusion polynucleotide v1, the mouse cyclophilin B gene, the human ALK gene and the human GAPDH gene. Also, to obtain a standard curve to calculate the amount of respective mRNA, PCR was performed using human genomic DNA (Clontech) as a template for EML4-ALK fusion polynucleotide v1, human ALK gene and human GAPDH gene, and using mouse genomic DNA (Clontech) as a template for mouse cyclophilin B and the aforementioned primer sets under the same condition. Since a primer set corresponding to human ALK polynucleotide exon 29 was used to detect the EML4-ALK fusion polynucleotide v1, the standard curve can be obtained using human genomic DNA. The expression amount of the EML4-ALK fusion polynucleotide v1 and the human ALK gene in respective samples was calibrated with the expression amount of the mouse cyclophilin B gene and the human GAPDH gene to obtain the calibrated expression amount. Further, assuming the respective calibrated expression amount of the EML4-ALK fusion polynucleotide v1 and the human ALK gene in the absence of siRNA to be 100%, the relative expression amount of the calibrated expression amount of the EML4-ALK fusion polynucleotide v1 andt human ALK gene was determined and the inhibition rate for expression was calculated when respective siRNAs were transfected (Table 4).

As the result, siRNA-1 to siRNA-8, which correspond to the EML4-ALK fusion polynucleotide v1, inhibited the expression of the EML4-ALK fusion polynucleotide by 50% or more. For the human ALK gene, siRNA-7 and siRNA-8, which correspond to the human ALK gene, inhibited by 50% or more. The negative control, siRNA-9, did not demonstrate strong expression inhibitory effect on the EML4-ALK fusion polynucleotide and the human ALK gene. These results demonstrate that substances inhibiting the expression of the EML4-ALK fusion polynucleotide can be screened.

Table 4

| siRNA | Inhibition rate for EML4-ALK gene expression (v1 expressing 3T3 cells) | Inhibition rate for ALK gene expression (U-87 MG cells) |
|---|---|---|
| siRNA-1 | 80% | -12% |
| siRNA-2 | 66% | 11% |
| siRNA-3 | 62% | 10% |
| siRNA-4 | 86% | 22% |
| siRNA-5 | 76% | -22% |
| siRNA-6 | 69% | 15% |
| siRNA-7 | 67% | 66% |
| siRNA-8 | 70% | 58% |
| siRNA-9 | 29% | -31% |
| siRNA not introduced | 0% | 0% |

(6) Inhibitory effect of siRNA on expression and autophosphorylation of the EML4-ALK fusion polypeptide v1 in v1 expressing 3T3 cells.

By the similar method described before, v1 expressing 3T3 cells were seeded to 12 well plates at 50, 000 cells per well, and 4 hours later, siRNA-1 to siRNA-9 were transfected. In addition, cells to which no siRNA was transfected were prepared as a control. After 72 hours of the transfection, the medium was removed, and the autophosphorylation of the intracellular EML4-ALK fusion polypeptide v1 and protein amount of the EML4-ALK fusion polypeptide v1 were quantitated by the similar method as in Example 7(4). Also, to confirm that total protein in each sample for measurement was the same amount, the actin protein was quantitated using anti-actin antibody (SIGMA-ALDRICH Inc.). siRNA -1 to siRNA-8 were clearly inhibited the expression of the EML4-ALK fusion polypeptide v1 and the kinase activity in the v1 expressing 3T3 cells.

(7) Growth inhibitory effect of siRNA on v1 expressing 3T3 cells and full length ALK expressing cells

Cell growth inhibition rate was calculated with or without transfecting siRNA by the similar method as in Example 8(3), except that each siRNA was added to 96 well spheroid plates beforehand and then v1 expressing 3T3 cells and U-87MG cells were seeded and cultured for 3 days.

Results of these experiments are shown in Table 5. Each siRNA (siRNA-1 to siRNA-8), which had been demonstrated to have inhibitory effect on the expression and kinase activity of the EML4-ALK fusion polypeptide v1 in Example 8(5) (6), strongly inhibited anchorage independent growth of v1 expressing 3T3 cells. Since the cell number of v1 expressing 3T3 cells, to which siRNA-1, 3, 4 and 5 were transfected, was lower at the measuring time (Day 3) than when siRNA was transfected (Day 0) resulting in the growth inhibition rate being over 100%, cell death is believed to have been induced. On the other hand, siRNA-7 and siRNA-8 were shown to inhibit the expression of the ALK gene in Example 8(5) but did not inhibit the growth of U-87MG cells expressing the full length ALK. siRNA-1 to siRNA-6 did not show growth inhibition on U-87MG cells and the negative control, siRNA-9, did not inhibit the growth of v1 expressing 3T3 cells and U-87MG cells.

Table 5

| siRNA | v1 expressing 3T3 cells growth inhibition rate | U-87MG cell growth inhibition rate |
|---|---|---|
| siRNA-1 | 110% | -13% |
| siRNA-2 | 94% | 25% |
| siRNA-3 | 108% | -21% |
| siRNA-4 | 120% | -8% |
| siRNA-5 | 110% | -13% |
| siRNA-6 | 97% | 16% |

(continued)

| siRNA | v1 expressing 3T3 cells growth inhibition rate | U-87MG cell growth inhibition rate |
|---|---|---|
| siRNA-7 | 87% | 15% |
| siRNA-8 | 94% | -21 % |
| siRNA-9 | -35% | -16% |
| siRNA not introduced | 0% | 0% |

From the above results, it became clear that, by transfecting siRNA which is against the EML4-ALK fusion polynucleotide v1 to cancer cells expressing the EML4-ALK fusion polynucleotide v1, the expression of the EML4-ALK fusion polynucleotide v1 mRNA is inhibited resulting in the reduction of the EML4-ALK fusion polypeptide and in the inhibition of autophosphorylation causing the growth inhibition of cancer cells. Also, for cancer cells expressing the full length ALK, the growth inhibition did not occur when the expression of ALK was inhibited. From the above results, it became clear that the siRNA against the EML4-ALK fusion polynucleotide v1, for example, siRNA-1 to siRNA-8, is useful as a therapeutic agent against the tumor expressing the EML4-ALK fusion polynucleotide for the EML4-ALK fusion polynucleotide positive patients.

(8) Anti-tumor test for inhibitors of the EML4-ALK fusion polypeptide against v1 expressing 3T3 cells

$3 \times 10^6$ cells of v1 expressing 3T3 cells suspended in PBS were inoculated subcutaneously by injection to the back of 5 weeks old male BALB/c nude mice (Japan Charles River Inc.). After 7 days of the inoculation, the administration of compound C, an inhibitor of the EML4-ALK fusion polypeptide (Table 1 to 3), was initiated. The test was conducted in the solvent group and compound C group, 4 animals per group. Compound C was dissolved in a solvent composed of 10% 1-methyl-2-pyrrolidinone (SIGMA-ALDRICH Inc.)/90% polyethylene glycol 300 (Fluka Inc.) and administered orally at the dose of 10 mg/kg. Administrations were performed once a day for 14 days, and body weight and tumor size were measured every other day. Tumor volume was calculated using the following formula.

$$[\text{Tumor volume } (\text{mm}^3)] = [\text{Tumor major axis } (\text{mm})] \times [\text{tumor minor axis } (\text{mm})]^2 \times 0.5$$

Assuming the tumor volume of the solvent group on the day of starting and the day of finishing administration was 100% inhibition and 0% inhibition, respectively, the inhibition rate of compound C was calculated. The results indicated that compound C inhibited the growth of v1 expressing 3T3 cells (tumor) by 103%.

The antitumor effect of compound D was investigated by the similar procedure with the following exceptions. The administration of the compound D was started after 6 days of the inoculation and carried out once a day for 10 days, and then the tumor size was measured. Compound D inhibited the growth of v1 expressing 3T3 cells (tumor) by 101%.

(9) Kinase inhibitory effect by repeated administrations of the inhibitors of EML4-ALK fusion polypeptide to v1 expressing 3T3 tumor

The kinase inhibitory effect of compound C was observed by a similar manner as in Example 8(8) with the following exceptions. $1 \times 10^6$ cells of v1 expressing 3T3 cells were inoculated and the administration of compound C was initiated after 13 day of the inoculation. The test was conducted in the solvent group and the compound C group, 3 animals for each group. The administrations were carried out once a day for 3 days. Animals were dissected 4 hours after the last administration, and the tumor was extirpated. Then, protein extracts were prepared from the tissues and immunoblotting was carried out using anti-phosphorylated ALK antibody. The results indicate that in the compound C group, tyrosine autophosphorylation of the EML4-ALK fusion polypeptide v1 in the tumor was significantly decreased compared to the solvent group. From this result it was confirmed that the anti-tumor effect of compound C in the animal model described above was based on the kinase inhibitory effect of the EML4-ALK fusion polypeptide v1 in the tumor.

[Example 9]

Detection of the EML4-ALK fusion polypeptide v1

A method for detecting the EML4-ALK fusion polypeptide v1 in cells was constructed as follows. The v1 expressing 3T3 cells and U-87MG cells were cultured. After washing 3 times with PBS, cells were lysed with the lysis solution (Example 7(1)). To 4 mg of the supernatant obtained after centrifugation, anti-EML4 antibody (Cell Signaling Inc.) was added and reacted overnight at 4°C. Then, protein G beads (Protein G Sepharose 4 Fast Flow; GE Healthcare Inc.) were added and immunoprecipitation was carried out for 2 hours. After centrifugation, the precipitates were washed 3 times with the washing solution (Example 7(1)) and suspended in an SDS dissolving solution. The supernatant was subjected to immunoblotting using anti-ALK antibody. As the result, the EML4-ALK fusion polypeptide v1 was detected in the immunoprecipitates of v1 expressing 3T3 cells but not detected in U-87MG cells. From the results described above, it became possible to detect the presence of the EML4-ALK fusion polypeptide v1 in cancer cells and cancer tissues expressing the EML4-ALK fusion polypeptide v1 using anti-EML4 antibody and anti-ALK antibody in combination, and it became clear that the EML4-ALK fusion polypeptide v1 positive cancer patients can be determined.

[Example 10]

Analysis of the EML4-ALK fusion polypeptide v2

(1) Construction of an expression vector of EML4-ALK fusion polypeptide v2

The vector in which the cleavage sequence of restriction enzyme HindIII was integrated at the 5' terminus side of the start codon of the EML4-ALK fusion polynucleotide v2 (EML4-ALKv2/pCR2.1) was produced by using the pCR2.1-TOPO vector in which the EML4-ALK fusion polynucleotide v2 was cloned in Example 4(1).

EML4-ALKv2/pCR2.1 was digested with restriction enzyme HindIII, the EML4-ALK fusion polynucleotide v2 was excised, the both termini were blunted, an adapter (EcoRI-NotI-BamHI adaptor; Takara Bio Inc.) was then ligated to both termini, and the fragment was inserted to the EcoRI site of a retrovirus vector pMXS. This was designated as EML4-ALKv2/pMXS.

Also, EML4-ALKv2/pCR2.1 was digested with restriction enzyme HindIII and XbaI, the EML4-ALK fusion polynucleotide v2 was excised and inserted to the HindIII/XbaI site of an expression vector pcDNA3.1/Zeo (Invitrogen Inc.) which was modified so that the FLAG tag was attached to the N-terminus on expression to produce an expression plasmid for EML4-ALK fusion polypeptide v2 to which the FLAG tag was attached to the N terminus (FLAG-EML4-ALKv2/pcDNA3).

(2) Investigation of tumorigenicity of EML4-ALK fusion polypeptide v2

FLAG-EML4-ALKv2/pcDNA3 (Example 10(1)) was transfected into 3T3 cells using a transfection regent (FuGENE HD; Roche Diagnostics Inc.) according to the attached instruction. The EML4-ALK fusion polynucleotide v2 stably expressing 3T3 cells were established by resistance to 80 μg/ml zeocin. The expression of EML4-ALK fusion polynucleotide v2 in the 3T3 cells was confirmed by immunoblotting using anti-ALK antibody and anti-phosphorylated ALK antibody. The 3T3 cells in which EML4-ALK fusion polypeptide v2 is expressed are designated as the v2 expressing 3T3 cells. The v2 expressing 3T3 cells were inoculated subcutaneously to 5 weeks old male BALB/c nude mice (Japan Charles River Inc.) at $2 \times 10^6$ cells/mouse and observed for 15 days. As in the case of the v1 expressing cells (the lower section of the figure 3), it turned out that tumor was also formed in EML4-ALK fusion polypeptide v2 expressing 3T3 cells. The tumor formation number was 4 among 4.

From above results, it was confirmed that the EML4-ALK fusion polynucleotide v2, like the EML4-ALK fusion polynucleotide v1, is also an oncogene which codes for the polypeptides having tumorigenicity to 3T3 cells.

(3) Screening for the substances which inhibit intracellular autophosphorylation activity of the EML4-ALK fusion polypeptide v2

To 293EBNA cells (Invitrogen Inc.) seeded in collagen I coated 24 well plates (IWAKI; Asahi techno glass corp.) at $1 \times 10^5$ cells per well in DMEM medium containing 10% fetal bovine serum, 100 ng of FLAG-EMLA-ALKv2/pcDNA3 (Example 10(1) or pcDNA3 (blank vector) as a control was introduced using a transfection reagent (Lipofectamin2000; Invitrogen Inc.). After culturing for 20 hours, compound C or D or DMSO each was added, and the culture was incubated for 4 hours and then cells were recovered. Expression of EML4-ALK fusion polypeptide v2 and tyrosine phosphorylation level were measured by immunoblotting using anti-ALK antibody and anti-phosphorylated ALK antibody.

As the results, in the immunoblot using anti-ALK antibody, a band was confirmed at the location of about 160 kDa where the EML4-ALK fusion polynucleotide v2 was expected to be present, and it was demonstrated that the amount of protein expression itself of the EML4-ALK fusion polynucleotide v2 was almost constant in all the samples. In addition, a band was confirmed at the same location in the immunoblot using anti-phosphorylated ALK antibody. The amount of phosphorylation was calculated by quantitating in a similar manner as in Example 7(2). The inhibition rate of phosphorylation by a compound was calculated from the amount of phosphorylation when the compound was added, assuming the value when no compound was added (the solvent of the compound, DMSO, was added) was 0% inhibition rate, and the value when the empty vector, pcDNA3, was introduced was 100% inhibition rate. The results indicate that each compound clearly inhibited the kinase activity of the EML4-ALK fusion polynucleotide v2 in 293EBNA cells (Table 6). In a similar manner as in Example 7(4-2) except compound A to D or DMSO were added to v2 expressing 3T3 cells (Example 10(2-2)) at 10 μM or 10 nM, the amount of phosphorylation of tyrosine of EML4-ALK fusion polypeptide v2 and the total protein amount of the EML4-ALK fusion polypeptide v2 were measured, and the inhibition rate of respective compounds on intracellular kinase activity was calculated. Each compound inhibited clearly the kinase activity of the EML4-ALK fusion polypeptide v2 in the v2 expressing 3T3 cells (Table 6).

Compound A and B could be selected as substances which inhibited the activity of the EML4-ALK fusion polypeptide v2 by 50% or more at a concentration of 10 μM or less, and compound C and D could be selected as substances which inhibited the activity of the EML4-ALK fusion polypeptide v2 by 50% or more at a concentration of 0.1 μM or less. It is confirmed that screening for the substances inhibiting the activity of the polypeptide of the present invention (cell-based screening) can be performed using the EML4-ALK fusion polypeptide v2 as well as the EML4-ALK fusion polypeptide v1.

Table 6

| Compound | Final concentration | Inhibition on autophosphorylation (293EBNA cells) | Inhibition on autophosphorylation (v2 expressing 3T3 cells) |
|---|---|---|---|
| A | 10 μM | not tested | 68% |
| B | 10 μM | not tested | 55% |
| C | 10 nM | 90% | 73% |
| D | 10 nM | 95% | 88% |

(4) Inhibitory effect of the inhibitors of EML4-ALK fusion polypeptide on anchorage independent growth of v2 expressing 3T3 cells

By a similar manner described as in Example 8(3), inhibitory effect of the inhibitors of EML4-ALK fusion polypeptide on anchorage independent cell growth was investigated using v2 expressing 3T3 cells (Example 10(2-2)). The results showed that all the compounds had growth inhibitory activity on v2 expressing 3T3 cells. The inhibition rate of the compounds was calculated assuming the cell number at Day 0 and Day 3 were 100% inhibition and 0% inhibition, respectively (Table 7).

Table 7

| Compound | Final concentration | v2 expressing cells |
|---|---|---|
| A | 10 μM | 157% |
| B | 10 μM | 121% |
| C | 10 nM | 76% |
| D | 10 nM | 157% |

Above results indicated that compound A-D inhibited the anchorage independent cell growth of v2 expressing 3T3 cells by inhibiting the kinase activity of the EML4-ALK fusion polypeptide v2.

[Example 11]

Analysis of the EML4-ALK fusion polypeptide v3

(1) Detecting EML4-ALK fusion polynucleotide v3 in clinical specimens and cloning of EML4-ALK fusion polynucleotide v3

PCR (after 50°C for 2 minutes and 95°C for 15 minutes, 50 cycles of 94°C for 15 seconds, 60°C for 30 seconds and 72°C for 90 seconds) was carried out using 75 cDNA prepared from the clinical specimens and a quantitative PCR kit as those in Example 3(1) and oligonucleotides of SEQ ID NO: 131 and 82 as primers. As a result, in two cases, it was confirmed a sequence of 515 bp (SEQ ID NO:86) or 548 bp (SEQ ID NO:90) made by fusion of a part of EML4 and a part of ALK, which has a fusion point different from EML4-ALK fusion polynucleotide v1 and EML4-ALK fusion polynucleotide v2, was amplified Using the cDNA of a patient that is shown to be positive for PCR product described above as a template and oligonucleotides of SEQ ID NO: 94 and 46 as primers, PCR (35 cycles of 98°C for 10 seconds and 68°C for 6 minutes) was carried out with a DNA polymerase (PrimeStar HS DNA polymerase, Takara Bio Inc.). The PCR product was cloned in pT7Blue-2 vector. As a result, new polynucleotide (SEQ ID NO: 129) which consisted of 700 bases from the start codon ATG of the EML4 gene to the portion of intron 6 and 1691 bases from the ALK gene exon 21 to the stop codon of the exon 30 was obtained. The plasmid in which the cDNA of SEQ ID NO: 129 was cloned was designated as EML4-ALKv3/pT7Blue-2.

(2) Investigation of tumorgenicity of EML4-ALK fusion polypeptide v3

Using the EML4-ALKv3/pT7Blue-2, the EML4-ALK fusion polypeptide v3 expression vector (designated as EML4-ALKv3/pMXS) was constructed according to a method of Example 4(1). Also, using the EML4-ALKv3/pT7Blue-2, an expression vector (designated as FLAG-EML4-ALKv3/pMX-iresCD8) which expresses both EML4-ALK fusion polypeptide v3 having the FLAG tag at the N-terminus and CD8 was constructed using a similar method of Example 4(1).

The 3T3 cells trandfected with the EML4-ALKv3/pMXS described above were inoculated subcutaneously to nude mice at $5 \times 10^6$ cells/mouse and observed after 20 days. It turned out that tumor was formed. Because the EML4-ALK fusion polypeptide v3 was tumorgenic, it was indicated the EML4-ALK fusion polynucleotide v3 is the causal gene of cancer as same as v1 and v2. Hereinafter, the 3T3 cells in which EML4-ALK fusion polypeptide v3 is expressed are designated as the v3 expressing 3T3 cells.

(3) Method for detecting mRNA of EML4-ALK fusion polynucleotide v3

Using 1 ng of the plasmid which expresses the EML4-ALKv3 as the template, and (SEQ ID NO: 132 and SEQ ID NO: 62, SEQ ID NO: 133 and SEQ ID NO: 64, SEQ ID NO: 134 and SEQ ID NO: 66, SEQ ID NO: 135 and SEQ ID NO: 68, SEQ ID NO: 136 and SEQ ID NO: 70, SEQ ID NO: 137 and SEQ ID NO: 72, SEQ ID NO: 138 and SEQ ID NO: 74, SEQ ID NO: 139 and SEQ ID NO: 76, SEQ ID NO: 140 and SEQ ID NO: 78, and SEQ ID NO: 141 and SEQ ID NO: 80) as the primer set, PCR (30 cycles of 94°C for 15 seconds, 55°C for 30 seconds and 72°C for 1 minute) was carried out by a DNA polymerase (rTaq DNA polymerase; Takara Bio Inc.). As the result, in all the primer set, a single DNA fragment, each having the expected size was amplified. From the above results, it was proved that detection of the presence of the EML4-ALK fusion polynucleotide v3 is possible by carrying out RT-PCR according to the present Example using mRNA extracted from the samples of test subjects as the template.

(4) Method for screening for the EML4-ALK fusion polypeptide v3 inhibitors

According to the method of Example 1, Example 5(1), and Example 7(1), the EML4-ALK fusion polypeptide v3 was prepared by using FLAG-EML4-ALKv3/pMX-iresCD8.

The *in vitro* kinase activity of the EML4-ALK fusion polypeptide v3 purified as described above can be detected using a method of Example 7(2). The inhibitory effect of compound A-D against the *in vitro* kinase activity of the EML4-ALK fusion polypeptide v3 was investigated using the method according to method of Example 7(3). As the result, it was found that compound A-D inhibited the autophosphorylation and the phosphorylation activity on the peptide substrate by the purified EML4-ALK fusion polypeptide v3 (Table 8). These compounds could be selected as substances which inhibited the activity of the EML4-ALK fusion polypeptide v3.

Table 8

| Compound | Final concentration | Inhibition on autophosphorylation | Inhibition on peptide substrate |
|---|---|---|---|
| A | 10 μM | 100% | 98% |
| B | 10 μM | 100% | 77% |
| C | 10 nM | 103% | 100% |
| D | 10 nM | 107% | 98% |

The above results indicated that screening (an *in vitro* type screening) for a substance which inhibits the activity of the polypeptide of the present invention could be performed by preparing the EML4-ALK fusion polypeptide v3 and using the *in vitro* kinase activity as an index.

(5) Screening for the substances which inhibit an intracellular autophosphorylation activity of the EML4-ALK fusion polypeptide v3

DMSO (control) or compound A-D were added to v3 expressing 3T3 cells (Example 11(2)) at 10 μM or 10 nM and the amount of phosphorylation of tyrosine of EML4-ALK fusion polypeptide v3 and the total protein amount of the EML4-ALK fusion polypeptide v3 were measured and the inhibition rate of respective compounds on intracellular kinase activity was calculated in a similar manner as in Example 7(4-2). As the results, in the immunoblot using anti-ALK antibody, the bands were confirmed at the location of about 90 kDa where the EML4-ALK fusion polynucleotide v3 was expected at the almost constant level in all the samples. In addition, the bands were confirmed at the same location in the immunoblot using anti-phosphorylated ALK antibody.

Each compound clearly inhibited the kinase activity of the EML4-ALK fusion polypeptide v3 in the v3 expressing 3T3 cells (Table 9). It was confirmed that screening for the substances inhibiting the activity of the polypeptide of the present invention (cell-based screening) can be performed using the EML4-ALK fusion polypeptide v3.

Table 9

| Compound | Final concentration | Inhibition on autophosphorylation (v3 expressing 3T3 cells) |
|---|---|---|
| A | 10 μm | 94% |
| B | 10 μm | 76% |
| C | 10 nM . | 95% |
| D | 10 nM | 106% |

(6) Inhibitory effect of the inhibitors of EML4-ALK fusion polypeptide on anchorage independent growth of the v3 expressing 3T3 cells

By a similar manner described as in Example 8(3), inhibitory effect of the inhibitors (compound A-D) of EML4-ALK fusion polypeptide on anchorage independent growth of cells was investigated using v3 expressing 3T3 cells (Example 11 (2)). The results showed that all the compounds had growth inhibitory activity on v3 expressing 3T3 cells (Table 10).

Table 10

| Compound | Final concentration | v2 expressing cells |
|---|---|---|
| A | 10 μm | 172% |
| B | 10 μm | 53% |
| C | 30 nM | 182% |
| D | 30 nM | 206% |

Above results indicate that compound A-D inhibited the anchorage independent cell growth of v3 expressing 3T3 cells by inhibiting the kinase activity of the EML4-ALK fusion polypeptide v3.

(7) The expression and cloning of EML4-ALK fusion polypeptide v3 mRNA in NCI-H2228 cells

Using the cDNA which prepared by reverse transcription from total RNA of NCI-H2228 cells, a human non-small cell lung cancer cell line (American Type Culture Collection) as a template, PCR (35 cycles of 98°C for 10 seconds, 68°C for 4 minute) was carried out using oligonucleotides represented by the SEQ ID NO: 94 and the SEQ ID NO: 46 as primers and a DNA polymerase (primeSTAR HS DNA polymerase, Takara Bio Inc.).. Using a part of the PCR product as a template, PCR (35 cycles of 98°C for 10 seconds, 68°C for 4 minute) was carried out using an oligonucleotide represented by the SEQ ID NO:105 which was provided with the cleavage sequence of restriction enzyme HindIII at the 5' terminus side of the start codon ATG of the EML4 gene and an oligonucleotide represented by SEQE ID NO:106 which was provided with the cleavage sequence of restriction enzyme Xba I at the 3' terminus side of stop codon TGA of ALK gene as primers and a DNA polymerase *(Pyrobest* DNA polymerase; Takara Bio Inc.). PCR product of about 2400 bp was obtained. This product was TA-cloned into pCR2.1-TOPO vector using TOPO TA Cloning kit (Invitrogen Inc.), and DNA sequence was analyzed. As a result, two kinds of polynucleotide were identified. One was a 2391 bp sequence (SEQ ID NO:107) which was different in 4 bases, namely, 685th, 903rd, 2000th, and 2115th bases from SEQ ID NO:129 (Example 11(1)). The another was a 2358 bp sequence (SEQ ID NO: 109) which consisted of 667 bases from the start codon ATG of the EML4 gene to the exon 6 and 1691 bases from the ALK gene exon 21 to the stop codon of the exon 30. The portion corresponding to these ALK gene was different about 1 base from the sequence (4080-5770) of NM_004304 registered in Genbank, without the aminoacid variation. Therefore, It was suggested that the differences in portion of ALK gene between the polynucleotide (SEQ ID NO: 129) identified in lung cancer patients and the polynucleotide (SEQ ID NO:: 107) cloned from NCI-H2228 were the polymorphism.

Neither the EML4-ALK fusion polynucleotide vl nor the EML4-ALK. fusion polynucleotide v2 endogenously expressed in NCI-H2228 cells.

(8) Screening for the substances which inhibit an intracellular autophosphorylation activity of the EML4-ALK fusion polypeptide on NCI-H2228 cells

DMSO (control) or compound C was added to NCI-H2228 cells at 100 nM as a final concentration and the amount of phosphorylation of tyrosine of EML4-ALK fusion polypeptide and the inhibition rate of compound C on autophosphorylation activity was calculated in a similar manner as in Example 11(5). Compound C inhibited the kinase activity of the EML4-ALK fusion polypeptide in the NCI-H2228 cells at 81%. It is confirmed that screening for the substances inhibiting the activity of the EML4-ALK fusion polypeptide of the present invention (cell type screening) can be performed by using as an index the autophosphorylation in the NCI-H2228 cells.

(9) Inhibitory effect of the inhibitors of EML4-ALK fusion polypeptide on anchorage independent growth of NCI-H2228 cells.

DMSO (control) or compound A-D was added to the NCI-H2228 cells at 10 μM or 10 nM and this investigation was performed by a similar manner described as in Example 8(3), except cells were cultured for 5 days after the addition of drugs. The results showed that the compound A-D had growth inhibitory activity on the NCI-H2228 cells. The inhibition rate of the compounds was calculated assuming the cell number at Day 0 and Day 5 were 100% inhibition and 0% inhibition, respectively (Table 11).

Table 11

| Compound | Final concentration | NCI-H2228 cells |
|---|---|---|
| A | 10μm | 164% |
| B | 1.0 μM | 102% |
| C | 30 nM | 137% |
| D | 30 nM | 141% |

Above results indicate that compound A-D inhibited the anchorage independent cell growth of NCI-H2228, non-small cell lung cancer cells, by inhibiting the kinase activity of the EML4-ALK fusion polypeptide. From the above results, it became clear that inhibitor against the EML4-ALK fusion polynucleotide is useful as a therapeutic agent for the EML4-ALK fusion polynucleotide positive lung cancer patients.

## Claims

**1.** A method for detecting a fusion gene of echinoderm microtubule-associated protein like protein 4 (EML4) gene and anaplastic lymphoma kinase (ALK) gene, comprising the step of detecting the presence of a polynucleotide in a sample obtained from a test subject, wherein the polynucleotide encodes a polypeptide selected from the group consisting of: ’

(1) a polypeptide comprising the amino acid sequence represented by SEQ ID NO: 2, 7 or 130 and having a kinase activity,
(2) a polypeptide comprising an amino acid sequence derived from the amino acid sequence represented by SEQ ID NO: 2 or 7 with the deletion, substitution, and/or insertion of 1 to 10 amino acids and having a kinase activity,
(3) a polypeptide comprising an amino acid sequence with 90% or higher identity to the amino acid sequence represented by SEQ ID NO: 2 or 7 and having a kinase activity, and
(4) a polypeptide consisting of the amino acid sequence represented by SEQ ID NO: 2, 7 or 130.

**2.** A method for detecting a fusion protein encoded by a fusion gene of EML4 gene and ALK gene, comprising the step of detecting the presence of a polypeptide in a sample obtained from a test subject, wherein the polypeptide is selected from the group consisting of:

(1) a polypeptide comprising the amino acid sequence represented by SEQ ID NO: 2, 7 or 130 and having a kinase activity,
(2) a polypeptide comprising an amino acid sequence derived from the amino acid sequence represented by SEQ ID NO: 2 or 7 with the deletion, substitution, and/or insertion of 1 to 10 amino acids and having a kinase activity,
(3) a polypeptide comprising an amino acid sequence with 90% or higher identity to the amino acid sequence represented by SEQ ID NO: 2 or 7 and having a kinase activity, and
(4) a polypeptide consisting of the amino acid sequence represented by SEX ID NO: 2, 7 or 130.

**3.** The method of claim 1 or claim 2, wherein the polypeptide comprises the amino acid sequence represented by SEQ ID NO: 2, 7 or 130 and having a kinase activity.

**4.** The method of claim 1 or claim 2, wherein the polypeptide consists of the amino acid sequence represented by SEQ ID NO: 2, 7 or 130.

**5.** The method of claim 1 or claim 2, wherein the polypeptide comprises the amino acid sequence represented by SEQ ID NO: 2 or 7 and having a kinase activity.

**6.** The method of claim 1 or claim 2, wherein the polypeptide consists of the amino acid sequence represented by SEPA ID NO: 2 or 7.

**7.** A kit for detection of a fusion gene of EML4 gene and ALK gene, comprising sense and antisense primers which specifically amplify a polynucleotide encoding a polypeptide selected from the group consisting of:

(1) a polypeptide comprising the amino acid sequence represented by SEQ ID NO: 2, 7 or 130 and having a kinase activity,
(2) a polypeptide comprising an amino acid sequence derived from the amino acid sequence represented by SEQ ID NO: 2 or 7 with the deletion, substitution, and/or insertion of 1 to 10 amino acids and having a kinase activity,
(3) a polypeptide comprising an amino acid sequence with 90% or higher identity to the amino acid sequence represented by SEQ ID NO: 2 or 7 and having a kinase activity, and
(4) a polypeptide consisting of the amino acid sequence represented by SEQ ID NO: 2, 7 or 130.

**8.** The kit of claim 7, wherein the polypeptide comprises the amino acid sequence represented by SEQ ID NO: 2, 7 or 130 and having a kinase activity.

**9.** The kit of claim 7, wherein the polypeptide consists of the amino acid sequence represented by SEQ ID NO: 2, 7 or 130.

**10.** The kit of claim 7, wherein the polypeptide comprises the amino acid sequence represented by SEQ ID NO: 2 or 7 and having a kinase activity.

**11.** The kit of claim 7, wherein the polypeptide consists of the amino acid sequence represented by SEQ ID NO: 2 or 7.

**12.** A primer set for detecting a fusion gene of EML4 gene and ALK gene, wherein the primer set is selected from the following a) - c):

a) a primer set comprising an antisense primer consisting of a nucleic acid molecule hybridizing under stringent conditions to an ALK-encoding portion of a polynucleotide, and a sense primer consisting of a nucleic acid molecule hybridizing under stringent conditions to the complementary strand of an EML4-encoding portion of the polynucleotide, wherein the polynucleotide encodes a polypeptide selected from the group consisting of:

(1) a polypeptide comprising the amino acid sequence represented by SEQ ID NO: 2 or 7 and having a kinase activity,
(2) a polypeptide comprising an amino acid sequence derived from the amino acid sequence represented by SEQ ID NO: 2 or 7 with the deletion, substitution, and/or insertion of 1 to 10 amino acids and having a kinase activity,
(3) a polypeptide comprising an amino acid sequence with 90% or higher identity to the amino acid sequence represented by SEQ ID NO: 2 or 7 and having a kinase activity, and
(4) a polypeptide consisting of the amino acid sequence represented by SEQ ID NO: 2 or 7;

b) a primer set comprising an antisense primer consisting of a nucleic acid molecule hybridizing under stringent conditions to an ALK-encoding portion of the polynucleotide consisting of the nucleotide sequence represented by SEQ ID NO: 4, and a sense primer consisting of a nucleic acid molecule hybridizing under stringent conditions to the complementary strand of an EML4-encoding portion of the polynucleotide; and
c) a primer set comprising an antisense primer consisting of a nucleic acid molecule hybridizing under stringent conditions to an ALK-encoding portion of the polynucleotide consisting of the nucleotide sequence represented by SEQ ID NO: 5, and a sense primer consisting of a nucleic acid molecule hybridizing under stringent conditions to the complementary strand of an EML4-encoding portion of the polynucleotide..

**13.** The primer set of claim 12, wherein the polypeptide comprises the amino acid sequence represented by SEQ ID NO: 2 or 7 and having a kinase activity.

**14.** The primer set of claim 12, wherein the polypeptide consists of the amino acid sequence represented by SEQ ID NO: 2 or 7.

**15.** A primer set of a sense primer comprising an oligonucleotide with at least any 16 consecutive bases located at base Nos. 1 to 2242 in SEQ ID NO: 6 and an antisense primer comprising an oligonucleotide complementary to an oligonucleotide with at least any 16 consecutive bases located at base Nos. 2243 to 3933 in SEQ ID NO: 6, or a primer set consisting of complementary strands thereof, wherein the sense and antisense primers give amplification products of 1 kb or less in size.

**16.** A primer set of a sense primer comprising an oligonucleotide with at least any 16 consecutive bases located at base Nos. 1 to 3629 in SEQ ID NO: 4 and an antisense primer comprising an oligonucleotide complementary to an oligonucleotide with at least any 16 consecutive bases located at base Nos. 3630 to 3979 in SEQ ID NO: 4, or a primer set consisting of complementary strands thereof, wherein the sense and antisense primers give amplification products of 1 kb or less in size.

**17.** A primer set of a sense primer comprising an oligonucleotide with at least any 16 consecutive bases located at base Nos. 1 to 579 in SEQ ID NO: 5 and an antisense primer comprising an oligonucleotide complementary to an oligonucleotide with at least any 16 consecutive bases located at base Nos. 580 to 853 in SEQ ID NO: 5, or a primer set consisting of complementary strands thereof.

## Patentansprüche

**1.** Verfahren zur Detektion eines Fusionsgens aus dem Gen für Echinoderm-Mikrotubuli-assoziiertem Protein ähnliches Protein 4 (EML4-Gen) und dem Gen für anaplastische Lympohomkinase (ALK-Gen), umfassend den Schritt des Nachweisens der Gegenwart eines Polynucleotids in einer von einem Testsubjekt erhaltenen Probe, wobei das Polynucleotid für ein aus der aus Folgendem bestehenden Gruppe ausgewähltes Polypeptid kodiert:

(1) ein Polypeptid, das die durch Seq.-ID Nr. 2, 7 oder 130 dargestellte Aminosäuresequenz umfasst und das eine Kinase-Aktivität aufweist,
(2) ein Polypeptid, das eine Aminosäuresequenz umfasst, die von der durch Seq.-ID Nr. 2 oder 7 dargestellten Aminosäuresequenz durch Deletion, Substitution und/oder Insertion von 1 bis 10 Aminosäuren abgeleitet ist, und das eine Kinase-Aktivität aufweist,
(3) ein Polypeptid, das eine Aminosäuresequenz umfasst, die 90 % oder ein höheres Ausmaß an Sequenzidentität mit der durch Seq.-ID Nr. 2 oder 7 dargestellten Aminosäuresequenz aufweist, und das eine Kinase-Aktivität aufweist, und
(4) ein Polypeptid, das aus der durch Seq.-ID Nr. 2, 7 oder 130 dargestellten Aminosäuresequenz besteht.

**2.** Verfahren zur Detektion eines Fusionsproteins, für das ein Fusionsgen aus EML4-Gen und ALK-Gen kodiert, umfassend den Schritt des Nachweisens der Gegenwart eines Polypeptids in der von einem Testobjekt erhaltenen Probe, wobei das Polypeptid aus der aus Folgendem bestehenden Gruppe ausgewählt ist:

(1) einem Polypeptid, das die durch Seq.-ID Nr. 2, 7 oder 130 dargestellte Aminosäuresequenz umfasst und das eine Kinase-Aktivität aufweist,
(2) einem Polypeptid, das eine Aminosäuresequenz umfasst, die von der durch Seq.-ID Nr. 2 oder 7 dargestellten Aminosäuresequenz durch Deletion, Substitution und/oder Insertion von 1 bis 10 Aminosäuren abgeleitet ist, und das eine Kinase-Aktivität aufweist,
(3) einem Polypeptid, das eine Aminosäuresequenz umfasst, die 90 % oder ein höheres Ausmaß an Sequenzidentität mit der durch Seq.-ID Nr. 2 oder 7 dargestellten Aminosäuresequenz aufweist, und das eine Kinase-Aktivität aufweist, und
(4) einem Polypeptid, das aus der durch Seq.-ID Nr. 2, 7 oder 130 dargestellten Aminosäuresequenz besteht.

**3.** Verfahren nach Anspruch 1 oder 2, worin das Polypeptid die durch Seq.-ID Nr. 2, 7 oder 130 dargestellte Aminosäuresequenz umfasst und eine Kinase-Aktivität aufweist.

**4.** Verfahren nach Anspruch 1 oder 2, worin das Polypeptid aus der durch Seq.-ID Nr. 2, 7 oder 130 dargestellten Aminosäuresequenz besteht.

**5.** Verfahren nach Anspruch 1 oder 2, worin das Polypeptid die durch Seq.-ID Nr. 2 oder 7 dargestellte Aminosäuresequenz umfasst und eine Kinase-Aktivität aufweist.

**6.** Verfahren nach Anspruch 1 oder 2, worin das Polypeptid aus der durch Seq.-ID Nr. 2 oder 7 dargestellten Aminosäuresequenz besteht.

**7.** Set zur Detektion eines Fusionsgen von EML4-Gen und ALK-Gen, umfassend Sense- und Antisense-Primer, die spezifisch ein Polynucleotid amplifizieren, das für ein aus der aus Folgendem bestehenden Gruppe ausgewähltes Polypeptid kodiert:

(1) ein Polypeptid, das die durch Seq.-ID Nr. 2, 7 oder 130 dargestellte Aminosäuresequenz umfasst und das eine Kinase-Aktivität aufweist,
(2) ein Polypeptid, das eine Aminosäuresequenz umfasst, die von der durch Seq.-ID Nr. 2 oder 7 dargestellten Aminosäuresequenz durch Deletion, Substitution und/oder Insertion von 1 bis 10 Aminosäuren abgeleitet ist, und das eine Kinase-Aktivität aufweist,
(3) ein Polypeptid, das eine Aminosäuresequenz umfasst, die 90 % oder ein höheres Ausmaß an Sequenzidentität mit der durch Seq.-ID Nr. 2 oder 7 dargestellten Aminosäuresequenz aufweist, und das eine Kinase-Aktivität aufweist, und
(4) ein Polypeptid, das aus der durch Seq.-ID Nr. 2, 7 oder 130 dargestellten Aminosäuresequenz besteht.

**8.** Set nach Anspruch 7, worin das Polypeptid die durch Seq.-ID Nr. 2, 7 oder 130 dargestellte Aminosäuresequenz umfasst und eine Kinase-Aktivität aufweist.

**9.** Set nach Anspruch 7, worin das Polypeptid aus der durch Seq.-ID Nr. 2, 7 oder 130 dargestellten Aminosäuresequenz besteht.

**10.** Set nach Anspruch 7, worin das Polypeptid die durch Seq.-ID Nr. 2 oder 7 dargestellte Aminosäuresequenz umfasst und eine Kinase-Aktivität aufweist.

**11.** Set nach Anspruch 7, worin das Polypeptid aus der durch Seq.-ID Nr. 2 oder 7 dargestellten Aminosäuresequenz besteht.

**12.** Primerset zur Detektion eines Fusionsgens aus EML4-Gen und ALK-Gen, worin das Primerset aus den folgenden Sets a)-c) ausgewählt ist:

a) einem Primerset, das einen Antisenseprimer, der aus einem Nucleinsäuremolekül besteht, das unter stringenten Bedingungen an einen für ALK kodierenden Abschnitt eines Polynucleotids hybridisiert, und einen Senseprimer umfasst, der aus einem Nucleinsäuremolekül besteht, das unter stringenten Bedingungen an den komplementären Strang eines für EML4 kodierenden Abschnitts des Polynucleotids hybridisiert, wobei das Polynucleotid für ein aus der aus Folgendem bestehenden Gruppe ausgewähltes Polypeptid kodiert:

(1) ein Polypeptid, das die durch Seq.-ID Nr. 2, 7 oder 130 dargestellte Aminosäuresequenz umfasst und das eine Kinase-Aktivität aufweist,
(2) ein Polypeptid, das eine Aminosäuresequenz umfasst, die von der durch Seq.-ID Nr. 2 oder 7 dargestellten Aminosäuresequenz durch Deletion, Substitution und/oder Insertion von 1 bis 10 Aminosäuren abgeleitet ist, und das eine Kinase-Aktivität aufweist,
(3) ein Polypeptid, das eine Aminosäuresequenz umfasst, die 90 % oder ein höheres Ausmaß an Sequenzidentität mit der durch Seq.-ID Nr. 2 oder 7 dargestellten Aminosäuresequenz aufweist, und das eine Kinase-Aktivität aufweist, und
(4) ein Polypeptid, das aus der durch Seq.-ID Nr. 2, 7 oder 130 dargestellten Aminosäuresequenz besteht;

b) ein Primerset, das einen Antisenseprimer, der aus einem Nucleinsäuremolekül besteht, das unter stringenten Bedingungen an einen für ALK kodierenden Abschnitt des Polynucleotids hybridisiert, das aus der durch Seq.-ID Nr. 4 dargestellten Nucleotidsequenz besteht, und einen Senseprimer umfasst, der aus einem Nucleinsäuremolekül besteht, das unter stringenten Bedingungen an den komplementären Strang eines für EML4 kodierenden Abschnitts des Polynucleotids hybridisiert;
c) ein Primerset, das einen Antisenseprimer, der aus einem Nucleinsäuremolekül besteht, das unter stringenten Bedingungen an einen für ALK kodierenden Abschnitt des Polynucleotids hybridisiert, das aus der durch Seq.-ID Nr. 5 dargestellten Nucleotidsequenz besteht, und einen Senseprimer umfasst, der aus einem Nucleinsäuremolekül besteht, das unter stringenten Bedingungen an den komplementären Strang eines für EML4 kodierenden Abschnitts des Polynucleotids hybridisiert.

**13.** Primerset nach Anspruch 12, worin das Polypeptid die durch Seq.-ID Nr. 2 oder 7 dargestellte Aminosäuresequenz umfasst und eine Kinase-Aktivität aufweist.

**14.** Primerset nach Anspruch 12, worin das Polypeptid aus der durch Seq.-ID Nr. 2 oder 7 dargestellten Aminosäuresequenz besteht.

**15.** Primerset aus einem Senseprimer, der ein Oligonucleotid mit zumindest 16 beliebigen aufeinander folgenden Basen umfasst, die in Seq.-ID Nr. 6 an den Basen-Nrn. 1 bis 2242 angeordnet sind, und einem Antisenseprimer, der ein Oligonucleotid umfasst, das in Bezug auf ein Oligonucleotid mit zumindest 16 beliebigen aufeinander folgenden Basen, die in Seq.-ID Nr. 6 an den Basen-Nrn. 2243 bis 3933 angeordnet sind, komplementär ist, oder ein Primerset, das aus komplementären Strängen davon besteht, wobei der Sense- und der Antisenseprimer Amplifikationsprodukte mit einer Größe von 1 kb oder weniger ergeben.

**16.** Primerset aus einem Senseprimer, der ein Oligonucleotid mit zumindest 16 beliebigen aufeinander folgenden Basen umfasst, die in Seq.-ID Nr. 4 an den Basen-Nrn. 1 bis 3629 angeordnet sind, und einem Antisenseprimer, der ein Oligonucleotid umfasst, das in Bezug auf ein Oligonucleotid mit zumindest 16 beliebigen aufeinander folgenden Basen, die in Seq.-ID Nr. 4 an den Basen-Nrn. 3630 bis 3979 angeordnet sind, komplementär ist, oder ein Primerset, das aus komplementären Strängen davon besteht, wobei der Sense- und der Antisenseprimer Amplifikationsprodukte mit einer Größe von 1 kb oder weniger ergeben.

**17.** Primerset aus einem Senseprimer, der ein Oligonucleotid mit zumindest 16 beliebigen aufeinander folgenden Basen umfasst, die in Seq.-ID Nr. 5 an den Basen-Nrn. 1 bis 579 angeordnet sind, und einem Antisenseprimer, der ein Oligonucleotid umfasst, das in Bezug auf ein Oligonucleotid mit zumindest 16 beliebigen aufeinander folgenden Basen, die in Seq.-ID Nr. 5 an den Basen-Nrn. 580 bis 853 angeordnet sind, komplementär ist, oder ein Primerset, das aus komplementären Strängen davon besteht.

## Revendications

1. Méthode de détection d'un gène de fusion d'un gène de protéine 4 semblable à la protéine échinoderme microtubule associée 4 (EML4) et un gène de kinase anaplastique de lymphomes (ALK), comprenant l'étape consistant à détecter la présence d'un polynucléotide dans un échantillon obtenu d'un sujet test, où le polynucléotide code pour un polypeptide sélectionné dans le groupe consistant en:

    (1) un polypeptide comprenant la séquence des acides aminés représentée par SEQ ID NO: 2, 7 ou 130 et ayant une activité kinase,
    (2) un polypeptide comprenant une séquence d'acides aminés dérivée de la séquence d'acides aminés représentée par SEQ ID NO: 2 ou 7 avec délétion, substitution, et/ou insertion de 1 à 10 acides aminés et ayant une activité kinase,
    (3) un polypeptide comprenant une séquence d'acides aminés avec une identité de 90% ou plus élevée avec la séquence des acides aminés représentés par SEQ ID NO: 2 ou 7 et ayant une activité kinase, et
    (4) un polypeptide consistant en la séquence d'acides aminés représentée par SEQ ID NO: 2, 7 ou 130.

2. Procédé pour détecter une protéine de fusion codée par un gène de fusion du gène EML4 et du gène ALK, comprenant l'étape consistant à détecter la présence d'un polypeptide dans un échantillon obtenu d'un sujet test, où le polypeptide est sélectionné dans le groupe consistant en:

    (1) un polypeptide comprenant la séquence d'acides aminés représentée par SEQ ID NO 2, 7 ou 130 et ayant une activité kinase,
    (2) un polypeptide comprenant une séquence d'acides aminés dérivée de la séquence d'acides aminés représentée par SEQ ID NO: 2 ou 7 avec la délétion, substitution, et/ou insertion de 1 à 10 acides aminés et ayant une activité kinase,
    (3) un polypeptide comprenant une séquence d'acides aminés avec une identité de 90% ou plus élevée à la séquence des acides aminés représentée par SEQ ID NO: 2 ou 7 et ayant une activité kinase, et
    (4) un polypeptide consistant en la séquence des acides aminés représentée par SEQ ID NO: 2, 7 ou 130.

3. Méthode selon la revendication 1 ou la revendication 2, dans laquelle le polypeptide comprend la séquence des acides aminés représentée par SEQ ID NO: 2, 7 ou 130 et ayant une activité kinase.

4. Méthode selon la revendication 1 ou la revendication 2, où le polypeptide consiste en la séquence des acides aminés représentée par SEQ ID NO: 2, 7 ou 130.

5. Méthode selon la revendication 1 ou la revendication 2, où le polypeptide comprend la séquence des acides aminés représentée par SEQ ID NO: 2 ou 7 et ayant une activité kinase.

6. Méthode selon la revendication 1 ou la revendication 2, où le polypeptide consiste en la séquence des acides aminés représentée par SEQ ID NO: 2 ou 7.

7. Kit pour la détection d'un gène de fusion du gène EML4 et du gène ALK, comrpenant des amorces sens et antisens qui amplifient spécifiquement un polynucléotide codant pour un polypeptide sélectionné dans le groupe consistant en:

    (1) un polypeptide comprenant la séquence des acides aminés représentée par SEQ ID NO: 2, 7 ou 130 et ayant une activité kinase,
    (2) un polypeptide comprenant une séquence d'acides aminés dérivée de la séquence des acides aminés représentée par SEQ ID NO: 2 ou 7 avec la délétion, substitution et/ou insertion de 1 à 10 acides aminés et ayant une activité kinase;
    (3) un polypeptide comprenant une séquence d'acides aminés avec une identité de 90% ou plus élevée avec la séquence des acides aminés représentée par SEQ ID NO: 2 ou 7 et ayant une activité kinase, et
    (4) un polypeptide consistant en la séquence des acides aminés représentée par SEQ ID NO: 2, 7 ou 130.

8. Kit selon la revendication 7, où le polypeptide comprend la séquence des acides aminés représentée par SEQ ID NO: 2, 7 ou 130 et ayant une activité kinase.

9. Kit selon la revendication 7, où le polypeptide consiste en la séquence des acides aminés représentée par SEQ ID NO: 2, 7 ou 130.

**10.** Kit selon la revendication 7, où le polypeptide comprend la séquence des acides aminés représentée par SEQ ID NO: 2 ou 7 et ayant une activité kinase.

**11.** Kit selon la revendication 7, où le polypeptide consiste en la séquence des acides aminés représentée par SEQ ID NO: 2 ou 7.

**12.** Ensemble d'amorces pour détecter un gène de fusion du gène EML4 et du gène ALK, où l'ensemble d'amorces est sélectionné parmi les suivants a) -c):

a) un ensemble d'amorces comprenant une amorce antisens consistant en une molécule d'acide nucléique s'hybridant sous des conditions stringentes à une portion codant pour ALK d'un polynucléotide, et une amorce sens consistant en une molécule d'acide nucléique s'hybridant sous des conditions stringentes au brin complémentaire d'une portion codant pour EML4 du polynucléotide, où le polynucléotide code pour un polypeptide sélectionné dans le groupe consistant en:

(1) un polypeptide comprenant la séquence des acides aminés représentée par SEQ ID NO: 2 ou 7 et ayant une activité kinase,
(2) un polypeptide comprenant une séquence d'acides aminés dérivée de la séquence des acides aminés représentée par SEQ ID NO: 2 ou 7 avec la délétion, substitution et/ou insertion de 1 à 10 acides aminés et ayant une activité kinase,
(3) un polypeptide comprenant une séquence d'acides aminés avec une identité de 90% ou plus élevée avec la séquence des acides aminés représentée par SEQ ID NO: 2 ou 7 et ayant une activité kinase, et
(4) un polypeptide consistant en la séquence des acides aminés représentée par SEQ ID NO: 2 ou 7;

b) un ensemble d'amorces comprenant une amorce antisens consistant en une molécule d'acide nucléique s'hybridant sous des conditions stringentes à une portion codant pour ALK du polynucléotide consistant en la séquence de nucléotides représentée par SEQ ID NO: 4, et une amorce sens consistant en une molécule d'acide s'hybridant sous des conditions stringentes au brin complémentaire d'une portion codant pour EML4 du polynucléotide; et
c) un ensemble d'amorces comprenant une amorce antisens consistant en une molécule d'acide nucléique s'hybridant sous des conditions stringentes à une portion codant pour ALK du polynucléotide consistant en la séquence de nucléotides représentée par SEQ ID NO: 5, et une amorce sens consistant en une molécule d'acide s'hybridant sous des conditions stringentes au brin complémentaire d'une portion codant pour EML4 du polynucléotide.

**13.** Ensemble d'amorces selon la revendication 12, où le polypeptide comprend la séquence des acides aminés représentée par SEQ ID NO: 2 ou 7 et ayant une activité kinase.

**14.** Ensemble d'amorces selon la revendication 12, où le polypeptide consiste en la séquence des acides aminés représentée par SEQ ID NO: 2 ou 7.

**15.** Ensemble d'amorces d'une amorce sens comprenant un oligonucléotide avec au moins toutes les 16 bases consécutives situées aux bases Nos. 1 à 2242 dans la SEQ ID NO: 6 et une amorce antisens comprenant un oligonucléotide complémentaire à un oligonucléotide avec au moins toutes les 16 bases consécutives situées aux bases Nos. 2243 à 3933 dans la SEQ ID NO: 6, ou un ensemble d'amorces consistant en des brins complémentaires de celui-ci, où les amorces sens et antisens donnent des produits d'amplification de 1 kb ou moins en taille.

**16.** Ensemble d'amorces d'une amorce sens comprenant un oligonucléotide avec au moins toutes les 16 bases consécutives situées aux bases Nos. 1 à 3629 dans la SEQ ID NO: 4 et une amorce antisens comprenant un oligonucléotide complémentaire à un oligonucléotide avec au moins toutes les 16 bases consécutives situées aux bases Nos. 3630 à 3979 dans la SEQ ID NO: 4, ou bien un ensemble d'amorces consistant en brins complémentaires de celui-ci, où les amorces sens et antisens donnent des produits d'amplification de 1 kb ou moins en taille.

**17.** Ensemble d'amorces d'une amorce sens comprenant un oligonucléotide avec au moins toutes les 16 bases consécutives situées aux bases Nos. 1 à 579 dans la SEQ ID NO: 5 et une amorce antisens comprenant un oligonucléotide complémentaire à un oligonucléotide avec au moins toutes les 16 bases consécutives situées aux bases Nos. 580 à 853 dans la SEQ ID NO: 5, ou un ensemble d'amorces consistant en brins complémentaires de celui-ci.

Fig. 1

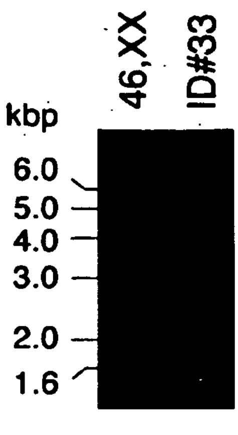

Fig. 2

| ID | Marker | NTC | 46,XX | #2 | #6 | #7 | #12 | #13 | #14 | #16 | #17 | #18 | #19 | #20 | #21 | #22 | #23 | #24 | #25 | #26 | #27 | #28 | #29 | #30 | #31 | #32 | #33 | #34 | #35 | #36 | #37 | #38 | #39 | #40 | #41 | #42 |
|---|---|---|---|---|---|---|---|---|---|---|---|---|---|---|---|---|---|---|---|---|---|---|---|---|---|---|---|---|---|---|---|---|---|---|---|---|
| Sex | | | F | M | M | M | M | F | M | M | F | F | F | M | M | M | F | F | M | M | M | M | M | M | M | M | M | F | M | F | F | M | F | M | F | M |
| Pathology | | | | S | S | A | S | A | A S | A S | A | A | A | S | S | A | A | B | B | S | A | A | S | S | A | S | A | A | A | A | A | S | A | A | A | S |
| EGFR mutation | | | | | | | | | | ○ | | ○ | | | | ○ | | ○ | | | | | | | | | | | ○ | | ○ | | | | | |
| Smoking habit | | | | ○ | ○ | ○ | ○ | ○ | ○ | ○ | | | | ○ | ○ | ○ | | | ○ | ○ | ○ | ○ | ○ | ○ | ○ | ○ | ○ | | ○ | | | ○ | | ○ | ○ | ○ |

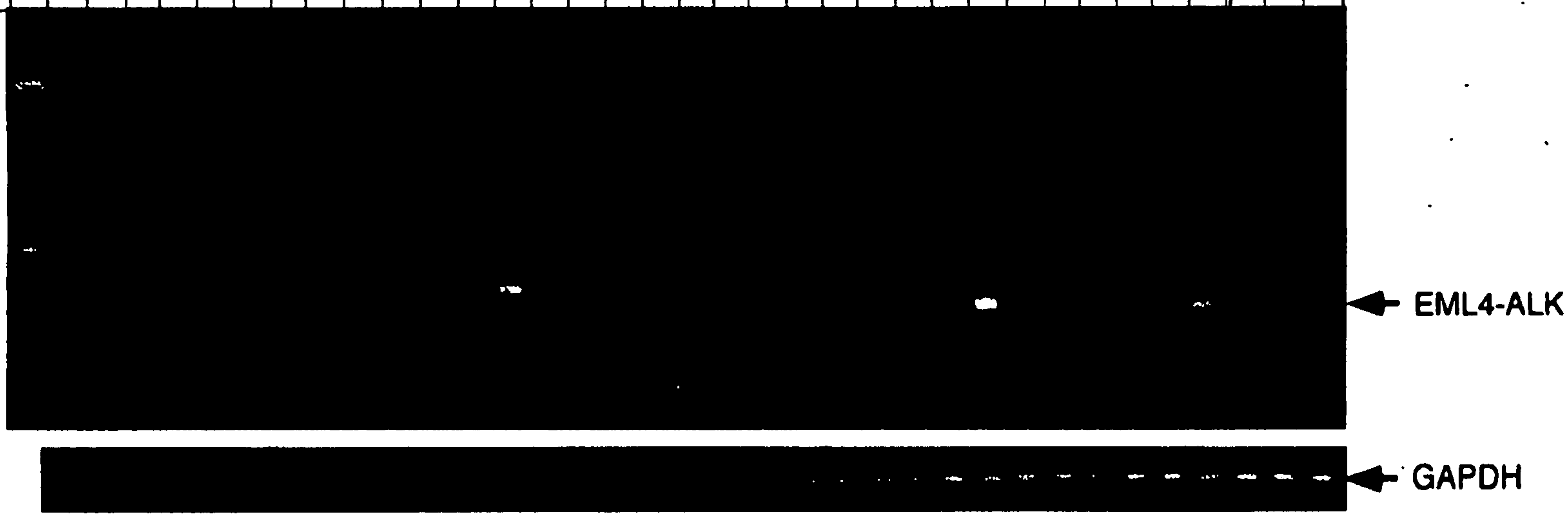

Fig. 3

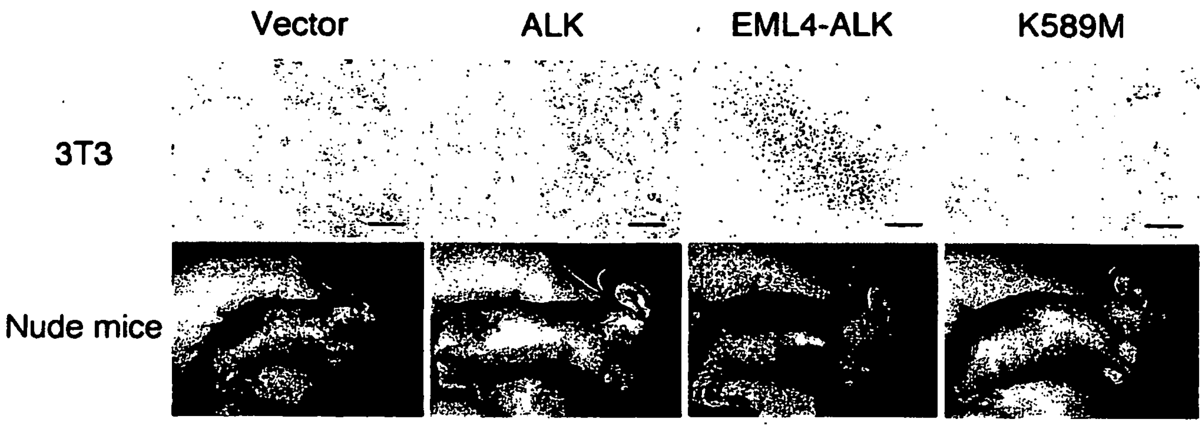

Fig. 4

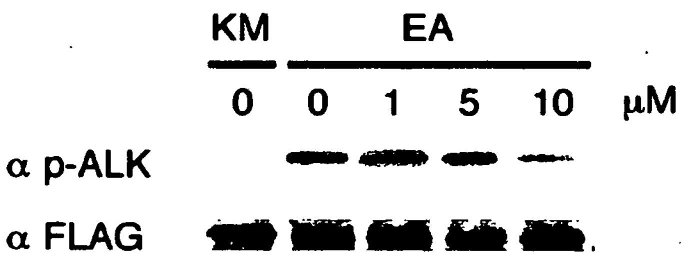

Fig. 5

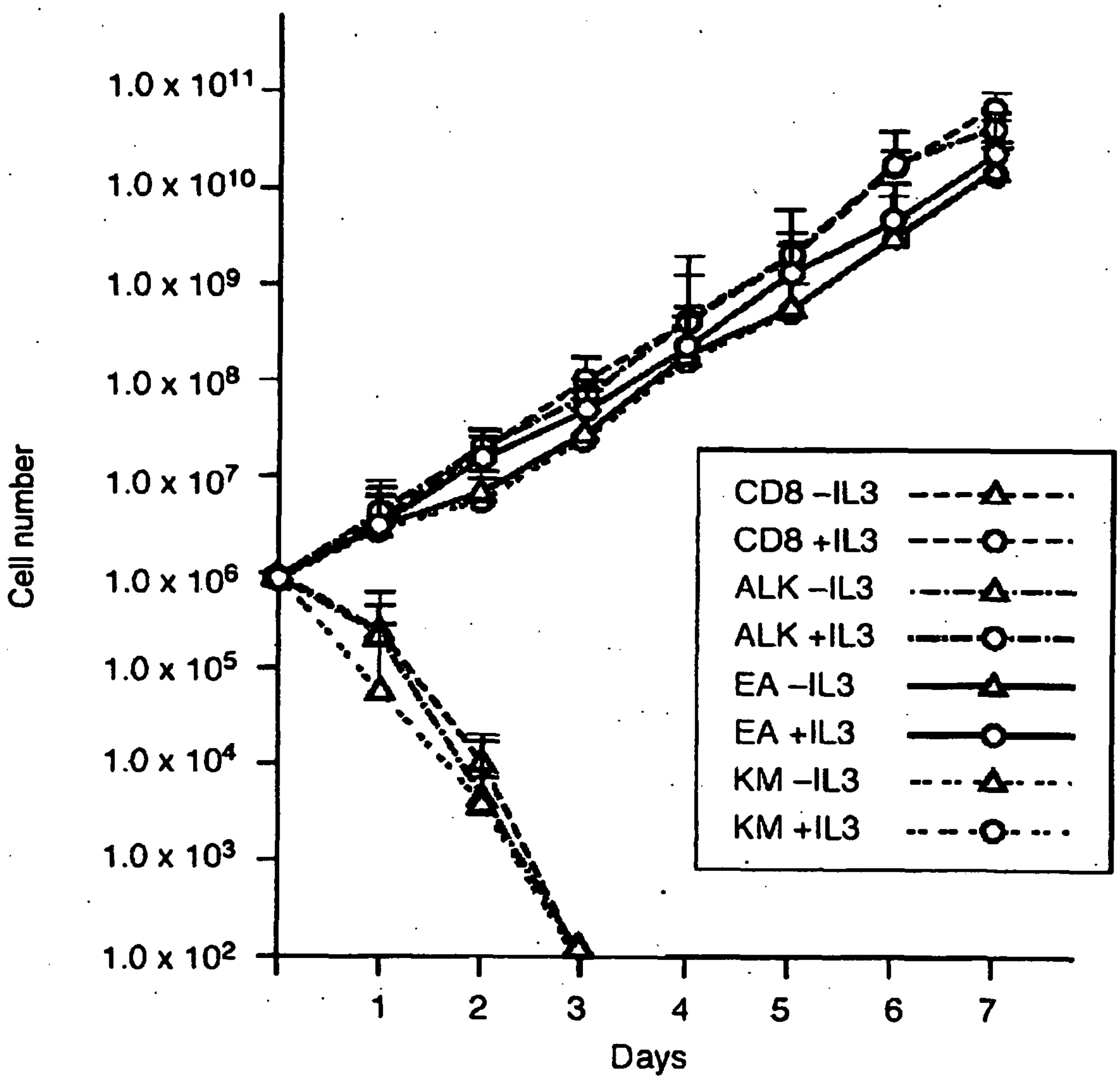
1.0 x 10^11
1.0 x 10^10
1.0 x 10^9
1.0 x 10^8
1.0 x 10^7
1.0 x 10^6
1.0 x 10^5
1.0 x 10^4
1.0 x 10^3
1.0 x 10^2
Cell number
1
2
3
4
5
6
7
Days
CD8 –IL3
CD8 +IL3
ALK –IL3
ALK +IL3
EA –IL3
EA +IL3
KM –IL3
KM +IL3

Fig. 6

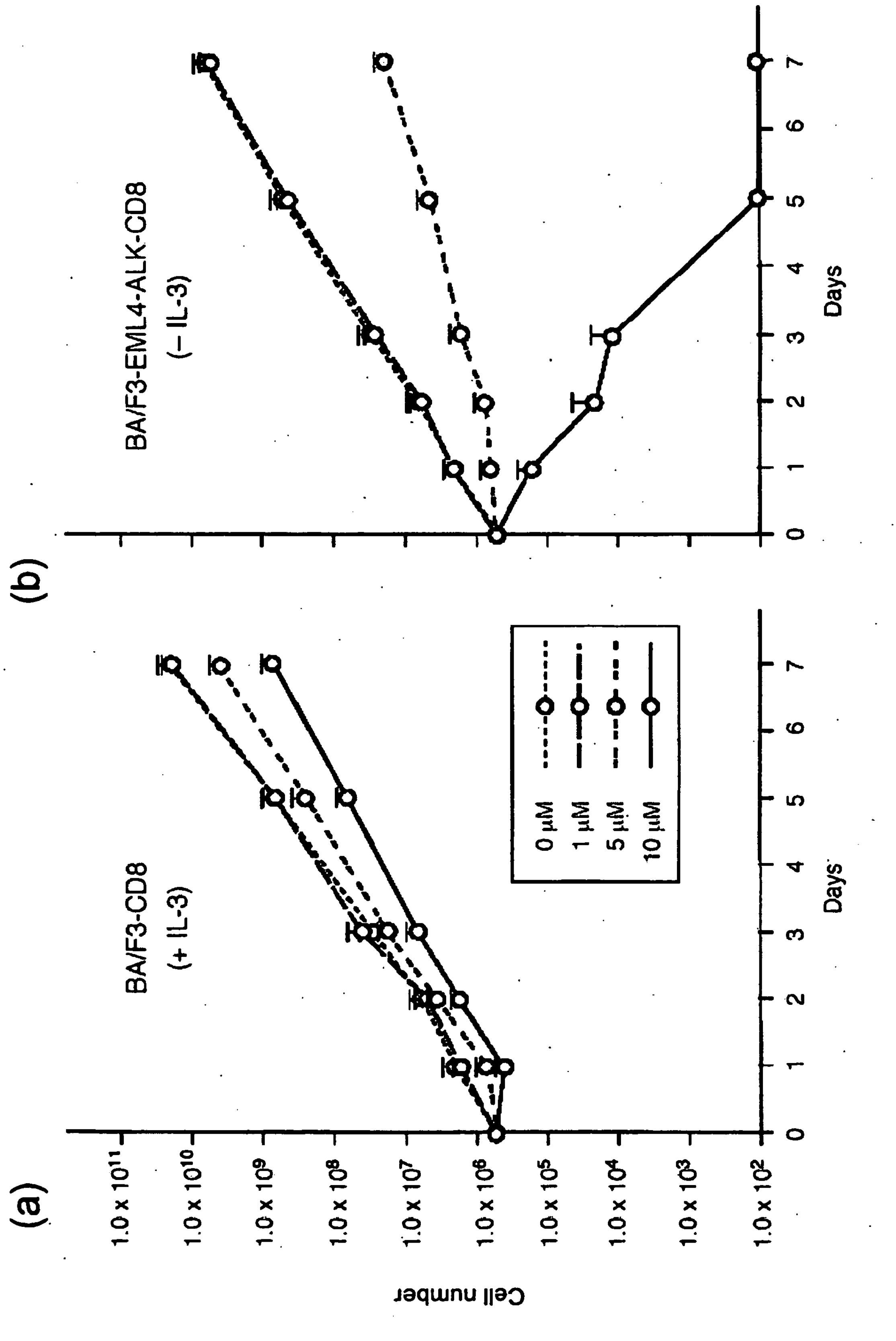
(a)
BA/F3-CD8
(+ IL-3)
(b)
BA/F3-EML4-ALK-CD8
(– IL-3)
Cell number
1.0 x 10^11
1.0 x 10^10
1.0 x 10^9
1.0 x 10^8
1.0 x 10^7
1.0 x 10^6
1.0 x 10^5
1.0 x 10^4
1.0 x 10^3
1.0 x 10^2
Days
0 1 2 3 4 5 6 7
0 μM
1 μM
5 μM
10 μM

# Fig. 7

siRNA-1
5' UGGGAAAGGACCUAAAGTGTA 3'
3' GGACCCUUUCCUGGATTTCAC 5'

siRNA-2
5' GGGAAAGGACCUAAAGTGTAC 3'
3' GACCCUUUCCUGGAUTTCACA 5'

siRNA-3
5' GGACCUAAAGUGUACCGCCGG 3'
3' UUCCUGGAUUUCACATGGCGG 5'

siRNA-4
5' CCUAAAGUGUACCGCCGGAAG 3'
3' CUGGAUUUCACAUGGCGGCCT 5'

siRNA-5
5' AAAGUGUACCGCCGGAAGCAC 3'
3' GAUUUCACAUGGCGGCCTTCG 5'

siRNA-6
5' AAGUGUACCGCCGGAAGCACC 3'
3' AUUUCACAUGGCGGCCTTCGT 5'

siRNA-7
5' GGCCUGUAUACCGGATAATGA 3'
3' ACCCGGACAUAUGGCCTATTA 5'

siRNA-8
5' GGCCUGUAUACCGGAUAAUGA 3'
3' ACCCGGACAUAUGGCCUAUUA 5'

siRNA-9
5' CGGCUGCAAUCGATTGATAGC 3'
3' AAGCCGACGUUAGCUAACTAT 5'

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2004080980 A
- WO 2005009389 A
- WO 2005016894 A
- WO 0134785 A
- WO 2005097765 A
- JP 2006277718 A
- JP 2007120670 A
- CA 2598893


### Non-patent literature cited in the description


- *The New England journal of medicine,* 2005, vol. 353, 172-187
- *Proceedings of the 65th Annual Meeting of the Japanese Cancer Association,* 28 August 2006, O-324
- *Oncogene,* 30 January 1997, vol. 14 (4), 439-49
- *PNAS,* 2006, vol. 103, 7402-7407
- *Seminars in oncology,* 1993, vol. 20, 105-127
- *Genomics,* 2000, vol. 68, 348-350
- *Oncogene,* 1994, vol. 9, 1567-1574
- *Cellular and molecular life sciences,* 2004, vol. 61, 2939-2953
- *Am J Pathol,* 2002, vol. 160, 1487-1494
- *Journal of cellular physiology,* 2004, vol. 199, 330-358
- *International journal of cancer,* 2002, vol. 100, 49-56
- *Science,* 1994, vol. 263, 1281-1284
- *Blood,* 1995, vol. 86, 1954-1960
- *Blood,* 2000, vol. 95, 3204-3207
- *Blood,* 1999, vol. 94, 3265-3268
- *Laboratory investigation; a journal of technical methods and pathology,* 2003, vol. 83, 1255-1265
- *International journal of cancer,* 2006, vol. 118, 1181-1186
- *Blood,* 2006, vol. 107, 689-697
- *Blood,* 2006, vol. 107, 1617-1623
- *Laboratory investigation; a journal of technical methods and pathology,* 2005, vol. 85, 1544-1554
- *Journal of medicinal chemistry,* 2006, vol. 49, 1006-1015
- *J Comb Chem.,* 2006, vol. 8, 401-409
- *Science,* 1997, vol. 278, 1309-1312
- *Am J Pathol,* 2000, vol. 157, 377-384
- *Blood,* 1997, vol. 90, 2901-2910
- *Am J Pathol.,* March 2000, vol. 156 (3), 781-9
- *PNAS,* December 2006, vol. 104 (1), 270-275
- **Sambrook, J et al.** Molecular Cloning. Cold Spring Harbor Laboratory Press, 1989
- Experimental Protocol on Proteins. Shujunsha Co. Ltd, 1997
- *J Mol Biol,* 1970, vol. 48, 443-453
- *Genome Res.,* 1996, vol. 6 (10), 986
- **N. Terrett et al.** *Drug Discov. Today,* 1999, vol. 4 (1), 41
- **Wan W et al.** *Blood,* 2006, vol. 107, 1617-1623
- **Marzec M et al.** *Lab Invest,* 2005, vol. 85, 1544-1554
- *Genes Dev.,* 2001, vol. 15, 485-490
- *J. Am. Chem. Soc.,* 1998, vol. 120, 11820-11821
- *Methods,* 2001, vol. 23, 206-217
- *JBC,* 2000, vol. 275, 24945-24952
- *J. Biol. Chem.,* 2001, vol. 276, 39012-39020
- **Morris, SW et al.** *Science,* 04 March 1994, vol. 263 (5151), 1281-4